# EUROPEAN PATENT APPLICATION

(11) **EP 1 938 823 A1**
(43) Date of publication of application: **02.07.2008**
(21) Application number: 06812002.1
(22) Date of filing: 19.10.2006
(51) Int. Cl.: A61K 31/519, A61P 35/00, A61P 43/00, C07D 491/22

(54) **AGENT FOR PREVENTING OR TREATING PANCREAS CANCER, OVARY CANCER OR LIVER CANCER CONTAINING NOVEL WATER-SOLUBLE PRODRUG**

(30) Priority: 19.10.2005 JP 2005304208
(71) Applicant: CHUGAI SEIYAKU KABUSHIKI KAISHA, Tokyo, 115-8543 (JP)
(72) Inventor: UMEDA, Isao, Kanagawa 2210053 (JP); OHWADA, Jun, Kamakura-shi, Kanagawa 2478530 (JP); OZAWA, Sawako, Kamakura-shi, Kanagawa 2478530 (JP); ENDO, Mika, Kamakura-shi, Kanagawa 2478530 (JP); URA, Masako, Kamakura-shi, Kanagawa 2478530 (JP)
(74) Representative: Vossius & Partner
(86) International application number: PCT/JP2006/320814
(87) International publication number: WO 2007/046456

(57) **Abstract**

Preventive or therapeutic agents for pancreatic cancer, ovarian cancer, or liver cancer of the present invention comprise a water-soluble prodrug represented by formula 1 described below, or a pharmaceutically acceptable salt, or a hydrate or solvate of the prodrug or pharmaceutically acceptable salt, 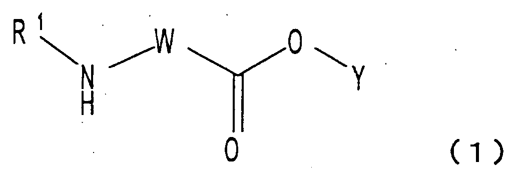 (wherein,
R¹ represents a hydrogen atom, or a C1-C6 alkyl group;
W represents a divalent group comprising a tertiary amino group or a divalent group comprising a sulfonyl group, and
Y represents a residue of a compound represented by Y-OH comprising an alcoholic hydroxyl group, wherein said Y-OH is a camptothecin, a taxane, or an anticancer nucleotide).

## Description

### Technical Field

The present invention relates to preventive or therapeutic agents comprising a novel water-soluble prodrug, a pharmaceutically acceptable salt thereof, or a hydrate or solvate of the prodrug or pharmaceutically acceptable salt, which are used for cell proliferative disorders such as pancreatic cancer, ovarian cancer, or liver cancer.

### Background Art

Camptothecins, taxanes, anticancer nucleotides and such are active against a wide range of tumor cells, and thus are expected to be useful as therapeutic agents, such as anticancer agents (Patent Documents 1 and 2). Many of these compounds are lipid soluble, and because of their low water solubility, there were instances where their use in injections (parenteral administration) was limited (Patent Document 1).

Water-soluble prodrugs have been studied in an attempt to solubilize such lipid-soluble pharmaceutical agents in water (Non-Patent Document 1 and Patent Document 1).
[Patent Document 1] WO03/043631
[Patent Document 2] WO03/045952
[Non-Patent Document 1] Shan et al., J. Pharm. Sci., 86(7), 765-767, 1997.

### Disclosure of the Invention

### [Problems to be Solved by the Invention]

Most water-soluble prodrugs of lipid-soluble pharmaceutical agents are converted into their active form after administration, mainly by enzymes. However, this conversion occurs after a certain period of time following administration and varies among species and individuals, and thus has been an obstacle to the development of these prodrugs. Therefore, there was a high demand to develop water-soluble prodrugs that can be parenterally administered, which do not depend on enzymatic conversion and show small interspecies or individual differences.

Furthermore, in some cases, the speed and efficiency in converting a prodrug to its active form in blood was insufficient, and therefore, there was a need to reduce the time taken for the increase of pharmaceutical agent concentration in blood.

More specifically, an objective of the present invention is to provide water-soluble prodrugs that can be parenterally administered, and whose conversion into the active form does not depend on enzymes and show small interspecies or individual differences, and that have an excellent conversion rate and efficiency, as well as to provide preventive or therapeutic agents comprising such a prodrug compound for cell proliferative disorders such as pancreatic cancer, ovarian cancer, or liver cancer.

### [Means for Solving the Problems]

Upon dedicated studies to solve the above-mentioned problems, the present inventors discovered that prodrug compounds having solubilizing side chains with particular structures show excellent water-solubility, and that their interspecies or individual differences are small due to the rapid chemical conversion to the active form. Further, the present inventors also discovered that the prodrug compounds are extremely useful for application to water-insoluble pharmaceutical agents such as camptothecins, which include a secondary alcoholic hydroxyl group or a tertiary alcoholic hydroxyl group. Furthermore, the present inventors discovered that pharmaceuticals comprising such a prodrug compound, a pharmaceutically acceptable salt thereof, or a hydrate or solvate of the prodrug compound or pharmaceutically acceptable salt are effective as preventive or therapeutic agents for cell proliferative disorders such as pancreatic cancer, ovarian cancer, or liver cancer, and thus, completed the present invention.

More specifically, the present invention comprises the followings:
[1] a preventive or therapeutic agent for pancreatic cancer, ovarian cancer, or liver cancer comprising a water-soluble prodrug represented by formula (1), or a pharmaceutically acceptable salt thereof, or a hydrate or solvate of the prodrug or pharmaceutically acceptable salt, 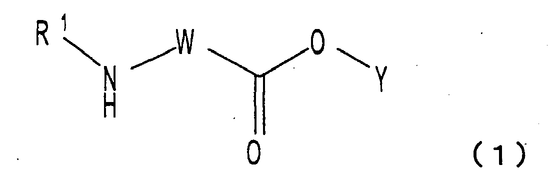 (wherein,
   R¹ represents a hydrogen atom, or a C1-C6 alkyl group;
   W represents a divalent group comprising a tertiary amino group or a divalent group comprising a sulfonyl group; and
   Y represents a residue of a compound represented by Y-0H comprising an alcoholic hydroxyl group, wherein said Y-OH is a camptothecin, a taxane, or an anticancer nucleotide);
[2] the preventive or therapeutic agent for pancreatic cancer, ovarian cancer, or liver cancer of [1], wherein the water-soluble prodrug is represented by formula (2): 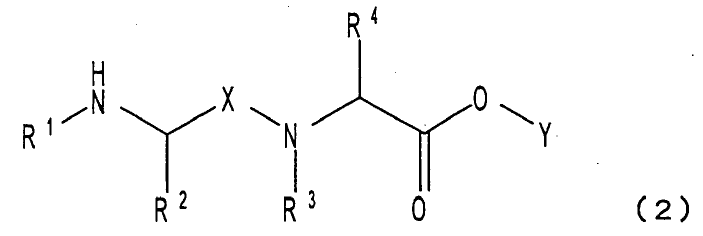 (wherein,
   R¹ and Y are defined as in formula (1);
   X represents a C=O or a C1-C3 alkylene group;
   R² and R⁴ each independently represents a hydrogen atom, a C1-C6 alkyl group, or an amino acid side chain; and R³ represents a C 1-C6 alkyl group);
[3] the preventive or therapeutic agent for pancreatic cancer, ovarian cancer, or liver cancer of [2], wherein R¹ is a hydrogen atom, a methyl group, or an ethyl group;
[4] the preventive or therapeutic agent for pancreatic cancer, ovarian cancer, or liver cancer of [2] or [3], wherein R² is a hydrogen atom or a methyl group;
[5] the preventive or therapeutic agent for pancreatic cancer, ovarian cancer, or liver cancer of any one of [2] to [4], wherein R³ is a C1-C3 alkyl group;
[6] the preventive or therapeutic agent for pancreatic cancer, ovarian cancer, or liver cancer of any one of [2] to [5], wherein R⁴ is a hydrogen atom or a methyl group;
[7] the preventive or therapeutic agent for pancreatic cancer, ovarian cancer, or liver cancer of [1], wherein the water-soluble prodrug is represented by formula (3): 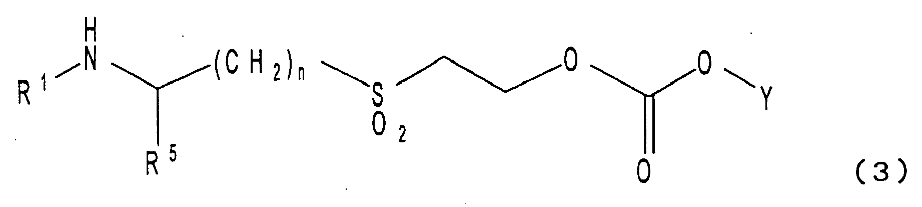 [wherein,
   R¹ and Y are defined as in formula (1);
   n represents an integer from 1 to 6; and
   R⁵ represents a hydrogen atom or -COOR⁶ (wherein R⁶ represents a hydrogen atom, or a C1-C6 alkyl group)];
[8] the preventive or therapeutic agent for pancreatic cancer, ovarian cancer, or liver cancer of [7], wherein R¹ is a hydrogen atom, a methyl group, or an ethyl group;
[9] the preventive or therapeutic agent for pancreatic cancer, ovarian cancer, or liver cancer of [7] or [8], wherein n is 1, and R⁵ is a hydrogen atom or -COOR⁶ (wherein R⁶ represents a hydrogen atom or a C1-C6 alkyl group);
[10] the preventive or therapeutic agent for pancreatic cancer, ovarian cancer, or liver cancer of [7] or [8], wherein n is an integer from 2 to 6, and R⁵ is a hydrogen atom;
[11] the preventive or therapeutic agent for pancreatic cancer, ovarian cancer, or liver cancer of [1] to [10], wherein the hydroxyl group (-OH) of Y-OH is a secondary or tertiary alcoholic hydroxyl group;
[12] the preventive or therapeutic agent for pancreatic cancer, ovarian cancer, or liver cancer of any one of [1] to [11], wherein Y-OH is an insoluble compound;
[13] the preventive or therapeutic agent for pancreatic cancer, ovarian cancer, or liver cancer of any one of [1] to [12], wherein Y is a group represented by formula (4): 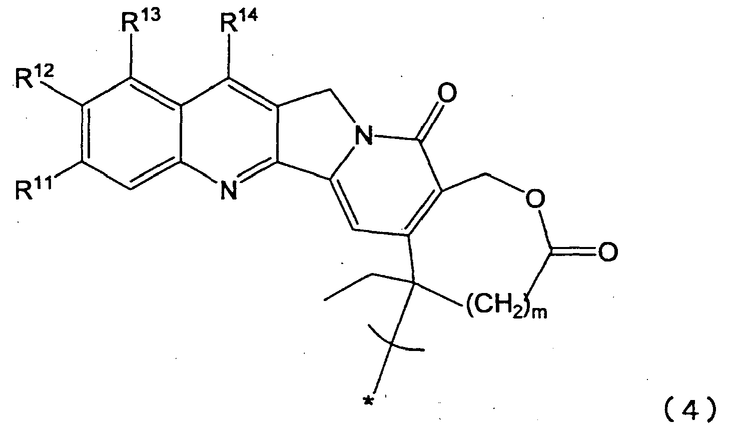 [wherein,
   * indicates a linkage site;
   m is either 0 or 1;
   R¹¹ represents a hydrogen atom, a halogen atom, or a C1-C6 alkyl group;
   R¹² represents a hydrogen atom, a halogen atom, a C1-C6 alkyl group, or a hydroxyl group;
   R¹³ represents a hydrogen atom, an amino group, a nitro group, or a (dimethylamino)methyl group;
   R¹⁴ represents a hydrogen atom, a C1-C6 alkyl group, a (4-methylpiperazinyl)methyl group, or a (*tert*-butoxyimino)methyl group;
   R¹³ and R¹⁴, and R¹¹ and R¹², may each be linked to each other to form a 5- or 6-membered ring, wherein the 5- or 6-membered ring may comprise one to two heteroatoms, and one to three substituents selected from Group A described below, wherein the substituents of Group A may further comprise one to three substituents selected from Group B described below:
   Group A: a C1-C10 alkyl group, an amino group, a mono-C1-C8 alkylamino group, a di-C1-C8 alkylamino group, a C1-C8 alkoxy group, a C1-C8 alkylthio group, and a group represented by X= (wherein X represents an oxygen atom or a sulfur atom);
   Group B: a C1-C6 alkoxy group, a hydroxy group, a halogen atom, an amino group, a mono-C1-C6 alkylamino group, a di-C1-C6 alkylamino group, a C3-C7 cycloalkyl group, a heterocycle, and an aryl ring (the aryl ring may comprise one to three substituents selected from the group consisting of a hydroxy group, a C1-C6 alkoxy group, a halogen atom, an amino group, a mono-C1-C6 alkylamino group, and a di-C1-C6 alkylamino group)];
[14] the preventive or therapeutic agent for pancreatic cancer, ovarian cancer, or liver cancer of [13], wherein Y is a group represented by formula (5): 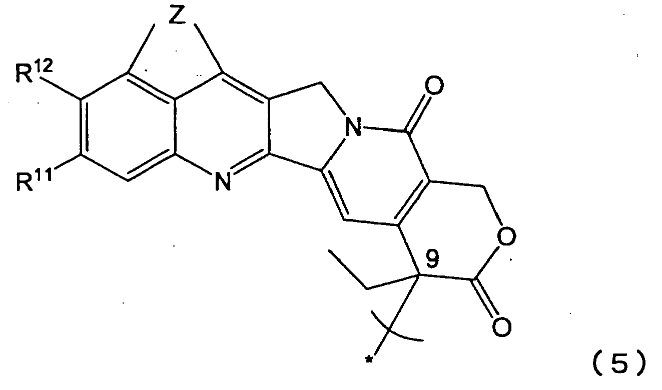 {wherein,
   * indicates a linkage site;
   R¹¹ and R¹² are each defined as in [13]; and
   Z represents -NH-C(=X)-N(R²¹)- or -N=C(R²²)-N(R²¹)-
   [wherein R²¹ represents a hydrogen atom or a C1-C10 alkyl group that may comprise
   one to three substituents selected from Group B described below:
   Group B: a C1-C6 alkoxy group, a hydroxy group, a halogen atom, an amino group, a mono-C1-C6 alkylamino group, a di-C1-C6 alkylamino group, a C3-C7 cycloalkyl group, a heterocycle, and an aryl ring (the aryl ring may comprise one to three substituents selected from the group consisting of a hydroxy group, a C1-C6 alkoxy group, a halogen atom, an amino group, a mono-C1-C6 alkylamino group, and a di-C1-C6 alkylamino group);
   R²² represents a hydrogen atom, an amino group, or a C1-C6 alkyl group that may comprise one to three substituents selected from Group C described below, a C1-C6 alkoxy group that may comprise one to three substituents selected from Group C described below, a C1-C6 alkylthio group that may comprise one to three substituents selected from Group C described below, a mono-C1-C6 alkylamino group that may comprise one to three substituents selected from Group C described below, or a di-C1-C6 alkylamino group that may comprise one to three substituents selected from Group C described below:
   Group C: a C1-C6 alkoxy group, a hydroxy group, a halogen atom, an amino group, a mono-C1-C6 alkylamino group, a di-C1-C6 alkylamino group, a C3-C7 cycloalkyl group, a heterocycle, and an aryl ring (the aryl ring may comprise one to three substituents selected from the group consisting of a hydroxy group, a C1-C6 alkoxy group, a halogen atom, an amino group, a mono-C1-C6 alkylamino group, and a di-C1-C6 alkylamino group); and
   X represents an oxygen atom or a sulfur atom]};
[15] the preventive or therapeutic agent for pancreatic cancer, ovarian cancer, or liver cancer of [14], wherein Y is a group represented by formula (6): 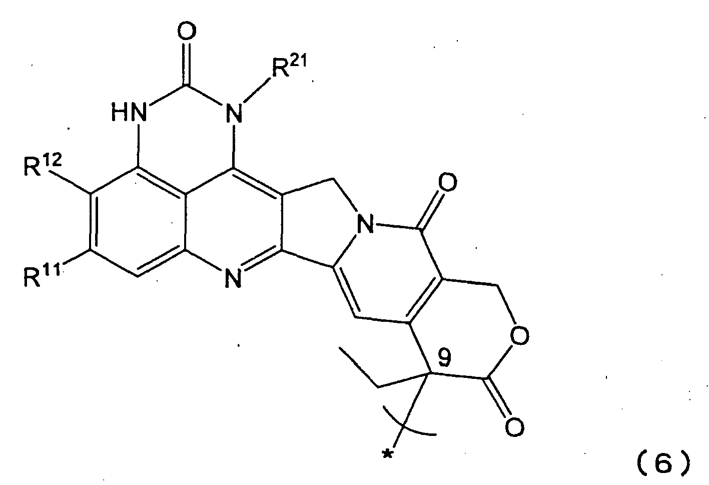 [wherein * indicates a linkage site; and
   R¹¹, R¹², and R²¹ are each defined as in [14]];
[16] the preventive or therapeutic agent for pancreatic cancer, ovarian cancer, or liver cancer of [15], wherein R¹¹ and R¹² are hydrogen atoms; and
   R²¹ is a hydrogen atom, or a C1-C8 alkyl group that may comprise a substituent selected from Group D described below:
   Group D: a C1-C3 alkoxy group, a hydroxy group, a halogen atom, an amino group, a mono-C1-C3 alkylamino group, a di-C1-C3 alkylamino group, a C3-C7 cycloalkyl group, a heterocycle, and an aryl ring (the aryl ring may comprise one to three substituents selected from the group consisting of a hydroxy group, a C1-C3 alkoxy group, and a halogen atom);
[17] the preventive or therapeutic agent for pancreatic cancer, ovarian cancer, or liver cancer of [15] or [16], wherein Y is a residue of a compound (Y-OH) comprising at least one alcoholic hydroxyl group, wherein the compound is selected from the group consisting of:
   a) (9S)-1-butyl-9-ethyl-9-hydroxy-1H,12H-pyrano[3",4":6',7']indolizino[1',2':6,5]pyrido[4,3,2-de]quinazoline-2,10,13(3H,9H,15H)-trione;
   b) (9S)-9-ethyl-9-hydroxy-1-[2-(4-morpholino)ethyl]-1H,12H-pyrano[3",4":6',7']indolizino[1',2':6,5]pyrido[4,3,2-de]quinazoline-2,10,13(3H,9H,15H)-trione;
   c) (9S)-1-[3-(dimethylamino)propyl]-9-ethyl-9-hydroxy-1H,12H-pyrano[3",4":6',7']indolizino[1',2':6,5]pyrido[4,3,2-de]quinazoline-2,10,13(3H,9H,15H)-trione;
   d) (9S)-9-ethyl-9-hydroxy-1-phenethyl-1H,12H-pyrano[3",4":6',7']indolizino[1',2':6,5]pyrido[4,3,2-de]quinazoline-2,10,13(3H,9H,15H)-trione;
   e) (9S)-9-ethyl-9-hydroxy-1-[2-(pyridin-2-yl)ethyl]-1H,12H-pyrano[3",4":6',7']indolizino[1',2':6,5]pyrido[4,3,2-de]quinazoline-2,10,13(3H,9H,15H)-trione;
   f) (9S)-9-ethyl-1-heptyl-9-hydroxy-1H,12H-pyrano[3",4":6',7']indolizino[1',2':6,5]pyrido[4,3,2-de]quinazoline-2,10,13(3H,9H,15H)-trione;
   g) (9S)-9-ethyl-9-hydroxy-1-propyl-1H,12H-pyrano[3",4":6',7']indolizino[1',2':6,5]pyrido[4,3,2-de]quinazoline-2,10,13(3H,9H,15H)-trione;
   h) (9S)-9-ethyl-9-hydroxy-1-[2-(pyridin-3-yl)ethyl]-1H,12H-pyrano[3",4":6',7']indolizino[1',2':6,5]pyrido[4,3,2-de]quinazoline-2,10,13(3H,9H,15H)-trione;
   i) (9S)-9-ethyl-9-hydroxy-1-(3-phenylpropyl)-1H,12H-pyrano[3",4":6',7']indolizino[1',2':6,5]pyrido[4,3,2-de]quinazoline-2,10,13(3H,9H,15H)-trione;
   j) (9S)-9-ethyl-9-hydroxy-1-(2-methylpropyl)-1H,12H-pyrano[3",4":6',7']indolizino[1',2':6,5]pyrido[4,3,2-de]quinazoline-2,10,13(3H,9H,15H)-trione;
   k) (9S)-9-ethyl-1-hexyl-9-hydroxy-1H,12H-pyrano[3",4":6',7']indolizino[1',2':6,5]pyrido[4,3,2-de]quinazoline-2,10,13(3H,9H,15H)-trione;
   l) (9S)-9-ethyl-9-hydroxy-1-pentyl-1H,12H-pyrano[3",4":6',7']indolizino[1',2':6,5]pyrido[4,3,2-de]quinazoline-2,10,13(3H,9H,15H)-trione;
   m) (9S)-9-ethyl-9-hydroxy-1-[2-(4-methoxyphenyl)ethyl]-1H,12H-pyrano[3",4":6',7']indolizino[1',2' :6,5]pyrido[4,3,2-de]quinazoline-2,10,13(3H,9H,15H)-trione;
   n) (9S)-1-benzyl-9-ethyl-9-hydroxy-1H,12H-pyrano[3",4":6',7']indolizino[1',2':6,5]pyrido[4,3,2-de]quinazoline-2,10,13(3H,9H,15H)-trione;
   o) (9S)-9-ethyl-9-hydroxy-1-(3-methylbutyl)-1H,12H-pyrano[3",4":6',7']indolizino[1';2':6,5]pyrido[4,3,2-de]quinazoline-2,10,13(3H,9H,15H)-trione;
   P) (9S)-1,9-diethyl-9-hydroxy-1H,12H-pyrano[3",4":6',7']indolizino[1',2':6,5]pyrido[4,3,2-de]quinazoline-2,10,13(3H,9H,15H)-trione;
   q) (9S)-1-[2-(4-chlorophenyl)ethyl]-9-ethyl-9-hydroxy-1H,12H-pyrano[3",4":6',7']indolizino[1',2':6,5]pyrido[4,3,2-de]quinazoline-2,10,13(3H,9H,15H)-trione;
   r) (9S)-9-ethyl-1-[2-(4-fluorophenyl)ethyl]-9-hydroxy-1H,12H-pyrano[3",4":6',7']indolizino[1',2':6,5]pyrido[4,3,2-de]quinazoline-2,10,13(3H,9H,15H)-trione;
   s) (9S)-9-ethyl-9-hydroxy-1-(1-methylethyl)-1H,12H-pyrano[3",4":6',7']indolizino[1',2':6,5]pyrido[4,3,2-de]quinazoline-2,10,13(3H,9H,15H)-trione; and
   t) (9S)-1-(3,3-dimethylbutyl)-9-ethyl-9-hydroxy-1H,12H-pyrano[3",4":6',7']indolizino[1',2':6,5]pyrido[4,3,2-de]quinazoline-2,10,13(3H,9H,15H)-trione;
[18] the preventive or therapeutic agent for pancreatic cancer, ovarian cancer, or liver cancer of [14], wherein Y is a group represented by formula (7): 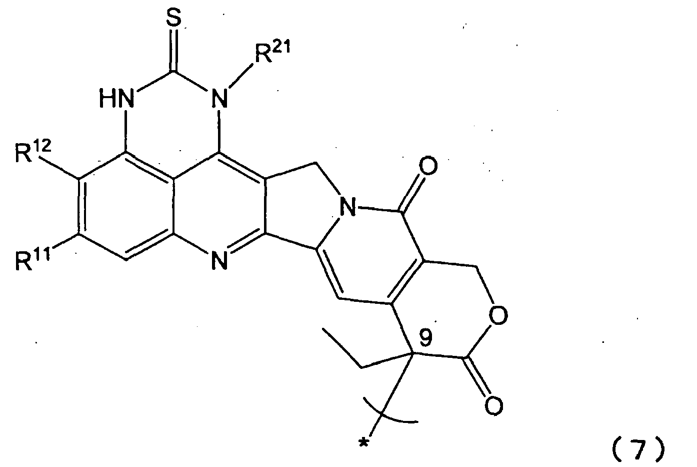 (wherein,
   * indicates a linkage site; and
   R¹¹, R¹², and R²¹ are each defined as in [14]);
[19] the preventive or therapeutic agent for pancreatic cancer, ovarian cancer, or liver cancer of [18], wherein R¹¹ and R¹² are hydrogen atoms; and
   R²¹ is a hydrogen atom, or a C1-C8 alkyl group that may comprise a substituent selected from Group D described below:
   Group D: a C1-C3 alkoxy group, a hydroxy group, a halogen atom, an amino group, a mono-C1-C3 alkylamino group, a di-C1-C3 alkylamino group, a C3-C7 cycloalkyl group, a heterocycle, and an aryl ring (the aryl ring may comprise one to three substituents selected from the group consisting of a hydroxy group; a C1-C3 alkoxy group, and a halogen atom);
[20] the preventive or therapeutic agent for pancreatic cancer, ovarian cancer, or liver cancer of [18] or [19], wherein Y is a residue of a compound (Y-OH) comprising at least one alcoholic hydroxyl group, wherein the compound is selected from the group consisting of:
   a) (9S)-9-ethyl-9-hydroxy-1-(3-methylbutyl)-1H,12H-pyrano[3",4":6',7']indolizino[1',2':6,5]pyrido[4,3,2-de]-quinazoline-2(3H)-thione-10,13 (9H,15H)-dione;
   b) (9S)-9-ethyl-9-hydroxy-1-phenethyl-1H,12H-pyrano[3",4":6',7']indolizino[1',2':6,5]pyrido[4,3,2-de]quinazoline-2(3H)-thione-10,13(9H,15H)-dione; and
   c) (9S)-9-ethyl-9-hydroxy-1-pentyl-1H,12H-pyrano[3",4":6',7']indolizino[1',2':6,5]pyrido[4,3,2-de]quinazoline-2(3H)-thione-10,13(9H,15H)-dione;
[21] the preventive or therapeutic agent for pancreatic cancer, ovarian cancer, or liver cancer of [14], wherein Y is a group represented by formula (8): 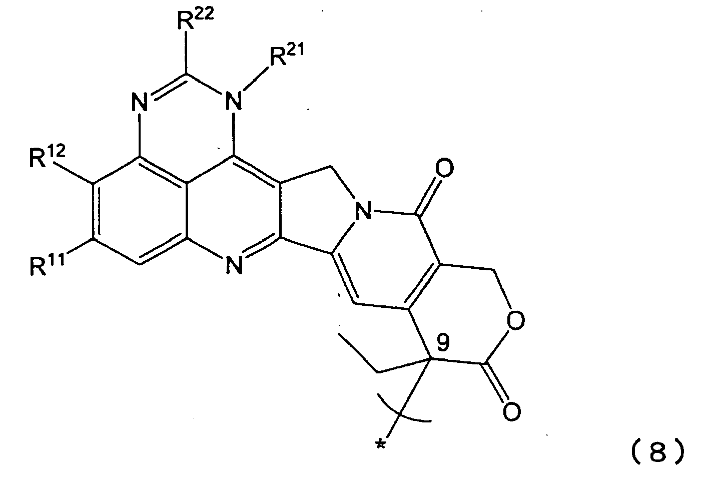 (wherein,
   * indicates a linkage site; and
   R¹¹, R¹², R²¹, and R²² are each defined as in [14]);
[22] the preventive or therapeutic agent for pancreatic cancer, ovarian cancer, or liver cancer of [21], wherein R¹¹ is a hydrogen atom;
   R¹² is a hydrogen atom or a C1-C3 alkyl group;
   R²¹ is a hydrogen atom, or a C1-C8 alkyl group that may comprise one to three substituents selected from Group D described below; and
   R²² is a hydrogen atom, an amino group, or a C1-C6 alkyl group that may comprise one to three substituents selected from Group D described below, a C1-C6 alkoxy group that may comprise one to three substituents selected from Group D described below, a C1-C6 alkylthio group that may comprise one to three substituents selected from Group D described below, a mono-C1-C6 alkylamino group that may comprise one to three substituents selected from Group D described below, or a di-C1-C6 alkyl amino group that may comprise one to three substituents selected from Group D described below:
   Group D: a C1-C3 alkoxy group, a hydroxy group, a halogen atom, an amino group, a mono-C1-C3 alkylamino group, a di-C1-C3 alkylamino group, a C3-C7 cycloalkyl group, a heterocycle, and an aryl ring (the aryl ring may comprise one to three substituents selected from the group consisting of a hydroxy group, a C1-C3 alkoxy group, and a halogen atom);
[23] the preventive or therapeutic agent for pancreatic cancer, ovarian cancer, or liver cancer of [21] or [22], wherein Y is a residue of a compound (Y-OH) comprising at least one alcoholic hydroxyl group, wherein the compound is selected from the group consisting of:
   a) (9S)-1-butyl-9-ethyl-9-hydroxy-1H,12H-pyrano[3",4":6',7']indolizino[1',2':6,5]pyrido[4,3,2-de]quinazoline-10,13(9H,15H)-dione;
   b) (9S)-9-ethyl-9-hydroxy-1-[2-(4-morpholino)ethyl]-1H,12H-pyrano[3",4":6',7']indolizino[1',2':6,5]pyrido[4,3,2-de]quinazoline-10,13(9H,15H)-dione;
   c) (9S)-9-ethyl-9-hydroxy-1-propyl-1H,12H-pyrano[3",4":6',7']indolizino[1',2':6,5]pyrido[4,3,2-de]quinazoline-10,13(9H,15H)-dione;
   d) (9S)-1-benzyl-9-ethyl-9-hydroxy-1H,12H-pyrano[3",4":6',7']indolizino[1',2':6,5]pyrido[4,3,2-de]quinazoline-10,13 (9H,15H)-dione;
   e) (9S)-9-ethyl-9-hydroxy-1-phenethyl-1H,12H-pyrano[3",4":6',7']indolizino[1',2':6,5]pyrido[4,3,2-de]quinazoline-10,13(9H,15H)-dione;
   f) (9S)-2,9-diethyl-9-hydroxy-1-phenethyl-1H,12H-pyrano[3",4":6',7']indolizino[1',2':6,5]pyrido[4,3,2-de]quinazoline-10,13(9H,15H)-dione;
   g) (9S)-9-ethyl-9-hydroxy-1-(3-phenylpropyl)-1H,12H-pyrano[3",4":6',7']indolizino[1',2':6,5]pyrido[4,3,2-de]quinazoline-10,13(9H,15H)-dione;
   h) (9S)-9-ethyl-9-hydroxy-1-(3-methylbutyl)-1H,12H-pyrano[3",4":6'7']mdolizino[1',2':6,5]pyrido[4,3,2-de]quinazoline-10,13(9H,15H)-dione;
   i) (9S)-2,9-diethyl-9-hydroxy-1-(3-methylbutyl)-1H,12H-pyrano[3",4":6',7']indolizino[1',2':6,5]pyrido[4,3,2-de]quinazoline-10,13(9H,15H)-dione;
   j) (9S)-2,9-diethyl-9-hydroxy-1-(2-methylpropyl)-1H,12H-pyrano[3",4":6',7']indolizino[1',2':6,5]pyrido[4,3,2-de]quinazoline-10,13(9H,15H)-dione;
   k) (9S)-9-ethyl-1-heptyl-9-hydroxy-1H,12H-pyrano[3",4":6',7']indolizino[1'2':6,5]pyrido[4,3,2-de]quinazoline-10,13(9H,15H)-dione;
   l) (9S)-9-ethyl-9-hydroxy-1-methyl-1H,12H-pyrano[3",4":6',7']indolizino[1',2':6,5]pyrido[4,3,2de]quinazoline-10,13(9H,15H)-dione;
   m) (9S)-9-ethyl-9-hydroxy-1-(2-methylpropyl)-1H,12H-pyrano[3",4":6',7']indolizino[1',2':6,5]pyrido[4,3,2-de]quinazoline-10,13(9H,15H)-dione;
   n) (9S)-9-ethyl-1-hexyl-9-hydroxy-1H,12H-pyrano[3",4":6',7']indolizino[1',2':6,5]pyrido[4,3,2-de] quinazoline-10,13(9H,15H)-dione;
   o) (9S)-9-ethyl-9-hydroxy-1-pentyl-1H,12H-pyrano[3",4":6',7']indolizino[1',2':6,5]pyrido[4,3,2-de]quinazoline-10,13(9H,15H)-dione;
   P) (9S)-1,9-diethyl-9-hydroxy-1H,12H-pyrano[3",4":6',7']indolizino[1',2':6,5]pyrido[4,3,2-de]quinazoline-10,13(9H,15H)-dione;
   q) (9S)-9-ethyl-9-hydroxy-1-[2-(4-methoxyphenyl)ethyl]-1H,12H-pyrano[3",4":6',7']indolizino[1',2':6,5]pyrido[4,3,2-de]quinazoline-10,13(9H,15H)-dione;
   r) (9S)-1-[2-(4-chlorophenyl)ethyl]-9-ethyl-9-hydroxy-1H,12H-pyrano[3",4":6',7']indolizino[1',2':6,5]pyrido[4,3,2-de]quinazoline-10,13(9H,15H)-dione;
   s) (9S)-9-ethyl-1-[2-(4-fluorophenyl)ethyl]-9-hydroxy-1H,12H-pyrano[3",4":6',7']indolizino[1',2':6,5]pyrido[4,3,2-de]quinazoline-10,13(9H,15H)-dione;
   t) (9S)-9-ethyl-1-[2-(4-fluorophenyl)ethyl]-9-hydroxy-2-methyl-1H,12H-pyrano[3",4":6',7']indolizino[1',2':6,5]pyrido[4,3,2-de]quinazoline-10,13(9H,15H)-dione;
   u) (9S)-9-ethyl-9-hydroxy-1-(1-methylethyl)-1H,12H-pyrano[3",4":6',7']indolizino[1',2':6,5]pyrido[4,3,2-de]quinazoline-10,13(9H,15H)-dione;
   v) (9S)-1-(3,3-dimethylbutyl)-9-ethyl-9-hydroxy-1H,12H-pyrano[3",4":6',7']indolizino[1',2':6,5]pyrido[4,3,2-de]quinazoline-10,13(9H,15H)-dione;
   w) (9S)-9-ethyl-9-hydroxy-2-methoxy-1-(3-methylbutyl)-1H,12H-pyrano[3",4":6',7']indolizino[1',2':6,5]pyrido[4,3,2-de]quinazoline-10,13(9H,15H)-dione;
   x) (9S)-2,9-diethyl-9-hydroxy-1-(3-methylbutyl)-1H,12H-pyrano[3",4":6',7']indolizino[1',2':6,5]pyrido[4,3,2-de]quinazoline-10,13(9H,15H)-dione;
   y) (9RS)-9-ethyl-9-hydroxy-4-methyl-1-pentyl-1H,12H-pyrano[3",4":6',7']indolizino[1',2':6,5]pyrido[4,3,2-de]quinazoline-10,13(9H,15H)-dione;
   z) (9S)-9-ethyl-9-hydroxy-1-(2-hydroxyethyl)-1H,12H-pyrano[3",4":6',7']indolizino[1',2':6,5]pyrido[4,3,2-de]quinazoline-10,13(9H,15H)-dione;
   aa) (9S)-9-ethyl-9-hydroxy-1-(2-hydroxyethyl)-2-methyl-1H,12H-pyrano[3",4":6',7']indolizino[1',2':6,5]pyrido[4,3,2-de]quinazoline-10,13(9H,15H)-dione;
   bb) (9S)-9-ethyl-9-hydroxy-2-methyl-1-pentyl-1H,12H-pyrano[3",4":6',7']indolizino[1',2':6,5]pyrido[4,3,2-de]quinazoline-10,13(9H,15H)-dione;
   cc) (9S)-2,9-diethyl-9-hydroxy-1-pentyl-1H,12H-pyrano[3",4":6',7']indolizino[1',2':6,5]pyrido[4,3,2-de]quinazoline-10,13(9H,15H)-dione;
   dd) (9S)-9-ethyl-9-hydroxy-1-pentyl-2-propyl-1H,12H-pyrano[3",4":6',7]indolizino[1',2':6,5]pyrido[4,3,2-de]quinazoline-10,13(9H,15H)-dione;
   ee) (9S)-9-ethyl-9-hydroxy-2-hydroxymethyl-1-pentyl-1H,12H-pyrano[3",4":6',7']indolizino[1',2':6,5]pyrido[4,3,2-de]quinazoline-10,13(9H,15H)-dione;
   ff) (9S)-9-ethyl-9-hydroxy-2-hydroxymethyl-1-(2-methylpropyl)-1H,12H-pyrano[3",4":6',7']indolizino[1',2':6,5]pyrido[4,3,2-de]quinazoline-10,13(9H,15H)-dione;
   gg) (9S)-9-ethyl-9-hydroxy-2-hydroxymethyl-1-(3-methylbutyl)-1H,12H-pyrano[3",4":6',7']indolizino[1',2':6,5]pyrido[4,3,2-de]quinazoline-10,13(9H,15H)-dione;
   hh) (9S)-2-chloromethyl-9-ethyl-9-hydroxy-1-(3-methylbutyl)-1H,12H-pyrano[3",4":6',7']indolizino[1',2':6,5]pyrido[4,3,2-de]quinazoline-10,13(9H,15H)-dione;
   ii) (9S)-2-aminomethyl-9-ethyl-9-hydroxy-1-pentyl-1H,12H-pyrano[3",4":6',7']indolizino[1',2':6,5]pyrido[4,3,2-de]quinazoline-10,13(9H,15H)-dione;
   jj) (9S)-9-ethyl-9-hydroxy-1-pentyl-2-trifluoromethyl-1H,12H-pyrano[3",4":6',7']indolizino[1',2': 6,5]pyrido[4,3,2-de]quinazoline-10,13(9H,15H)-dione;
   kk) (9S)-9-ethyl-9-hydroxy-1-(3-methylbutyl)-2-methylthio-1H,12H-pyrano[3",4":6',7']indolizino[1',2':6,5]pyrido[4,3,2-de]quinazoline-10,13(9H,15H)-dione;
   ll) (9S)-9-ethyl-2-ethylthio-9-hydroxy-1-(3-methylbutyl)-1H,12H-pyrano[3",4":6',7']indolizino[1',2':6,5]pyrido[4,3,2-de]quinazoline-10,13(9H,15H)-dione;
   mm) (9S)-2-(dimethylamino)-9-ethyl-9-hydroxy-1-(2-methylpropyl)-1H,12H-pyrano[3",4":6',7']ind olizino[1',2':6,5]pyrido[4,3,2-de]quinazoline-10,13(9H,15H)-dione; and
   nn) (9S)-2-(butylamino)-9-ethyl-9-hydroxy-1-(3-methylbutyl)-1H,12H-pyrano[3",4":6',7']indolizin o[1',2':6,5]pyrido[4,3,2-de]quinazoline-10,13(9H,15H)-dione;
[24] the preventive or therapeutic agent for pancreatic cancer, ovarian cancer, or liver cancer of [1], wherein the water-soluble prodrug represented by formula (1) is at least one prodrug selected from the group consisting of:
   (a) (9S)-9-ethyl-9-{[methyl-(2-methylamino-ethyl)-amino]-acetoxy}-1-pentyl-1H,12H-pyrano[3",4'':6',7']indolizino[1',2':6,5]pyrido[4,3,2-de]quinazoline-10,13(9H,15H)-dione;
   (b) (9S)-9-ethyl-9-(glycyl-sarcosyloxy)-1-pentyl-1H,12H-pyrano[3",4":6',7']indolizino[1',2':6,5]pyrido[4,3,2-de]quinazoline-10,13(9H,15H)-dione;
   (c) (9S)-9-{[(2-amino-ethyl)-methyl-amino]-acetoxy}-9-ethyl-1-pentyl-1H,12H-pyrano[3",4":6',7']indolizino[1',2':6,5]pyrido[4,3,2-de]quinazoline-10,13(9H,15H)-dione;
   (d) (9S)-9-ethyl-9-(sarcosyl-sarcosyloxy)-1-pentyl-1H,12H-pyrano[3",4":6',7']indolizino[1',2':6,5]pyrido[4,3,2-de]quinazoline-10,13(9H,15H)-dione;
   (e) (9S)-9-[2-(2-aminoethanesulfonyl)ethoxycarbonyloxy]-9-ethyl-1-pentyl-1H,12H-pyrano[3",4":6',7']indolizino[1',2':6,5]pyrido[4,3,2-de]quinazoline-10,13(9H,15H)-dione;
   (f) (aminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester;
   (g) (9S)-9-{2-[(R-2-amino-2-methoxycarbonyl)ethanesulfonyl]ethoxycarbonyloxy}-9-ethyl-1-pentyl -1H,12H-pyrano[3",4":6',7']indolizino[1',2':6,5]pyrido[4,3,2-de]quinazoline-10,13(9H,15H)-dione;
   (h) (9S)-9-{2-[(R-2-amino-2-ethoxycarbonyl)ethanesulfonyl]ethoxycarbonyloxy}-9-ethyl-1-pentyl-1H,12H-pyrano[3",4":6',7']indolizino[1',2':6,5]pyrido[4,3,2-de]quinazoline-10,13(9H,15H)-dione;
   (i) (9S)-9-ethyl-9-(*N*-methylalanyl-*N*-methylalanyloxy)-1-pentyl-1H,12H-pyrano[3",4":6',7']indolizino[1',2':6,5]pyrido[4,3,2-de]quinazoline-10,13(9H,15H)-dione; and
   (j) (9S)-9-ethyl-9-(sarcosyl-*N*-methylalanyloxy)-1-pentyl-1H,12H-pyrano[3",4":6',7']indolizino[1',2':6,5]pyrido[4,3,2-de]quinazoline-10,13(9H,15H)-dione;
[25] use of a compound represented by formula (9) in the production of a preventive or therapeutic agent for pancreatic cancer, ovarian cancer, or liver cancer, comprising a water-soluble prodrug represented by formula (2), a pharmaceutically acceptable salt thereof, or a hydrate or solvate of the prodrug or pharmaceutically acceptable salt: 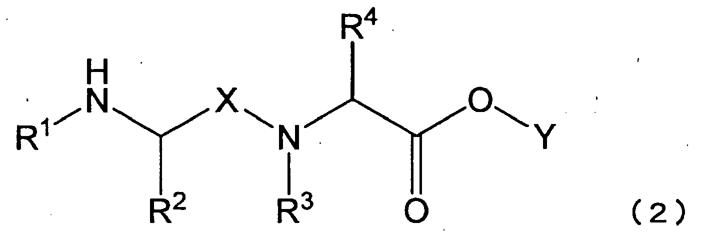 [wherein,
   X represents C=O or a C1-C3 alkylene group;
   Y represents a residue of a compound represented by Y-OH which comprises an alcoholic hydroxyl group, wherein said Y-OH is a camptothecin, a taxane, or an anticancer nucleotide;
   R¹ represents a hydrogen atom or a C1-6 alkyl group;
   R² and R⁴ each independently represents a hydrogen atom, a C1-6 alkyl group, or an amino acid side chain; and
   R³ represents a C1-C6 alkyl group, 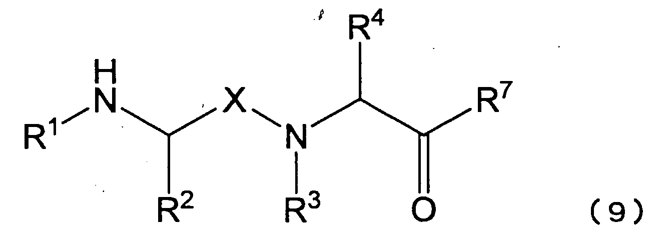 [wherein,
   X, R¹, R², R³, and R⁴ are defined as in the above-mentioned formula (2);
   the nitrogen atom where R¹ binds to may be protected with a protecting group, and
   R⁷ represents a halogen atom or a group represented by OR⁸ (wherein R⁸ represents a hydrogen atom or a C1-C6 alkyl group)]; and
[26] a method for preventing or treating pancreatic cancer, ovarian cancer or liver cancer, which comprises the step of administering an effective dose of a water-soluble prodrug represented by formula (1), or a pharmaceutically acceptable salt, or a hydrate or solvate of the prodrug or pharmaceutically acceptable salt to a patient in need of a prevention or treatment of pancreatic cancer, ovarian cancer, or liver cancer: 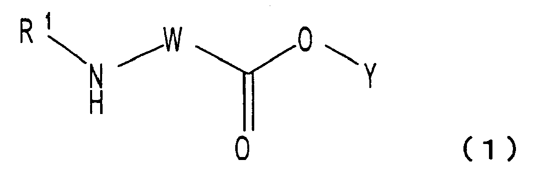 (wherein,
   R¹ represents a hydrogen atom or a C1-C6 alkyl group;
   W represents a divalent group comprising a tertiary amino group or a divalent group comprising a sulfonyl group;
   Y represents a residue of a compound represented by Y-OH comprising an alcoholic hydroxyl group, wherein said Y-OH is a camptothecin, a taxane, or an anticancer nucleotide).

As used herein, the term "alkyl group" refers to a monovalent group, which is derived by removing a single hydrogen atom from an aliphatic hydrocarbon, and has a partial assembly of hydrocarbyl or hydrocarbon structure comprising hydrogen and carbon atoms, and does not contain a heteroatom or an unsaturated carbon-carbon bond in its backbone. The alkyl group may have a straight-chain or branched-chain structure.

The term "C1-C3 alkyl group" refers to an alkyl group with 1 to 3 carbon atoms, the term "C 1-C6 alkyl group" refers to an alkyl group with 1 to 6 carbon atoms, the term "C 1-C8 alkyl group" refers to an alkyl group with 1 to 8 carbon atoms, and the term "C1-C10 alkyl group" refers to an alkyl group with 1 to 10 carbon atoms.

Specific examples of the alkyl group include a methyl group, ethyl group, *n*-propyl group, *i*-propyl group, *n*-butyl group, *sec*-butyl group, *t*-butyl group, isobutyl group, pentyl group, isopentyl group, 2,3-dimethylpropyl group, hexyl group, 2,3-dimethylhexyl group, 1,1-dimethylpentyl group, heptyl group, and octyl group.

As used herein, the term "alkylene group" refers to a divalent group derived by removing a second hydrogen atom from the alkyl group defined above, and examples of the alkylene group preferably include a C1-C3 alkylene group, and more preferably a C1-C2 alkylene group. Specific examples of the alkylene group include a methylene group, 1,2-ethylene group, 1,1-ethylene group, 1,3-propylene group, tetramethylene group, pentamethylene group, and hexamethylene group.

As used herein; the term "alkoxy group" refers to an -O-R' group, wherein R' is the alkyl group defined above. Examples of the "C1-C6 alkoxy group" include a methoxy group, ethoxy group, propoxy group, isopropoxy group, butoxy group, isobutoxy group, *tert*-butoxy group, pentoxy group, 3-methylbutoxy group, and 2,2-dimethylpropoxy group.

As used herein, the term "alkylthio group" refers to an -S-R' group, in which R' is the alkyl group defined above. Examples of the "C1-C8 alkylthio group" include a methylthio group, ethylthio group, propylthio group, butylthio group, pentylthio group, hexylthio group, heptylthio group, and octylthio group.

As used herein, the term "hydroxy group" refers to an HO- group.

As used herein, the term "halogen atom" refers to a fluorine atom, chlorine atom, bromine atom, or iodine atom.

As used herein, the term "amino group" refers to an NH₂- group, and includes amino groups protected with formyl, acetyl, trityl, *tert*-butoxycarbonyl, benzyl, benzyloxycarbonyl, and such protecting groups that are well known in the art. Among the amino groups, NH₂- is preferred.

As used herein, the term "monoalkylamino group" refers to an -NH-R' group, wherein R' is the alkyl group defined above, and includes amino groups that are protected by substituting the hydrogen atom on the nitrogen atom with formyl, acetyl, trityl, *tert*-butoxycarbonyl, benzyl, benzyloxycarbonyl, and such groups that are well known in the art. Examples of the "mono-C1-C6 alkylamino group" preferably include an *N*-methylamino group, *N*-ethylamino group, *N*-propylamino group, *N*-isopropylamino group, *N*-butylamino group, *N*-(1-methylpropyl)amino group, *N*-(2-methylpropyl)amino group, and *N*-pentylamino group, and more preferably include *N*-ethylamino group, *N*-propylamino group, and *N*-butylamino group.

As used herein, the term "dialkylamino group" refers to an -NR'R" group, wherein R' and R" each independently represents an alkyl group defined above. Examples of the "di-C1-C6 alkylamino group" preferably include an *N*,*N-*dimethylamino group, *N*,*N-*diethylamino group, *N*,*N-*dipropylamino group, *N*,*N*-diisopropylamino group, *N*,*N*-dibutylamino group, *N*-methyl-*N*-ethylamino group, and *N*-methyl-*N*-propylamino group, and more preferably include an *N*,*N*-dimethylamino group and *N*,*N*-diethylamino group.

As used herein, the term "tertiary amino group" refers to a group in which all of the hydrogens of an amino group are substituted.

As used herein, the term "sulfonyl group" refers to a group represented by -SO₂-.

As used herein, the term "alcoholic residue" refers to the portion represented by Y in the alcohol represented by Y-OH.

As used herein, the term "C3-C7 cycloalkyl group" refers to a 3- to 7-membered ring that does not comprise any heteroatom in the ring. Examples of the "cycloalkyl group" preferably include a cyclopropyl group, cyclobutyl group, cyclopentyl group, cyclohexyl group, and cycloheptyl group, and more preferably include a cyclopentyl group and cyclohexyl group.

As used herein, the term "heterocycle" refers to a 3- to 10-membered ring comprising one or more heteroatoms selected from N, S, and O. Preferred examples of such include an oxazolyl group, thiazolyl group, 4,5-dihydrooxazolyl group, 4,5-dihydrothiazolyl group, furyl group, pyrolyl group, thienyl group, imidazolyl group, triazolyl group, tetrazolyl group, pyridyl group, pyrazinyl group, pyrimidinyl group, triazinyl group, oxadiazolyl group, thiadiazolyl group, pyrrolidinyl group, tetrahydrothienyl group, tetrahydrofuryl group, morpholinyl group, piperidyl group, piperazinyl group, and 1-methylpiperazinyl group, and more preferably include an imidazolyl group, pyridyl group, morpholinyl group, and pyrrolidinyl group.

As used herein, the term "aryl ring" refers to an aromatic carbocyclic group, or more specifically a 6- to 10-membered aromatic ring or a partially aromatic ring, and examples include phenyl, naphthyl, and tetrahydronaphthyl rings, preferably phenyl and naphthyl rings, and most preferably phenyl rings.

As used herein, the term "phenyl-C1-C8 alkyl group" refers to a group in which one of the hydrogen atoms of the C1-C8 alkyl group is substituted with a phenyl group.

As used herein, the term "heterocyclic-C1-C8 alkyl group" refers to a group in which one of the hydrogen atoms of the C1-C8 alkyl group is substituted with a heterocycle.

As used herein, the term "alkoxyphenyl C1-C8 alkyl group" refers to a group in which one of the hydrogen atoms of the C1-C8 alkyl group is substituted with an alkoxyphenyl group. The term "alkoxyphenyl group" refers to a group in which one of the hydrogen atoms of the phenyl group is substituted with an alkoxy group.

As used herein, the term "halogen phenyl C1-C8 alkyl group" refers to a group in which one of the hydrogen atoms of the C1-C8 alkyl group is substituted with a halogen atom.

The term "pharmaceutically acceptable salt" refers to a common salt of the water-soluble prodrug represented by formula (1), which is formed with an appropriate nontoxic organic or inorganic acid, or an organic or inorganic base, and which maintains the prodrug's biological efficacy and characteristics.

Examples of the salt with an acid include those derived from inorganic acids such as hydrochloric acid, hydrobromic acid, hydroiodic acid, sulfuric acid, sulfamic acid, phosphoric acid, and nitric acid; and those derived from organic acids such as p-toluenesulfonic acid, salicylic acid, methanesulfonic acid, oxalic acid, succinic acid, citric acid, malic acid, lactic acid, and fumaric acid.

Examples of the salt with a base include those derived from potassium hydroxide, sodium hydroxide, ammonium hydroxide, and quaternary ammonium hydroxide such as tetramethylammonium hydroxide.

The water-soluble prodrug of the present invention may absorb moisture, adsorb water, or form hydrates when it is left to stand in the atmosphere, and such hydrates are also included in this invention.

Furthermore, the water-soluble prodrug of the present invention may absorb certain other types of solvents to form solvates, and such solvates are also included in this invention.

Examples of the "amino acid side chain" as used herein include naturally occurring amino acid side chains and non-naturally occurring amino acid side chains.

Examples of the "naturally occurring amino acid side chain" are preferably side chains of naturally occurring amino acids such as a methyl group, isopropyl group, 2-methylpropyl group, 1-methylpropyl group, benzyl group, indol-3-ylmethyl group, 2-(methylthio)ethyl group, 4-aminobutyl group, and 3-aminopropyl group, and more preferably side chains of naturally occurring lipophilic amino acids such as a methyl group, 2-methylpropyl group, benzyl group, and indol-3-ylmethyl group.

Examples of the "non-naturally occurring amino acid side chain" are preferably C5-C12 alkyl groups, cycloalkylmethyl groups, substituted or unsubstituted arylmethyl groups, (cycloalkylthio)methyl groups, and alkylthio-(CH₂)ᵣ- in which r is an integer of 1 or 2.

Examples of the "C5-C12 alkyl group" are straight-chain or branched-chain alkyl groups comprising 5 to 12 carbon atoms; and more preferably C8-C12 straight-chain alkyl groups such as an *n*-octyl group, nonyl group, decyl group, undecyl group, and dodecyl group.

Examples of "alkylthio-(CH₂)ᵣ-" are alkylthiomethyl groups or alkylthioethyl groups comprising a straight or branched alkyl chain containing 2 to 10 carbon atoms, such as an ethylthiomethyl group, ethylthioethyl group, *n*-propylthiomethyl group, *n*-butylthiomethyl group, *n*-pentylthiomethyl group, *n*-octylthiomethyl group, *n*-nonylthiomethyl group, *n*-decylthiomethyl group, and *tert*-butylthiomethyl group; and more preferably an ethylthioethyl group, *n*-propylthiomethyl group, and *n*-butylthiomethyl group.

Examples of the "substituted or unsubstituted arylmethyl group" preferably include a 4-phenylbenzyl group, naphtho-2-ylmethyl group, [4-(4-hydroxyphenoxy)phenyl]methyl group, and (4-lower-alkoxyphenyl)methyl group (the term "lower-alkoxy" refers to a straight or branched alkyl chain containing 1 to 6 carbon atoms, and preferred examples include a methoxy group, ethoxy group, propoxy group, butoxy group, and isopropoxy group). The most preferred embodiments of the "substituted or unsubstituted arylmethyl group" include a 4-phenylbenzyl group, naphtho-2-ylmethyl group, (4-methoxyphenyl)methyl group, and [4-(4-hydroxyphenoxy)phenyl]methyl group.

As used herein, the term "insoluble compound" refers to all compounds insoluble in water, and examples are compounds whose solubility in distilled water is preferably 1 mg/mL or less, more preferably 0.1 mg/mL or less (the solubility is pursuant to "Japanese Pharmacopoeia, 14th edition, Generic Rule 23"). Examples of such compounds include camptothecins, taxanes, and anticancer nucleotides.

As used herein, the term "solubilizing side chain" refers to a group that binds to "Y", or for example in equation (1), it refers to "R¹-NH-W-CO-O-".

As used herein, the term "active form" refers to a compound (Y-O-) that is given by hydrolysis of the water-soluble prodrug, a pharmaceutically acceptable salt thereof, or a hydrate or solvate of the prodrug or pharmaceutically acceptable salt.

The term "taxanes" in the present invention refers to taxol [Front. Biotechnol. Pharm. (2000), 1, 336-348], taxotere [J. Med. Aromat. Plant Sci. (2001), 22/4A-23/1A 4-5], IDN 5109 [Chirality, (2000), 12(5/6), 431-441], BMS 188797 [Clinical Cancer Research. 5 (suppl.), 3859, Nov 1999], and BMS184476 [J. Clinical Oncology19: 2493-2503, 1 May 2001].

As used herein, the term "camptothecins" [(a) Cancer Chemotherapy and Biotherapy: Principle and Practice, 2nd edition, Lippincott-Ravenmeans, p.463-484, (b) Biochim. Biophys. Acta (1998), 1400(1-3), 107-119] refers to any compound comprising a camptothecin backbone, such as camptothecin, SN-38, 9-aminocamptothecin, 9-nitrocamptothecin, and BN-80915 [Anti-cancer Drugs (2001), 12(1), 9-19].

As used herein, the term "anticancer nucleosides" refers to cytidine derivatives [Cancer Chemotherapy and Biotherapy: Principle and Practice, 2nd edition, Lippincott-Ravenmeans, p.213-233] such as DFDC (gemcitabine), DMDC [Clin. Cancer Res. (2000), 6(6), 2288-2294], FMDC [Curr. Opin. Invest. Drugs (PharmaPress Ltd.) (2000), 1(1), 135-140], Ara-C, decitabine, [IDrugs (2000), 3(12), 1525-1533], troxacitabine [Clin. Cancer Res. (2000), 6(4), 1574-1588], 2'-cyano-2'-deoxycytidine (CNDAC), 3'-ethynylcytidine (TAS106) [Jpn. J. Cancer Res. (2001), 92(3), 343-351], 5-fluoro-5'-deoxycytidine [Bioorg. Med. Chem. Lett., (2000), 8, 1697-1706], and 5-vinyl-5'-deoxycytidine, or adenosine derivatives [Cancer Chemotherapy and Biotherapy: Principle and Practice, 2nd edition, Lippincott-Ravenmeans, p.235-252] such as fludarabine, and cladribine.

As used herein, the term "cell proliferation disorder" refers to a disorder caused by a defect in the intracellular signal transduction system, or in the signal transduction mechanism of a certain protein, and examples include those that cause cancer.

Among the prodrugs of the present invention represented by formula (1), examples of preferred compounds are the following.

Among the water-soluble prodrugs represented by formula (1), the compounds represented by formula (2) or (3) are preferred.

In the compounds represented by formula (2), R¹ is preferably a hydrogen atom, methyl group or ethyl group; and more preferably a hydrogen atom or methyl group; and particularly preferably a hydrogen atom.

X is preferably a carbonyl group or methylene group, and is more preferably a carbonyl group.

R² is preferably a hydrogen atom or methyl group.

R⁴ is preferably a hydrogen atom or methyl group.

R³ is preferably a C1-C3 alkyl group, and is more preferably a methyl group or ethyl group, and particularly preferably a methyl group.

Examples of preferred combinations of X, and R¹ to R⁴ that constitute the solubilizing side chain portion of the compound represented by formula (2) are shown below, but the present invention is not to be construed as being limited thereto.

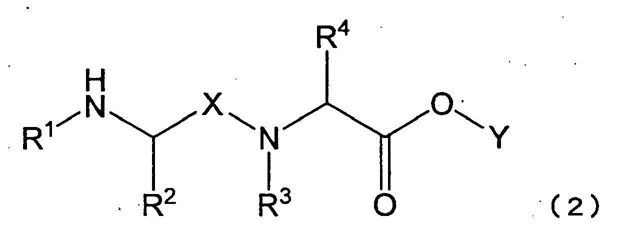

**Table 1**

| | R¹ | R² | R³ | R⁴ | X |
|---|---|---|---|---|---|
| 1 | H | H or -CH₃ | -CH₃ | H or -CH₃ | C=O |
| 2 | H | H or -CH₃ | -C₂H₅ | H or -CH₃ | C=O |
| 3 | -CH₃ | H or -CH₃ | -CH₃ | H or -CH₃ | C=O |
| 4 | -CH₃ | H or -CH₃ | -C₂H₅ | H or -CH₃ | C=O |
| 5 | -C₂H₅ | H or -CH₃ | -CH₃ | H or -CH₃ | C=O |
| 6 | -C₂H₅ | H or -CH₃ | -C₂H₅ | H or -CH₃ | C=O |
| 7 | H | H or -CH₃ | -CH₃ | H or -CH₃ | -CH₂- |
| 8 | H | H or -CH₃ | -C₂H₅ | H or -CH₃ | -CH₂- |
| 9 | -CH₃ | H or -CH₃ | -CH₃ | H or -CH₃ | -CH₂- |
| 10 | -CH₃ | H or -CH₃ | -C₂H₅ | H or -CH₃ | -CH₂- |
| 11 1 | -C₂H₅ | H or -CH₃ | -CH₃ | H or -CH₃ | -CH₂- |
| 12 | -C₂H₅ | H or -CH₃ | -C₂H₅ | H or -CH₃ | -CH₂- |

Within Table 1, examples of compounds of formula (2) are preferably those that comprise solubilizing side chains included in reference numbers 1, 2, 3, and 4, and more preferably those that comprise solubilizing side chains included in reference numbers 1 and 2.

If such a solubilizing side chain is present, even if Y-OH is an insoluble compound, it can be converted into a compound having good water solubility. For example, such a water-soluble prodrug can exist stably for a long period of time in a solution at pH4 or lower; but when at pH5 or higher, and particularly when at physiological conditions of pH7 to 8, the active form derived from the secondary or tertiary alcoholic hydroxyl group can be dissociated quantitatively and rapidly within a short period of time.

In the compound represented by formula (3), R¹ is preferably a hydrogen atom, methyl group, or ethyl group; and more preferably a hydrogen atom or methyl group; and particularly preferably a hydrogen atom.

In equation (3), n is preferably in the range of 1 to 3, and is more preferably 1.

When n is 1, R⁵ is preferably a hydrogen atom or -COOR⁶ (R⁶ is a C1-C3 alkyl group), and more preferably a hydrogen atom, -COOCH₃, or -COOC₂H₅.

When n is in the range of 2 to 6, R⁵ is preferably a hydrogen atom.

Examples of preferred combinations of n, R¹, and R⁵ that constitute the solubilizing side chain portion of such a compound represented by formula (3) are shown below, but the present invention is not to be construed as being limited thereto.

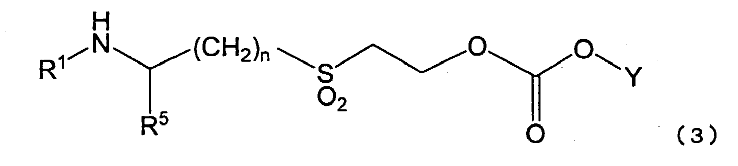

**Table 2**

| | R¹ | n | R⁵ |
|---|---|---|---|
| 1 | H | 1 | H |
| 2 | H | 1 | -CO₂CH₃ |
| 3 | H | 1 | -CO₂C₂H₅ |
| 4 | -CH₃ | 1 | H |
| 5 | -CH₃ | 1 | -CO₂CH₃ |
| 6 | -CH₃ | 1 | -CO₂C₂H₅ |
| 7 | -C₂H₅ | 1 | H |
| 8 | -C₂H₅ | 1 | -CO₂CH₃ |
| 9 | -C₂H₅ | 1 | -CO₂C₂H₅ |
| 10 | H | 2 | H |
| 11 | H | 2 | -CO₂CH₃ |
| 12 | H | 2 | -CO₂C₂H₅ |
| 13 | -CH₃ | 2 | H |
| 14 | -CH₃ | 2 | -CO₂CH₃ |
| 15 | -CH₃ | 2 | -CO₂C₂H₅ |
| 16 | -C₂H₅ | 2 | H |
| 17 | -C₂H₅ | 2 | -CO₂CH₃ |
| 18 | -C₂H₅ | 2 | -CO₂C₂H₅ |
| 19 | H | 3 | H |
| 20 | H | 3 | -CO₂CH₃ |
| 21 | H | 3 | -CO₂C₂H₅ |
| 22 | -CH₃ | 3 | H |
| 23 | -CH₃ | 3 | -CO₂CH₃ |
| 24 | -CH₃ | 3 | -CO₂C₂H₅ |
| 25 | -C₂H₅ | 3 | H |
| 26 | -C₂H₅ | 3 | -CO₂CH₃ |
| 27 | -C₂H₅ | 3 | -CO₂C₂H₅ |

Within Table 2, examples of compounds of formula (3) that comprise a solubilizing side chain are preferably those of reference numbers 1, 2, 3, 4, 5, and 6, and more preferably those of reference numbers 1, 3, and 4.

If such a solubilizing side chain is present, even if Y-OH is an insoluble compound, it can be converted into a compound having good water solubility. For example, the water-soluble prodrug can exist stably for a long period of time in a solution at pH4 or lower; but when at pH5 or higher, and particularly when at physiological conditions of pH7 to 8, the active form derived from the secondary or tertiary alcoholic hydroxyl group can be dissociated quantitatively and rapidly within a short period of time.

In the present invention, Y in formula (1) is a residue of a compound represented by Y-OH which has an alcoholic hydroxyl group.

The hydroxyl group of Y-OH is preferably a secondary or tertiary alcoholic hydroxyl group. Such Y-OH may be an insoluble compound, but even if it is insoluble, it will show good water solubility upon attachment of an aforementioned group. Furthermore, the water-soluble prodrug can exist stably for a long period of time in a solution at pH4 or lower; but when at pH5 or higher, and particularly when at physiological conditions of pH7 to 8, the active form (Y-O-) derived from the alcoholic hydroxyl group can be dissociated quantitatively and rapidly within a short period of time.

Y-OH included in the aforementioned formula of the present invention that may be attached with a solubilizing group is not particularly limited as long as it is a compound comprising an alcoholic hydroxyl group, preferably a secondary or tertiary alcoholic hydroxyl group, and more preferably a tertiary alcoholic hydroxyl group.

Examples of such compounds are preferably camptothecins, taxanes, and anticancer nucleotides.

### Camptothecins

Herein, examples of groups derived from camptothecins include the groups represented by Y shown below.

The aforementioned Y is a group represented by formula (4): 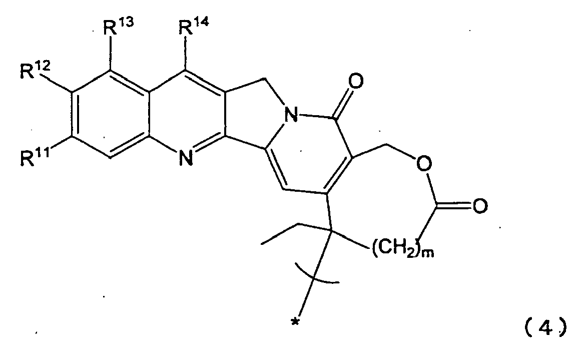 wherein,
* indicates a linkage site;
m is either 0 or 1;
R¹¹ represents a hydrogen atom, halogen atom, or C1-C6 alkyl group;
R¹² represents a hydrogen atom, halogen atom, C1-C6 alkyl group, or hydroxyl group;
R¹³ represents a hydrogen atom, amino group, nitro group, or (dimethylamino)methyl group;
R¹⁴ represents a hydrogen atom, C1-C6 alkyl group, (4-methylpiperazinyl)methyl group, or (*tert*-butoxyimino)methyl group; and
R¹³ and R¹⁴, and R¹¹ and R¹², respectively, may be linked to each other to form a 5- or 6-membered ring, wherein the 5- or 6-membered ring may comprise 1 to 2 heteroatoms, and may comprise 1 to 3 substituents selected from Group A described below, wherein the substituents of said Group A may further comprise 1 to 3 substituents selected from Group B described below:
Group A: a C1-C10 alkyl group, amino group, mono-C1-C8 alkylamino group, di-C1-C8 alkylamino group, C1-C8 alkoxy group, C1-C8 alkylthio group, and group represented by X= (wherein X represents an oxygen atom or sulfur atom);
Group B: a C1-C6 alkoxy group, hydroxy group, halogen atom, amino group, mono-C1-C6 alkylamino group, di-C1-C6 alkylamino group, C3-C7 cycloalkyl group, heterocycle, and aryl ring (which may comprise 1 to 3 substituents selected from the group consisting of a hydroxy group, C1-C6 alkoxy group, halogen atom, an amino group, mono-C1-C6 alkylamino group, and di-C1-C6 alkylamino group).

In the group represented by formula (4), the stereochemistry of the carbon, to which the oxygen atom derived from the alcoholic hydroxyl group binds, preferably has S configuration.

Herein, a preferred embodiment of the compounds represented by formula (4) includes compounds in which R¹¹ is preferably a hydrogen atom, R¹² is preferably a hydrogen atom or hydroxyl group, R¹³ represents a hydrogen atom or (dimethylamino)methyl group, and R¹⁴ represents a hydrogen atom or ethyl group.

Examples of compounds represented by formula (4) include the following compounds:
4(S)-ethyl-4-hydroxy-1H-pyrano[3',4':6,7]indolizino[1,2-b]quinoline-3,14(4H,12H)-dione(camp tothecin);
9-aminocamptothecin;
9-nitrocamptothecin;
5(R)-ethyl-9,10-difluoro-1,4,5,13-tetrahydro-5-hydroxy-3H,15H-oxepino[3',4':6,7]indolizino[1, 2-b]quinoline-3,15-dione (BN-80915); and
7-ethyl-10-hydroxycamptothecin (SN-38).

Another preferred embodiment of the compounds represented by formula (4) includes compounds in which the aforementioned Y is represented by generic formula (5): 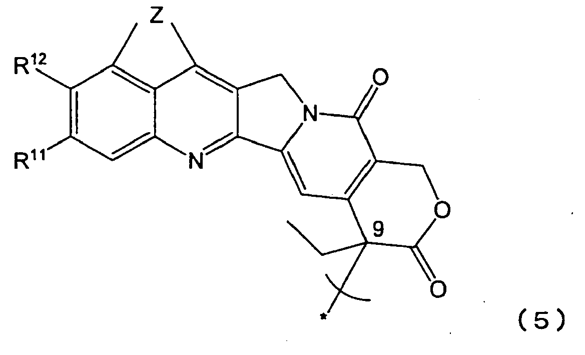 wherein,
* indicates a linkage site;
R¹¹ and R¹² have the same meaning as R¹¹ and R¹² defined in formula (4), respectively; and
Z represents -NH-C(=X)-N(R²¹)- or -N=C(R²²)-N(R²¹)-.

Herein,
R²¹ represents a hydrogen atom or a C1-C10 alkyl group that may comprise 1-3 substituents selected from Group B described below:
Group B: a C1-C6 alkoxy group, hydroxy group, halogen atom, amino group, mono-C1-C6 alkylamino group, di-C1-C6 alkylamino group, C3-C7 cycloalkyl group, heterocycle and an aryl ring (which may comprise 1 to 3 substituents selected from the group consisting of a hydroxy group, C1-C6 alkoxy group, halogen atom, an amino group, mono-C1-C6 alkylamino group, and di-C1-C6 alkylamino group);
R²² represents a hydrogen atom, amino group, or a C1-C6 alkyl group that may comprise 1 to 3 substituents selected from Group C described below, a C1-C6 alkoxy group that may comprise 1 to 3 substituents selected from Group C described below, a C1-C6 alkylthio group that may comprise 1 to 3 substituents selected from Group C described below, a mono-C1-C6 alkylamino group that may comprise 1 to 3 substituents selected from Group C described below, or a di-C1-C6 alkylamino group that may comprise 1 to 3 substituents selected from Group C described below:
Group C: a C1-C6 alkoxy group, hydroxy group, halogen atom, amino group, a mono-C1-C6 alkylamino group, a di-C1-C6 alkylamino group, C3-C7 cycloalkyl group, heterocycle, and aryl ring (which may comprise 1 to 3 substituents selected from the group consisting of a hydroxy group, C1-C6 alkoxy group, halogen atom, amino group, mono-C1-C6 alkylamino group, and di-C1-C6 alkylamino group); and,
X represents an oxygen atom or sulfur atom.

Furthermore, preferred examples of compounds in which Y is represented by formula (5) are compounds that comprise Y represented by formula (6), (7), or (8).

The aforementioned Y is a water-soluble prodrug represented by - formula (6) below. 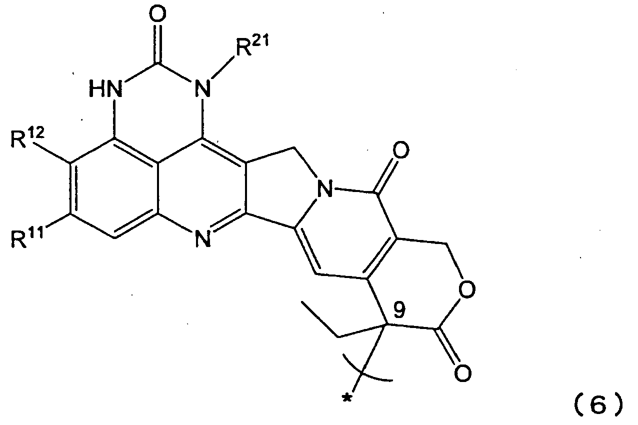 R¹¹, R¹², and R²¹ are defined as in formulae (4) and (5).

In formula (6), R¹¹ and R¹² are preferably hydrogen atoms.

R²¹ is preferably a hydrogen atom, or a C1-C8 alkyl group that may comprise a substituent selected from Group D shown below:
Group D: a C1-C3 alkoxy group, hydroxy group, halogen atom, amino group, mono-C1-C3 alkylamino group, di-C1-C3 alkylamino group, C3-C7 cycloalkyl group, heterocycle, and aryl ring (which may comprise 1 to 3 substituents selected from the group consisting of a hydroxy group, C1-C3 alkoxy group, and halogen atom).
R²¹ is more preferably a C1-C8 alkyl group, phenyl-C1-C8 alkyl group, heterocyclic-C1-C8 alkyl group, alkoxyphenyl C1-C8 alkyl group, or halogenated phenyl C1-C8 alkyl group.
R²¹ is even more preferably a methyl group, ethyl group, *n*-propyl group, 1-methylethyl group, *n*-butyl group, 1,1-dimethylethyl group, 2-methylpropyl group, 2,2-dimethylpropyl group, *n*-pentyl group, 3-methylbutyl group, 2-*n*-hexyl group, 3,3-dimethylbutyl group, *n*-heptyl group, *n*-octyl group, benzyl group, phenethyl group, 2-(dimethylamino)ethyl group, 2-(4-moipholino)ethyl group, 3-(dimethylamino)propyl group, 2-(pyridin-2-yl)ethyl group, 2-(pyridin-3-yl)ethyl group, 2-(4-methoxyphenyl)ethyl group, 2-(4-chlorophenyl)ethyl group, 2-(4-fluorophenyl)ethyl group, or 3-phenylpropyl group.

Examples of such compounds represented by formula (6) include those in which Y represents a residue of a compound (Y-OH) comprising at least one alcoholic hydroxyl group (for example at position 9), wherein the compound is selected from the group consisting of:
a) (9S)-1-butyl-9-ethyl-9-hydroxy-1H,12H-pyrano[3",4":6',7']indolizino[1',2':6,5]pyrido [4,3,2-de] quinazoline-2,10,13(3H,9H,15H)-trione;
b) (9S)-9-ethyl-9-hydroxy-1-[2-(4-morpholino)ethyl]-1H,12H-pyrano[3",4":6',7']indolizino [1',2':6,5]pyrido[4,3,2-de]quinazoline-2,10,13(3H,9H,15H)-trione;
c) (9S)-1-[3-(dimethylamino)propyl]-9-ethyl-9-hydroxy-1H,12H-pyrano[3",4":6',7']indolizino [1',2':6,5]pyrido[4,3,2-de]quinazoline-2,10,13(3H,9H,15H)-trione;
d) (9S)-9-ethyl-9-hydroxy-1-phenethyl-1H,12H-pyrano[3",4":6',7']indolizino[1',2':6,5]pyrido [4,3,2-de]quinazoline-2,10,13(3H,9H,15H)-trione;
e) (9S)-9-ethyl-9-hydroxy-1-[2-(pyridin-2-yl)ethyl]-1H,12H-pyrano[3",4":6',7']indolizino [1',2':6,5]pyrido[4,3,2-de]quinazoline-2,10,13(3H,9H,15H)-trione;
f) (9S)-9-ethyl-1-heptyl-9-hydroxy-1H,12H-pyrano[3",4":6',7']indolizino[1',2':6,5]pyrido [4,3,2-de]quinazoline-2,10,13(3H,9H,15H)-trione;
g) (9S)-9-ethyl-9-hydroxy-1-propyl-1H,12H-pyrano[3",4":6',7']indolizino[1',2':6,5]pyrido [4,3,2-de]quinazoline-2,10,13(3H,9H,15H)-trione;
h) (9S)-9-ethyl-9-hydroxy-1-[2-(pyridin-3-yl)ethyl]-1H,12H-pyrano[3",4":6',7']indolizino [1',2':6,5]pyrido[4,3,2-de]quinazoline-2,10,13(3H,9H,15H)-trione;
i) (9S)-9-ethyl-9-hydroxy-1-(3-phenylpropyl)-1H,12H-pyrano[3",4":6',7']indolizino[1',2':6,5] pyrido[4,3,2-de]quinazoline-2,10,13(3H,9H,15H)-trione;
j) (9S)-9-ethyl-9-hydroxy-1-(2-methylpropyl)-1H,12H-pyrano[3",4":6',7']indolizino[1',2':6,5] pyrido[4,3,2-de]quinazoline-2,10,13(3H,9H,15H)-trione;
k) (9S)-9-ethyl-1-hexyl-9-hydroxy-1H,12H-pyrano[3",4":6',7']indolizino[1',2':6,5]pyrido [4,3,2-de]quinazoline-2,10,13(3H,9H,15H)-trione;
l) (9S)-9-ethyl-9-hydroxy-1-pentyl-1H,12H-pyrano[3",4":6',7']indolizino[1',2':6,5]pyrido [4,3,2-de]quinazoline-2,10,13(3H,9H,15H)-trione;
m) (9S)-9-ethyl-9-hydroxy-1-[2-(4-methoxyphenyl)ethyl]-1H,12H-pyrano[3",4":6',7']indolizino [1',2':6,5]pyrido[4,3,2-de]quinazoline-2,10,13(3H,9H,15H)-trione;
n) (9S)-1-benzyl-9-ethyl-9-hydroxy-1H,12H-pyrano[3",4":6',7']indolizino[1',2':6,5]pyrido [4,3,2-de]quinazoline-2,10,13(3H,9H,15H)-trione;
o) (9S)-9-ethyl-9-hydroxy-1-(3-methylbutyl)-1H,12H-pyrano[3",4":6',7']indolizino[1',2':6,5] pyrido[4,3,2-de]quinazoline-2,10,13(3H,9H,15H)-trione;
P) (9S)-1,9-diethyl-9-hydroxy-1H,12H-pyrano[3",4",:6',7']indolizino[1',2':6,5]pyrido[4,3,2-de] quinazoline-2,10,13(3H,9H,15H)-trione;
q) (9S)-1-[2-(4-cblorophenyl)ethyl]-9-ethyl-9-hydroxy-1H,12H-pyrano[3",4":6',7']indolizino [1',2':6,5]pyrido[4,3,2-de]quinazoline-2,10,13(3H,9H,15H)-trione;
r) (9S)-9-ethyl-1-[2-(4-fluorophenyl)ethyl]-9-hydroxy-1H,12H-pyrano[3",4":6',7']indolizino [1',2':6,5]pyrido[4,3,2-de]quinazoline-2,10,13(3H,9H,15H)-trione;
s) (9S)-9-ethyl-9-hydroxy-1-(1-methylethyl)-1H,12H-pyrano[3",4":6',7']indolizino[1',2':6,5] pyrido[4,3,2-de]quinazoline-2,10,13(3H,9H,15H)-trione; and
t) (9S)-1-(3,3-dimethylbutyl)-9-ethyl-9-hydroxy-1H,12H-pyrano[3",4":6',7']indolizino[1',2':6,5] pyrido[4,3,2-de]quinazoline-2,10,13(3H,9H,15H)-trione.

Among them, the following compounds are more preferred:
a) (9S)-1-butyl-9-ethyl-9-hydroxy-1H,12H-pyrano[3",4":6',7']indolizino[1',2':6,5]pyrido[4,3,2-de]quinazoline-2,10,13(3H,9H,15H)-trione;
b) (9S)-9-ethyl-9-hydroxy-1-phenethyl-1H,12H-pyrano[3",4":6',7']indolizino[1',2':6,5]pyrido[4,3,2-de]quinazoline-2,10,13(3H,9H,15H)-trione;
c) (9S)-9-ethyl-9-hydroxy-1-(2-methylpropyl)-1H,12H-pyrano[3",4":6',7']indolizino[1',2':6,5]pyr ido[4,3,2-de]quinazoline-2,10,13(3H,9H,15H)-trione;
d) (9S)-9-ethyl-1-hexyl-9-hydroxy-1H,12H-pyrano[3",4":6',7']indolizino[1',2':6,5]pyrido[4,3,2-d e]quinazoline-2,10,13(3H,9H,15H)-trione;
e) (9S)-9-ethyl-9-hydroxy-1-pentyl-1H,12H-pyrano[3",4":6',7']indolizino[1',2':6,5]pyrido[4,3,2-d e]quinazoline-2,10,13(3H,9H, 15H)-trione;
f) (9S)-9-ethyl-9-hydroxy-1-(3-methylbutyl)-1H,12H-pyrano[3",4":6',7']indolizino[1',2':6,5]pyri do[4,3,2-de]quinazoline-2,10,13(3H,9H,15H)-trione;
g) (9S)-1-[2-(4-chlorophenyl)ethyl]-9-ethyl-9-hydroxy-1H,12H-pyrano[3",4":6',7']indolizino[1',2 ':6,5]pyrido[4,3,2-de]quinazoline-2,10,13(3H,9H,15H)-trione;
h) (9S)-9-ethyl-1-[2-(4-fluorophenyl)ethyl]-9-hydroxy-1H,12H-pyrano[3",4":6',7']indolizino[1',2':6,5]pyrido[4,3,2-de]quinazoline-2,10,13(3H,9H,15H)-trione; and
i) (9S)-1-(3,3-dimethylbutyl)-9-ethyl-9-hydroxy-1H,12H-pyrano[3",4":6',7']indolizino[1',2':6,5] pyrido [4,3,2-de]quinazoline-2,10,13(3H,9H,15H)-trione.

The aforementioned Y is a compound represented by formula (7) below. 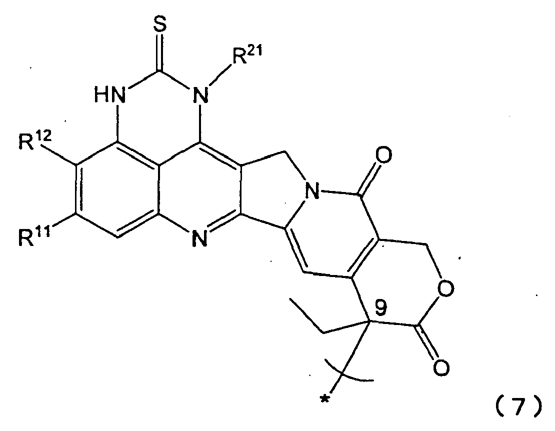 R¹¹, R¹², and R²¹ are defined as in formulae (4) and (5).

In formula (7), R¹¹, and R¹² are preferably hydrogen atoms.
R²¹ is preferably a hydrogen atom or a C1-C8 alkyl group that may comprise a substituent selected from Group D listed below:
Group D: a C1-C3 alkoxy group, hydroxy group, halogen atom, amino group, mono-C1-C3 alkylamino group, di-C1-C3 alkylamino group, C3-C7 cycloalkyl group, heterocycle, and aryl ring (which may comprise 1 to 3 substituents selected from the group consisting of a hydroxy group, C1-C3 alkoxy group, and halogen atom).
R²¹ is more preferably a phenyl group or C1-C6 alkyl group, and even more preferably a phenyl group, 3-methylbutyl group, or *n*-pentyl group.

Examples of such compounds represented by formula (7) include those in which Y represents a residue of a compound (Y-OH) comprising at least one alcoholic hydroxyl group (for example at position 9), in which the compound is selected from the group consisting of:
a) (9S)-9-ethyl-9-hydroxy-1-(3-methylbutyl)-1H,12H-pyrano[3",4":6',7']indolizino[1',2':6,5]pyri do[4,3,2-de]quinazoline-2(3H)-thione-10,13(9H,15H)-dione;
b) (9S)-9-ethyl-9-hydroxy-1-phenethyl-1H,12H-pyrano[3",4":6',7']indoiizino[1',2':6,5]pyrido[4,3,2-de]quinazoline-2(3H)-thione-10,13(9H,15H)-dione; and
c) (9S)-9-ethyl-9-hydroxy-1-pentyl-1H,12H-pyrano[3",4":6',7']indolizino[1',2':6,5]pyrido[4,3,2-d e]quinazoline-2(3H)-thione-10,13(9H,15H)-dione.

The compounds in which Y is represented by formula (8) are as described below. 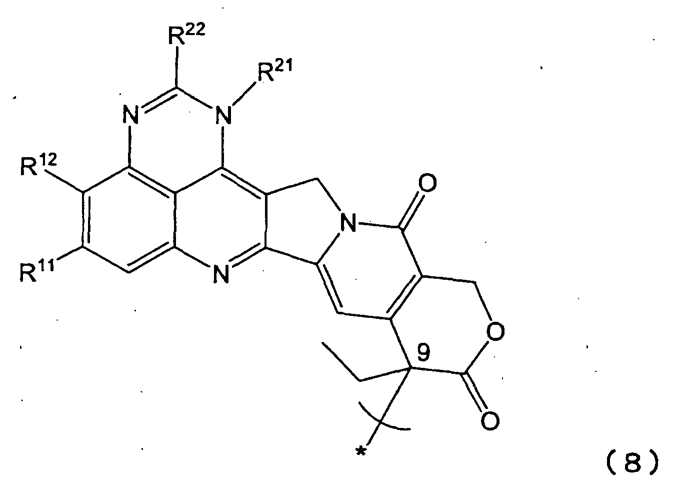 R¹¹, R¹², R²¹, and R²² have the same meanings as R¹¹, R¹², R²¹, and R²², respectively, of formulae (4) and (5).

In formula (8),
(i) R¹¹ is preferably a hydrogen atom.
(ii) R¹² is preferably a hydrogen atom or a C1-C3 alkyl group, and is more preferably a hydrogen atom or methyl group.
(iii) R²¹ is preferably a hydrogen atom, or a C1-C8 alkyl group that may comprise 1 to 3 substituents selected from Group D described below:
   Group D: a C1-C3 alkoxy group, hydroxy group, halogen atom, amino group, mono-C1-C3 alkylamino group, di-C1-C3 alkylamino group, C3-C7 cycloalkyl group, heterocycle, and aryl ring (which may comprise 1 to 3 substituents selected from the group consisting of a hydroxy group, C1-C3 alkoxy group, and halogen atom).
(iv) R²¹ is more preferably a methyl group, ethyl group, *n*-propyl group, 1-methylethyl group, *n*-butyl group, 1,1-dimethylethyl group, 2-methylpropyl group, 2,2-dimethylpropyl group, *n*-pentyl group, 3-methylbutyl group, 2-*n*-hexyl group, 3,3-dimethylbutyl group, *n*-heptyl group, *n*-octyl group, benzyl group, phenethyl group, 2-(dimethylamino)ethyl group, 2-(4-morpholino)ethyl group, 3-(dimethylamino)propyl group, 2-(pyridin-2-yl)ethyl group, 2-(pyridin-3-yl)ethyl group, 2-(4-methoxyphenyl)ethyl group, 2-(4-chlorophenyl)ethyl group, 2-(4-fluorophenyl)ethyl group, or 3-phenylpropyl group.
(v) R²² is preferably a hydrogen atom, amino group, C1-C6 alkyl group, C1-C6 alkoxy group, a C1-C6 alkylthio group that may comprise 1 to 3 substituents selected from Group D described below, a mono-C1-C6 alkylamino group that may comprise 1 to 3 substituents selected from Group D described below, or a di-C1-C6 alkyl amino group that may comprise 1 to 3 substituents selected from Group D described below:

Group D: a C1-C3 alkoxy group, hydroxy group, halogen atom, amino group, mono-C1-C3 alkylamino group, di-C1-C3 alkylamino group, C3-C7 cycloalkyl group, heterocycle, and aryl ring (which may comprise 1 to 3 substituents selected from the group consisting of a hydroxy group, C1-C3 alkoxy group, and halogen atom).
   (vi) R²² is more preferably a hydrogen atom, methyl group, ethyl group, propyl group, hydroxymethyl group, aminomethyl group, (methylamino)methyl group, (dimethylamino)methyl group, chloromethyl group, trifluoromethyl group, phenyl group, 2-pyridyl group, methoxy group, ethoxy group, methylthio group, ethylthio group, methylamino group, butylamino group, or dimethylamino group.
Regarding the above-mentioned (i) to (vi), preferred embodiments can be randomly combined. Examples of combinations are (i), (ii), (iii), and (v); (i), (ii), (iii), and (vi); (i), (ii), (iv), and (v); and (i), (ii), (iv), and (vi).

Examples of such compounds represented by formula (8) include those in which Y represents a residue of a compound (Y-OH) comprising at least one alcoholic hydroxyl group (for example at position 9), in which the compound is selected from the group consisting of:
a) (9S)-1-butyl-9-ethyl-9-hydroxy-1H,12H-pyrano[3",4":6',7']indolizino[1',2':6,5]pyrido[4,3,2-de]quinazoline-10,13(9H,15H)-dione;
b) (9S)-9-ethyl-9-hydroxy-1-[2-(4-morpholino)ethyl]-1H,12H-pyrano[3",4":6',7']indolizino[1',2': 6,5]pyrido[4,3,2-de]quinazoline-10,13(9H,15H)-dione hydrochloride;
c) (9S)-9-ethyl-9-hydroxy-1-propyl-1H,12H-pyrano[3",4":6',7']indolizino[1',2':6,5]pyrido[4,3,2-de]quinazoline-10,13(9H,15H)-dione;
d) (9S)-1-benzyl-9-ethyl-9-hydroxy-1H,12H-pyrano[3",4":6',7']indolizino[1',2':6,5]pyrido[4,3,2-de]quinazoline-10,13(9H,15H)-dione;
e) (9S)-9-ethyl-9-hydroxy-1-phenethyl-1H,12H-pyrano[3'',4":6',7']indolizino[1',2':6,5]pyrido[4,3,2-de]quinazoline-10,13(9H,15H)-dione;
f) (9S)-2,9-diethyl-9-hydroxy-1-phenethyl-1H,12H-pyrano[3",4":6',7']indolizino[1',2':6,5]pyrido [4,3,2-de]quinazoline-10,13(9H,15H)-dione;
g) (9S)-9-ethyl-9-hydroxy-1-(3-phenylpropyl)-1H,12H-pyrano[3",4":6',7']indolizino[1',2':6,5]pyr ido[4,3,2-de]quinaoline-10,13(9H,15H)-dione;
h) (9S)-9-ethyl-9-hydroxy-1-(3-methylbutyl)-1H,12H-pyrano[3",4":6',7']indolizino[1',2':6,5]pyri do[4,3,2-de]quinazoline-10,13(9H,15H)-dione;
i) (9S)-2,9-diethyl-9-hydroxy-1-(3-methylbutyl)-1H,12H-pyrano[3",4":6',7']indolizino[1',2':6,5] pyrido[4,3,2-de]quinazoline-10,13(9H,15H)-dione;
j) (9S)-2,9-diethyl-9-hydroxy-1-(2-methylpropyl)-1H,12H-pyrano[3",4":6',7']indolizino[1',2':6,5]pyrido[4,3,2-de]quinazoline-10,13(9H,15H)-dione;
k) (9S)-9-ethyl-1-heptyl-9-hydroxy-1H,12H-pyrano[3",4":6',7']indolizino[1'2':6,5]pyrido[4,3,2-d e]quinazoline-10,13(9H,15H)-dione;
l) (9S)-9-ethyl-9-hydroxy-1-methyl-1H,12H-pyrano[3",4":6',7']indolizino[1',2':6,5]pyrido[4,3,2-de]quinazoline-10,13(9H,15H)-dione;
m) (9S)-9-ethyl-9-hydroxy-1-(2-methylpropyl)-1H,12H-pyrano[3",4":6',7']indolizino[1',2':6,5]pyr ido [4,3,2-de]quinazoline-10,13(9H, 15H)-dione;
n) (9S)-9-ethyl-1-hexyl-9-hydroxy-1H,12H-pyrano[3",4":6',7']indolizino[1',2':6,5]pyrido[4,3,2-d e]quinazoline-10,13(9H,15H)-dione;
o) (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione;
p) (9S)-1,9-diethyl-9-hydroxy-1H,12H-pyrano[3",4":6',7']indolizino[1',2':6,5]pyrido[4,3,2-de]qui nazoline-10,13(9H,15H)-dione;
q) (9S)-9-ethyl-9-hydroxy-1-[2-(4-methoxyphenyl)ethyl]-1H,12H-pyrano[3",4":6',7']indolizino[1',2':6,5]pyrido [4,3,2-de] quinazoline-10,13(9H,15H)-dione;
r) (9S)-1-[2-(4-chlorophenyl)ethyl]-9-ethyl-9-hydroxy-1H,12H-pyrano[3",4":6',7']indolizino[1',2':6,5]pyrido[4,3,2-de] quinazoline-10,13(9H,15H)-dione;
s) (9S)-9-ethyl-1-[2-(4-fluorophenyl)ethyl]-9-hydroxy-1H,12H-pyrano[3",4":6',7']indolizino[1',2':6,5]pyrido[4,3,2-de]quinazoline-10,13(9H,15H)-dione;
t) (9S)-9-ethyl-1-[2-(4-fluorophenyl)ethyl]-9-hydroxy-2-methyl-1H,12H-pyrano[3",4":6',7']indolizino[1',2':6,5]pyrido[4,3,2-de]quinazoline-10,13(9H,15H)-dione;
u) (9S)-9-ethyl-9-hydroxy-1-(1-methylethyl)-1H,12H-pyrano[3",4":6',7']indolizino[1',2':6,5]pyrido [4,3,2-de]quinazoline-10,13(9H,15H)-dione;
v) (9S)-1-(3,3-dimethylbutyl)-9-ethyl-9-hydroxy-1H,12H-pyrano[3",4":6',7']indolizino[1',2':6,5]pyrido [4,3,2-de]quinazoline-10,13(9H,15H)-dione;
w) (9S)-9-ethyl-9-hydroxy-2-methoxy-1-(3-methylbutyl)-1H,12H-pyrano[3",4":6',7']indolizino[1',2':6,5]pyrido[4,3,2-de]quinazoline-10,13(9H,15H)-dione;
x) (9S)-2,9-diethyl-9-hydroxy-1-(3-methylbutyl)-1H,12H-pyrano[3",4":6',7']indolizino[1',2':6,5]pyrido [4,3,2-de]quinazoline-10,13(9H,15H)-dione;
y) (9RS)-9-ethyl-9-hydroxy-4-methyl-1-pentyl-1H,12H-pyrano[3",4":6',7']indolizino[1',2':6,5]pyrido [4,3,2-de]quinazoline-10,13(9H,15H)-dione;
z) (9S)-9-ethyl-9-hydroxy-1-(2-hydroxyethyl)-1H,12H-pyrano[3",4":6',7']indolizino[1',2':6,5]pyrido [4,3,2-de]quinazoline-10,13 (9H,15H)-dione;
aa) (9S)-9-ethyl-9-hydroxy-1-(2-hydroxyethyl)-2-methyl-1H,12H-pyrano[3",4":6',7']indolizino[1',2':6,5]pyrido[4,3,2-de]quinazoline-10,13(9H,15H)-dione;
bb) (9S)-9-ethyl-9-hydroxy-2-methyl-1-pentyl-1H,12H-pyrano[3",4":6',7']indolizino[1',2':6,5]pyrido[4,3,2-de]quinazoline-10,13(9H,15H)-dione;
cc) (9S)-2,9-diethyl-9-hydroxy-1-pentyl-1H,12H-pyrano[3",4":6',7']indolizino[1',2':6,5]pyrido[4,3,2-de]quinazoline-10,13(9H,15H)-dione;
dd) (9S)-9-ethyl-9-hydroxy-1-pentyl-2-propyl-1H,12H-pyrano[3",4":6',7]indolizino[1',2':6,5]pyrido[4,3,2-de]quinazoline-10,13(9H,15H)-dione;
ee) (9S)-9-ethyl-9-hydroxy-2-hydroxymethyl-1-pentyl-1H,12H-pyrano[3",4":6',7']indolizino[1',2': 6,5]pyrido[4,3,2-de]quinazoline-10,13(9H,15H)-dione;
ff) (9S)-9-ethyl-9-hydroxy-2-hydroxymethyl-1-(2-methylpropyl)-1H,12H-pyrano[3",4":6',7']indol izino[1',2':6,5]pyrido[4,3,2-de]quinazoline-10,13(9H,15H)-dione;
gg) (9S)-9-ethyl-9-hydroxy-2-hydroxymethyl-1-(3-methylbutyl)-1H,12H-pyrano[3",4":6',7']indoliz ino[1',2':6,5]pyrido[4,3,2-de]quinazoline-10,13(9H,15H)-dione;
hh) (9S)-2-chloromethyl-9-ethyl-9-hydroxy-1-(3-methylbutyl)-1H,12H-pyrano[3",4":6',7']indolizin o[1',2':6,5]pyrido[4,3,2-de]quinazoline-10,13(9H,15H)-dione;
ii) (9S)-2-aminomethyl-9-ethyl-9-hydroxy-1-pentyl-1H,12H-pyrano[3",4":6',7']indolizino[1',2':6, 5]pyrido[4,3,2-de]quinazoline-10,13(9H,15H)-dione;
jj) (9S)-9-ethyl-9-hydroxy-1-pentyl-2-trifluoromethyl-1H,12H-pyrano[3",4":6',7']indolizino[1',2': 6,5]pyrido[4,3,2-de]quinazoline-10,13(9H,15H)-dione;
kk) (9S)-9-ethyl-9-hydroxy-1-(3-methylbutyl)-2-methylthio-1H,12H-pyrano[3",4":6',7']indolizino[1',2':6,5]pyrido[4,3,2-de]quinazoline-10,13(9H,15H)-dione;
ll) (9S)-9-ethyl-2-ethylthio-9-hydroxy-1-(3-methylbutyl)-1H,12H-pyrano[3",4":6',7']indolizino[1',2':6,5]pyrido[4,3,2-de]quinazoline-10,13(9H,15H)-dione;
mm) (9S)-2-(dimethylamino)-9-ethyl-9-hydroxy-1-(2-methylpropyl)-1H,12H-pyrano[3",4":6',7']ind olizino[1',2':6,5]pyrido[4,3,2-de]quinazoline-10,13(9H,15H)-dione hydrochloride; and
nn) (9S)-2-(butylamino)-9-ethyl-9-hydroxy-1-(3-methylbutyl)-1H,12H-pyrano[3",4":6',7']indolizin o[1',2':6,5]pyrido[4,3,2-de]quinazoline-10,13(9H,15H)-dione hydrochloride.

Among the camptothecins indicated above, particularly preferred examples are the following compounds:
4(S)-ethyl-4-hydroxy-1H-pyrano[3',4':6,7]indolizino[1,2-b]quinoline-3,14(4H,12H)-dio ne(camptothecin);
5(R)-ethyl-9,10-difluoro-1,4,5,13-tetrahydro-5-hydroxy-3H,15H-oxepino[3',4':6,7]indo lizino[1,2-b]quinoline-3,15-dione (BN-80915);
o) (9S)-9-ethyl-9-hydroxy-1-pentyl-1H,12H-pyrano[3",4":6',7']indolizino[1'.2':6,5]pyrido[4,3,2-d e]quinazoline-10,13(9H,15H)-dione;
bb) (9S)-9-ethyl-9-hydroxy-2-methyl-1-pentyl-1H,12H-pyrano[3",4":6',7']indolizino[1',2':6,5]pyri do[4,3,2-de]quinazoline-10,13(9H,15H)-dione; and
ee) (9S)-9-ethyl-9-hydroxy-2-hydroxymethyl-1-pentyl-1H,12H-pyrano[3'',4":6',7']indolizino[1',2': 6,5]pyrido[4,3,2-de]quinazoline-10,13(9H,15H)-dione.

Among the water-soluble prodrugs of the present invention, examples of compounds that are derived from camptothecins include the following:
(a) (9S)-9-ethyl-9-{[methyl-(2-methylamino-ethyl)-amino]-acetoxy}-1-pentyl-1H,12H-pyrano[3",4' ':6',7']indolizino[1',2':6,5]pyrido[4,3,2-de]quinazoline-10,13(9H,15H)-dione;
(b) (9S)-9-ethyl-9-(glycyl-sarcosyloxy)-1-pentyl-1H,12H-pyrano[3",4":6',7']indolizino[1',2':6,5]p yrido[4,3,2-de]quinazoline-10,13(9H,15H)-dione;
(c) (9S)-9-{[(2-amino-ethyl)-methyl-amino]-acetoxy}-9-ethyl-1-pentyl-1H,12H-pyrano[3",4":6',7']indolizino[1',2':6,5]pyrido[4,3,2-de]quinazoline-10,13(9H,15H)-dione;
(d) (9S)-9-ethyl-9-(sarcosyl-sarcosyloxy)-1-pentyl-1H,12H-pyrano[3",4":6',7']indolizino[1',2':6,5] pyrido[4,3,2-de]quinazoline-10,13(9H,15H)-dione;
(e) (9S)-9-[2-(2-aminoethanesulfonyl)ethoxycarbonyloxy]-9-ethyl-1-pentyl-1H,12H-pyrano[3",4": 6',7']indolizino[1',2':6,5]pyrido[4,3,2-de]quinazoline-10,13(9H,15H)-dione;
(f) (aminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester;
(g) (9S)-9-{2-[(R-2-amino-2-methoxycarbonyl)ethanesulfonyl]ethoxycarbonyloxy}-9-ethyl-1-pentyl -1H,12H-pyrano[3",4":6',7']indolizino[1',2':6,5]pyrido[4,3,2-de]quinazoline-10,13(9H,15H)-di one;
(h) (9S)-9-{2-[(R-2-amino-2-ethoxycarbonyl)ethanesulfonyl]ethoxycarbonyloxy}-9-ethyl-1-pentyl-1 H,12H-pyrano[3",4":6',7']indolizino[1',2':6,5]pyrido[4,3,2-de]quinazoline-10,13(9H,15H)-di one;
(i) (9S)-9-ethyl-9-(*N*-methylalanyl-*N*-methylalanyloxy)-1-pentyl-1H,12H-pyrano[3",4":6',7']indolizino[1',2':6,5]pyrido[4,3,2-de]quinazoline-10,13(9H,15H)-dione; and
(j) (9S)-9-ethyl-9-(sarcosyl-*N*-methylalanyloxy)-1-pentyl-1H,12H-pyrano[3",4":6',7']indolizino[1',2':6,5]pyrido[4,3,2-de]quinazoline-10,13(9H,15H)-dione.

The water-soluble prodrugs of the present invention are preferably the compounds of (b), (d), (e), and (h), and more preferably compounds of (b) and (e).

### Best Mode for Carrying Out the Invention

The compounds represented by formula (1) used in the present invention may be produced using the following methods, but the methods for producing the compounds of this invention are not to be construed as being limited thereto. Although the water-soluble prodrugs of this invention are all novel compounds, they can be produced by well-known chemical methods using commercially available raw materials, or those synthesized by standard methods, as necessary.

In the following production methods, R¹, R², R³, R⁴, X, Y, R⁵, n, R⁷, and R⁸ have the same meaning as R¹, R², R³, R⁴, X, Y, R⁵, n, R⁷, and R⁸, respectively, as defined in formulas (2), (3), and (9). P¹ represents an amino protecting group, P² represents a residue from a carbonylating reagent, and Hal represents a halogen atom (i.e., a chlorine atom, bromine atom, or iodine atom).

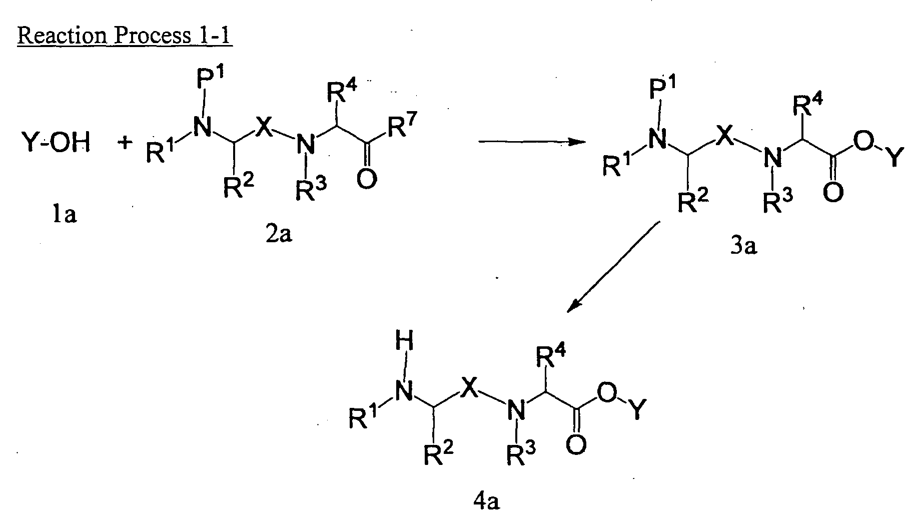

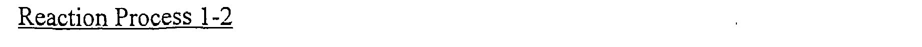

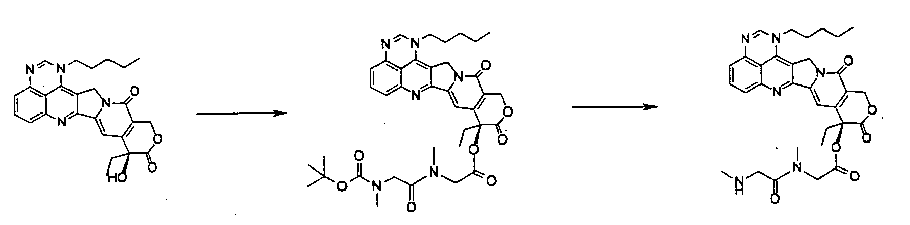

### <Reaction Process 1>

Reaction Processes 1-1 and 1-2 show an example of the production of a water-soluble prodrug comprising a tertiary amino group in its solubilizing side chain.

The water-soluble prodrug represented by formula (2) can be obtained easily by, for example, acylation of a secondary or tertiary hydroxyl group present in Y-OH.

### Preparation of compound 3a

As indicated in Reaction Process 1-1, ester (3 a) can be obtained by reacting a secondary or tertiary alcohol (1a) with a suitable compound (2a) in an appropriate solvent, in the presence of a coupling agent. Examples of compound 2a include a carboxylic acid (R⁷=OH), a carboxylate (R⁷=OR⁸), and a acyl halide compound (R⁷=halogen atom:chlorine atom or such), with carboxylic acid being the preferred one.

Y-OH can be obtained commercially or by known methods (WO03/045952, WO03/043631, etc.).

When compound (2a) is a dipeptide (X=CO) carboxylic acid, or a peptide derivative (X=CH₂), the amino acid derivative used to prepare such a compound (2a) is commercially available, or it can be prepared by known methods described in the literature (for example, J. Am. Chem. Soc. 2000, 122, 762-766; J. Org. Chem, 1998, 5240; Tetrahedron Asymmetry, 1995, 1741; Tetrahedron Asymmetry, 1998, 4249). The carboxylic acid can be converted into a carboxylate ester (R⁷=OR⁸) or a acyl halide compound (R⁷=halogen atom) by known methods.

Furthermore, the dipeptide derivative can be prepared by standard peptide chemistry well known to those skilled in the art [see, "The Practice of Peptide Synthesis" by M. Bodansky and A. Bodansky, 2nd edition, 1994 (Springer-Verlag)].

Examples of solvents used in the above coupling reaction include methylene chloride, ethyl acetate, tetrahydrofuran, acetonitrile, chloroform, dioxane, and dimethylformamide.

Examples of the coupling agents include 1-ethyl-3-(3-dimethylaminopropyl)-carbodiimide hydrochloride, dicyclohexylcarbodiimide, BOP, HBTU, TNTU, PyBroP™, PyBOP™, TBTU, TSTU, and HOBt [see, The Combinatorial Chemistry Catalog, Feb., 1997; Novabiochem., for commercially available coupling reagents].

### Preparation of compound 4a

While preparation of compound 4a depends on the type of the corresponding carboxylic acid, a corresponding carboxylic acid (2a) with a protected amino group is usually preferred. The coupling reaction is followed by removal of the protecting group from compound (3 a), to give the water-soluble prodrug represented by compound (4a).

The obtained water-soluble prodrug comprising an amino protecting group is deprotected, for example, as shown in Process 1-2.

The coupling reaction and selection of the amino protecting group P¹ in Processes 1-1 and 1-2 can be performed suitably using known methods (see, "The Practice of Peptide Synthesis" by M. Bodansky and A. Bodansky, 2nd edition, 1994 (Springer-Verlag); "Protective Groups in Organic Synthesis" by Theodora Greene, 1999 (Wiley-Interscience)).

Examples of amino protecting groups include the following:

### Carbamate types

a methyloxycarbonyl group, 9-fluorenylmethyloxycarbonyl group, 9-(2-sulfo)fluorenylmethyloxycarbonyl group, 9-(2,7-dibromo)fluorenylmethyloxycarbonyl group, 4-methoxyphenacyloxycarbonyl group, ethyloxycarbonyl group, 2,2,2-trichloroethyloxycarbonyl group, 2-trimethylsilylethyloxycarbonyl group, phenethyloxycarbonyl group, 1-(1-adamantyl)-1-methylethyloxycarbonyl group, 2-chloroethyloxycarbonyl group, 2-bromoethyloxycarbonyl group, 2-iodoethyloxycarbonyl group, 2,2-dichloroethyloxycarbonyl group, 2,2-dibromoethyloxycarbonyl group, 2,2,2-trichloroethyloxycarbonyl group, 2,2,2-tribromoethyloxycarbonyl group, 1,1-dimethyl-2-chloroethyloxycarbonyl group, 1,1-dimethyl-2-bromoethyloxycarbonyl group, 1,1-dimethyl-2,2-dibromoethyloxycarbonyl group, 1,1-dimethyl-2,2,2-trichloroethyloxycarbonyl group, 1-methyl-1-(4-biphenylyl)ethyloxycarbonyl group, 1-(3,5-di *t*-butylphenyl)-1-methylethyloxycarbonyl group, 2-(2'-pyridyl)ethyloxycarbonyl group, 2-(4'-pyridyl)ethyloxycarbonyl group, 2-(*N,N*-dicyclohexylcarboxamide) ethyloxycarbonyl group, *t*-butyloxycarbonyl group, 1-adamantyloxycarbonyl group, vinyloxycarbonyl group, allyloxycarbonyl group, 1-isopropylallyloxycarbonyl group, cinnamyloxycarbonyl group, 4-nitrocinnamyloxycarbonyl group, 8-quinolyloxycarbonyl group, piperidinyloxycarbonyl group, benzyloxycarbonyl group, *p*-methoxybenzyloxycarbonyl group, *p*-nitrobenzyloxycarbonyl group, *p*-chlorobenzyloxycarbonyl group, *p*-bromobenzyloxycarbonyl group, *p*-cyanobenzyloxycarbonyl group, *o*-nitrobenzyloxycarbonyl group, 2,4-dichlorobenzyloxycarbonyl group, 4-methylsulfinylbenzyloxycarbonyl group, 9-anthrylmethyloxycarbonyl group, diphenylmethyloxycarbonyl group, 2-methylthioethyloxycarbonyl group, 2-methylsulfonylethyloxycarbonyl group, 2-(*p*-toluenesulfonyl)ethyloxycarbonyl group, [2-(1,3-dithianyl)]methyloxycarbonyl group, 4-methylthiophenyloxycarbonyl group, 2,4-dimethylthiophenyloxycarbonyl group, 2-phosphinoethyloxycarbonyl group, 2-triphenylphosphonioisopropyloxycarbonyl group, 1,1-dimethyl-2-cyanoethyloxycarbonyl group, *m*-chloro-*p*-acetylbenzyloxycarbonyl group, *p*-(dihydroxyboryl)benzyloxycarbonyl group, 5-benzisoxazolylmethyloxycarbonyl group, 2-(trifluoromethyl)-6-chromonylmethyloxycarbonyl group, *m*-nitrophenyloxycarbonyl group, 3,5-dimethoxybenzyloxycarbonyl group, 3,4-dimethoxy-6-nitrobenzyloxycarbonyl group, and phenyl(o-nitrophenyl)methyloxycarbonyl group;

### Urea types

a piperidinylcarbonyl group, *p*-toluenesulfonylaminocarbonyl group, and phenylaminothiocarbonyl group;

### Others

a *t*-amyloxycarbonyl group, benzylthiocarbonyl group, cyclobutyloxycarbonyl group, cyclopentyloxycarbonyl group, cyclohexyloxycarbonyl group, cyclopropylmethyloxycarbonyl group, *p*-decyloxybenzyloxycarbonyl group, diisopropylmethyloxycarbonyl group, 2,2-dimethoxycarbonylvinyloxycarbonyl group, *o*-(*N*,*N*-dimethylcarboxamide)benzyloxycarbonyl group, 1,1-dimethyl-3-(*N*,*N*-dimethylcarboxamide)propyloxycarbonyl group, 1,1-dimethylpropynyloxycarbonyl group, di(2-pyridyl)methyloxycarbonyl group, 2-furanylmethyloxycarbonyl group, isobomyloxycarbonyl group, isobutyloxycarbonyl group, isonicotinyloxycarbonyl group, *p*-(*p*'-methoxyphenylazo)benzyloxycarbonyl group, 1-methylcyclobutyloxycarbonyl group, 1-methylcyclohexyloxycarbonyl group, 1-methyl-1-cyclopropylmethyloxycarbonyl group, 1-methyl-1-(3,5-dimethoxyphenyl)ethyloxycarbonyl group, 1-methyl-1-(*p*-phenylazophenyl)ethyloxycarbonyl group, 1-methyl-1-phenylethyloxycarbonyl group, 1-methyl-1-(4-pyridyl)ethyloxycarbonyl group, *p*-(phenylazo)benzyloxycarbonyl group, 2,4,6-tri-*t*-butylphenyloxycarbonyl group, 4-(trimethylammonium)benzyloxycarbonyl group, and 2,4,6-trimethylbenzyloxycarbonyl group; and

### Amide types

a formyl group, acetyl group, chloroacetyl group, trichloroacetyl group, trifluoroacetyl group, phenylacetyl group, 3-phenylpropionyl group, picolinoyl group, benzoyl group, *p*-phenylbenzoyl group, *o*-nitrophenylacetyl group, o-nitrophenoxyacetyl group, acetoacetyl group, (*N*-dithiobenzyloxycarbonylamino)acetyl group, 3-(*p*-hydroxyphenyl)propionyl group, 3-(*o*-nitrophenyl)propionyl group, 2-methyl-2-(*o*-nitrophenoxy)propionyl group, 2-methyl-2-(o-phenylazophenoxy)propionyl group, 4-chlorobutyryl group, 3-methyl-3-nitrobutyryl group, *o*-nitrocinnamoyl group, *o*-nitrobenzoyl group, and o-(benzoyloxymethyl)benzoyl group.

Removal of the amino protecting group after a coupling reaction can be performed by methods well known to those skilled in the art, such as by reacting trifluoroacetic acid for the removal of a Boc group, piperidine for an Fmoc group, and tetrabutylammonium fluoride for 2-(trimethylsilyl)ethoxycarbonyl (Teoc), trimethylsilylethyl and *tert*-butyldimethylsilyl groups, and carrying out catalytic hydrogenation for a Cbz group.

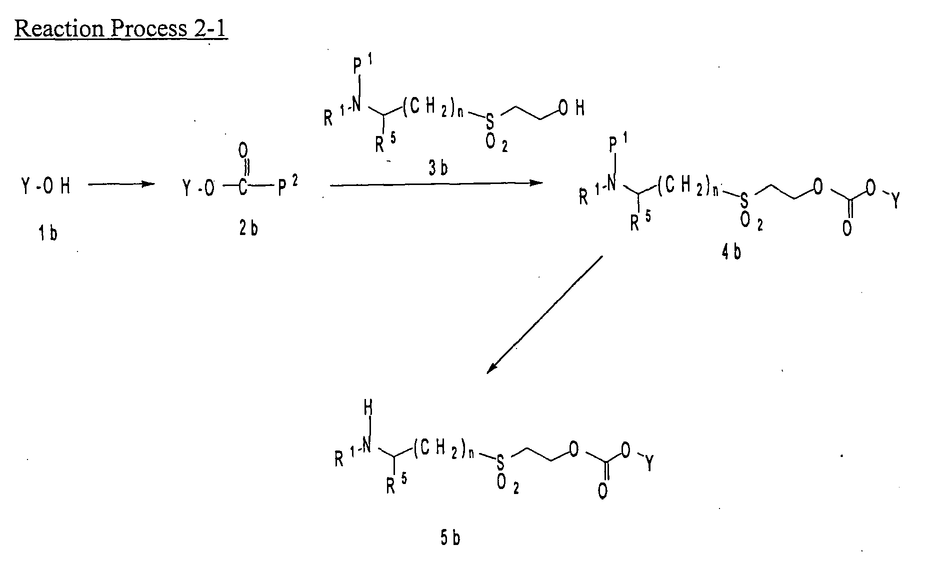

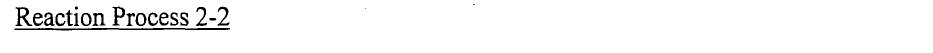

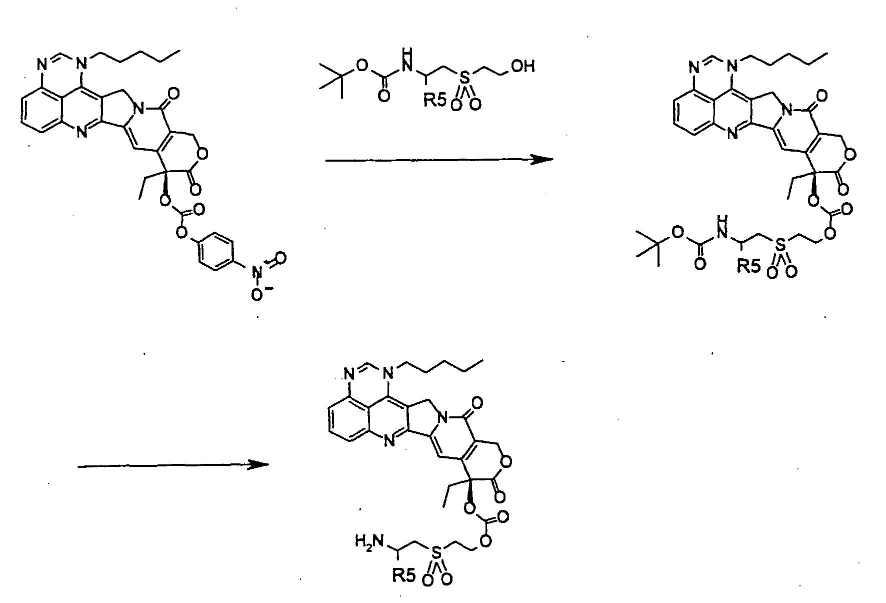

### <Reaction Process 2>

Reaction Processes 2-1 and 2-2 show an example of the method for producing water-soluble prodrugs whose solubilizing side chain comprises a sulfonyl group.

### Preparation of compound 2b

First, alcohol (1b) is carbonylated to produce compound (2b). Carbonylation of the hydroxyl group is accomplished by reacting alcohol (1b) with an appropriate carbonylating agent in an appropriate solvent.

Examples of a solvent that may be used include methylene chloride, ethyl acetate, tetrahydrofuran, acetonitrile, chloroform, dioxane, and dimethylformamide.

Examples of a carbonylating agent that may be used include *p*-nitrophenyl chloroformate, carbonyldiimidazole, and phosgenes.

Usually, the reaction can be performed at -10°C to 25°C, for 1 to 24 hours.

### Preparation of compound 3b

Compound 3b comprising a solubilizing side chain can be prepared by a known method (Tetrahedron (1999), 55: 6623-6634).

Protection of the amino group in alcohol (3b) can be performed suitably using known methods (see, The Practice of Peptide Synthesis, M. Bodansky, and A. Bodansky/2nd edition, 1994 (Springer-Verlag)).

### Preparation of compound 4b

Next, alcohol (3b) comprising a corresponding protected amino group can be reacted with carbonylated compound (2b) in the presence of an appropriate solvent to give an amino-group-protected carbonate (4b).

Examples of a solvent that may be used include methylene chloride, ethyl acetate, tetrahydrofuran, acetonitrile, chloroform, dioxane, and dimethylformamide.

Usually, the reaction can be performed at 15°C to 25°C, for 2 to 48 hours.

### Preparation of compound 5b

By removing the amino protecting group of carbonate (4b) by known methods, compound (5b) can be obtained. Removal of the amino protecting group can be performed using known methods, as shown in Reaction 1. Examples include by reacting trifluoroacetic acid for the removal of a Boc group, piperidine for an Fmoc group, and tetrabutylammonium fluoride for 2-(trimethylsilyl)ethoxycarbonyl (Teoc), trimethylsilylethyl and *tert*-butyldimethylsilyl groups, and carrying out catalytic hydrogenation for a Cbz group.

Reaction 2-2 shows a specific example of such.

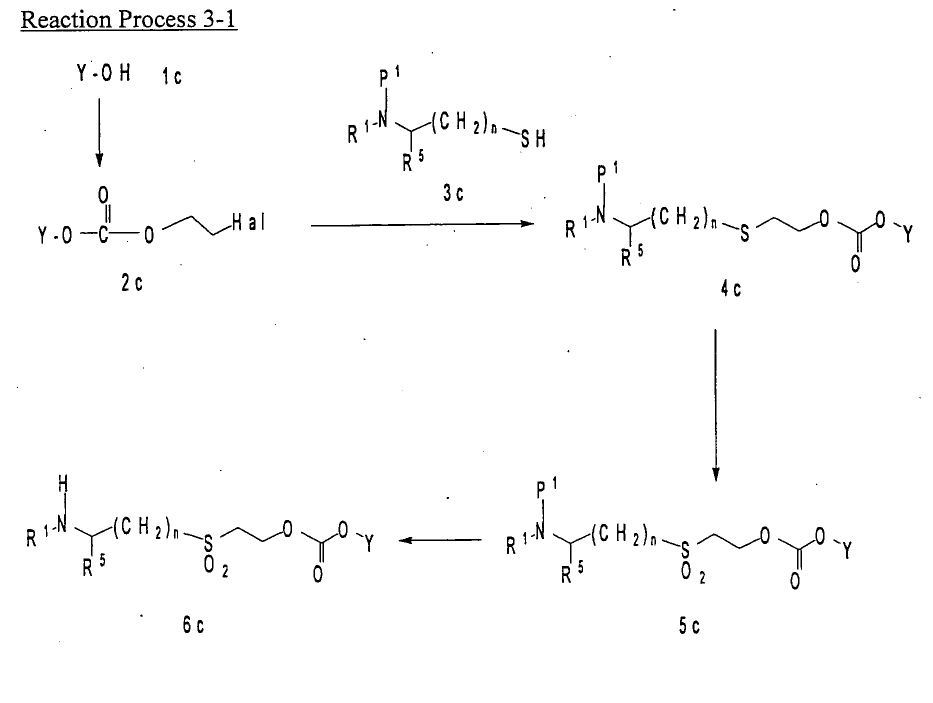

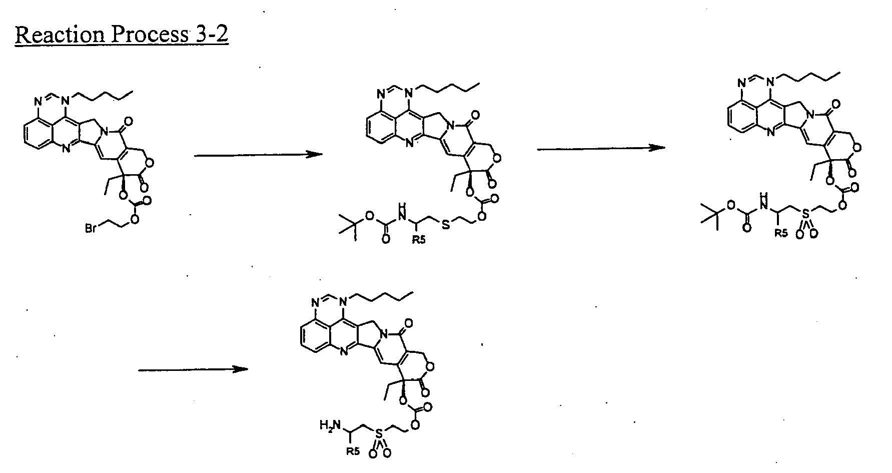

### <Reaction Process 3>

Reaction processes 3-1 and 3-2 show another example of methods for producing water-soluble prodrugs whose solubilizing side chain comprises a sulfonyl group.

### Preparation of compound 2c

First, a 2-halogenated ethyl carbonate (2c) is prepared from an alcohol (1c).

Conversion of the hydroxyl group of alcohol (1c) into a 2-halogenated ethyl carbonate (2) can be performed by reaction with a commercially available chloroformate in an appropriate solvent.

Examples of a solvent that may be used include methylene chloride, ethyl acetate, tetrahydrofuran, acetonitrile, chloroform, dioxane, and dimethylformamide.

Usually, the reaction can be performed at -10°C to 25°C, for 1 to 24 hours.

### Preparation of compound 3c

Thiol compound 3c comprising a solubilizing side chain can be prepared from a commercially available product, or it may be prepared by known methods (Tetrahedron, 1999, 55, 6623-6634; and J. Org. Chem. 1995, 60, 8105-8109).

Protection of the amino group in thiol compound (3c) can be performed suitably using known methods (see, The Practice of Peptide Synthesis, M. Bodansky, and A. Bodansky/2nd edition, 1994 (Springer-Verlag)).

### Preparation of compound 4c

Next, amino-group-protected carbonate (4c) can be obtained by reacting the corresponding amino-group-protected thiol compound (3c) with 2-halogenated ethyl carbonate (2c) in an appropriate solvent, under the presence of a base.

Examples of a solvent that may be used include methylene chloride, ethyl acetate, tetrahydrofuran, acetonitrile, chloroform, dioxane, dimethylformamide, methanol, and ethanol.

Examples of a base that may be used include triethylamine, diisopropylethylamine, potassium carbonate, and sodium carbonate.

Usually, the reaction can be performed at 15°C to 100°C, for 1 to 24 hours.

### Preparation of compound 5c

Furthermore, compound 5c can be obtained using known methods by reacting compound 4c with a peroxidizing reagent.

Examples of a peroxidizing reagent that may be used include oxone, hydrogen peroxide, and *m*-chloroperbenzoic acid.

Usually, the reaction can be performed at -10°C to 100°C, for 1 to 24 hours.

### Preparation of compound 6c

Compound (6c) can be obtained by removing the amino protecting group of compound 5c using known methods. Removal of the amino protecting group can be performed using known methods, as in Reaction 1. Examples include reacting trifluoroacetic acid for the removal of a Boc group, piperidine for an Fmoc group, and tetrabutylammonium fluoride for 2-(trimethylsilyl)ethoxycarbonyl (Teoc), trimethylsilylethyl and *tert*-butyldimethylsilyl groups, and carrying out catalytic hydrogenation for a Cbz group.

Reaction 3-2 shows a specific example of such.

The above illustrates an example of the methods for producing the water-soluble prodrugs of the present invention. The isolation and purification of the compounds of interest indicated in Reactions 1 to 3 can be performed by applying standard chemical operations, such as extraction, concentration, solvent removal, crystallization, filtration, recrystallization, and various chromatographies.

The water-soluble prodrugs represented by formulas (1) to (8) used in the present invention, and pharmaceutically acceptable salts, hydrates, and solvates thereof, include all stereoisomers of the water-soluble prodrugs mentioned above (for example, enantiomers, and diastereomers including cis and trans geometric isomers), racemates of the isomers, and other mixtures. The water-soluble prodrugs of the present invention represented by formulas (1) to (8) include stereoisomers, in particular.

Water-soluble prodrugs represented by formulas (1) to (8) used in the present invention, and pharmaceutically acceptable salts, hydrates, or solvates thereof, may exist in different tautomeric forms, such as the keto and enol forms, and the imine and enamine forms, or as mixtures of both. Tautomers exist as mixtures of a tautomeric set in solution. In solid form, usually one of the tautomers predominates. The present invention includes all tautomers of the compounds of this invention, including cases where only one of the tautomers is described.

Furthermore, the present invention includes atropisomers of this invention. Atropisomers refer to water-soluble prodrugs represented by formulas (1) to (8) that can be resolved into isomers with restricted rotation.

These isomers can be isolated by standard methods based on their differences in physicochemical properties. For example, racemic compounds can be resolved into sterically pure isomers by standard optical resolution methods, such as optical resolution by derivatization to diastereomeric salts using an optically active acid such as tartaric acid. Diastereomeric mixtures can be resolved by using fractional crystallization, and various chromatographic techniques (for example, thin layer chromatography, column chromatography, and gas chromatography).

When the compounds used in the present invention can be obtained in the free form, they can be formed into salts, or converted to hydrates or solvates thereof, by standard methods.

Alternatively, when the compounds used in the present invention are obtained as salts, hydrates, or solvates, these compounds can be converted to their free form by standard methods. Moreover, when the compounds used in the present invention can be obtained as salts, hydrates, or solvents of the compounds, the weight of these compounds can be converted to a free form weight (subtracting the weight accounting for the salt portion, water, or solvent from the weight of salts, hydrates or solvents) and thereby determining the amount of active ingredient in the formulation and the amount for administration.

The water-soluble prodrugs represented by formulas (1) to (8) used in the present invention, and their pharmaceutically acceptable salts, hydrates, and solvates show good water solubility, and since they are rapidly converted into the active form by chemical conversion, interspecies or individual differences are small.

In particular, the present invention is very useful when applied to insoluble pharmaceutical agents that comprise an alcoholic hydroxyl group, or preferably a secondary or tertiary alcoholic hydroxyl group.

For example, the water-soluble prodrugs of the present invention can easily solubilize insoluble compounds that are used as preventive or therapeutic agents for cell proliferation disorders, such as camptothecins, taxanes, and anticancer nucleotides. Therefore, pharmaceutical compositions comprising water-soluble prodrugs represented by formulas (1) to (8) of the present invention, or pharmaceutically acceptable salts, hydrates, or solvates derived from these insoluble compounds, as active ingredients are suitable preventive or therapeutic agents for cell proliferation disorders.

An example of the aforementioned cell proliferation disorder is cancer. Examples of cancers include colorectal cancer, lung cancer, breast cancer, gastric cancer, uterine cervical cancer, bladder cancer as well as pancreatic cancer, ovarian cancer, liver cancer, and such.

Furthermore, the present invention relates to preventive or therapeutic methods for cell proliferation disorders. The present invention also relates to methods comprising the step of administering a therapeutically effective amount of a water-soluble prodrugs represented by any of formulas (1) to (8), or a pharmaceutically acceptable salt, hydrate, or solvate thereof, to patients in need of such prevention or treatment. These methods are particularly useful for treating cell proliferation disorders including cancers and solid tumors, such as colorectal cancer, lung cancer, breast cancer, gastric cancer, uterine cervical cancer, bladder cancer, pancreatic cancer, ovarian cancer and liver cancer, and such.

When using a pharmaceutical composition of the present invention as a therapeutic agent or preventive agent for a cell proliferation disorder, such as pancreatic cancer, ovarian cancer or liver cancer, the method of administration preferably includes parenteral (intravenous, intramuscular, or subcutaneous) administration, local (drip infusion) administration, and inhalation (oral or nasal spray). The mode of administration preferably includes, for example, aqueous oral solutions and suspensions, and parenteral solutions loaded into an appropriate container that is suitable for administration in doses of small amounts. Furthermore, the mode of administration can be adjusted to various methods of administration, including those that involve controlled release of formulations, such as subcutaneous transplantation.

The pharmaceutical compositions of the present invention are preferably used as aqueous liquid formulations. More specifically, the aqueous liquid formulations of this invention comprise the water-soluble prodrugs represented by formulas (1) to (8), and pharmaceutically acceptable salts, hydrates, and solvates thereof.

Aqueous solutions that may be used include phosphate buffer, physiological saline, and various other infusion solutions.

The pH of the aqueous solution is preferably 4 or less, more preferably 3 or less, and even more preferably in the range of 2 to 3, and at such pH, the water-soluble prodrugs represented by formulas (1) to (8), and their pharmaceutically acceptable salts, hydrates, and solvates can exist stably in solution for long periods of time. On the other hand, preferably at pH5 or higher, or more preferably at physiological conditions of pH7-8, active forms derived from alcoholic hydroxyl groups can be dissociated rapidly and quantitatively in a short period of time.

Therefore, for example, when an aqueous liquid formulation that is particularly effective when used in its injectable form is administered at a pH value of preferably 4 or less, more preferably 3 or less, and even more preferably in the range of 2 to 3, its active form can be dissociated rapidly in the blood.

The aqueous solution formulation is produced by a well known method using additives such as stabilizers, flavoring agents, and diluents.

Examples of stabilizers include paraoxybenzoic acid esters such as methyl paraben, and propyl paraben; alcohols such as chlorobutanol, benzyl alcohol, and phenylethyl alcohol; benzalkonium chloride; phenols such as phenol and cresol; thimerosal; dehydroacetic acid; and sorbic acid.

Examples of flavoring agents include the commonly used sweetening agents, sour agents, and flavors.

Furthermore, examples of solvents that can be used to produce liquid agents include ethanol, phenol, chlorocresol, purified water, and distilled water.

Examples of surfactants or emulsifiers include polysorbate 80, polyoxyl 40 stearate, and lauromacrogol.

When using a pharmaceutical composition of the present invention as a therapeutic agent or preventive agent for a cell proliferation disorder, such as pancreatic cancer, ovarian cancer or liver cancer, the amounts of the water-soluble prodrugs represented by formulas (1) to (8), or their pharmaceutically acceptable salts, hydrates, or solvates that are used, will differ depending on the patient's symptoms, age, body weight, relative state of health, presence of other administered agents, method of administration, and such.

For example, the active ingredient (water-soluble prodrug) may be delivered to a patient (warm-blooded animal, especially human), at an effective dose; for example, a daily dose of preferably 0.001 to 1000 mg per kg body weight, and even more preferably 0.01 to 10 mg per kg body weight, in the case of a parenteral agent. This is suitably administered once daily or in several portions throughout the day, or alternatively, once in two to 60 days, or preferably once in five to 25 days, depending on the symptoms.

There are no limitations on the concentration of the water-soluble prodrugs represented by formulas (1) to (8), or their pharmaceutically acceptable salts, hydrates, or solvates in the aqueous solution, which differs depending on the extent and type of the disease, but is usually in a preferred range of 1 µM to 500 µM.

All prior art references cited herein are incorporated herein by reference.

### Examples

Hereinafter, an embodiment of the present invention is specifically illustrated with reference to Examples, but it is not to be construed as being limited thereto.

NMR analysis was performed using JEOL JNM-EX270 (270 MHz), JNMGSX400 (400 MHz), or JNM-A500 (500MHz). NMR data were reported in ppm (parts per million) and referenced to the deuterium lock signal of the sample solvent.

The mass spectral data were obtained using JEOL JMS-DX303, or JMS-SX/SX102A.

Data from mass spectrometers equipped with a high performance liquid chromatography instrument were obtained using micromass (Micromass, ZMD) equipped with a Waters 996-600E gradient high performance liquid chromatography instrument, or micromass (Finnigan, Navigator) equipped with an Agilent 1100 gradient high performance liquid chromatography instrument (Agilent Technologies).

For synthetic organic reactions, commercially available reagents were used without further purification.

In the Examples, room temperature refers to a temperature in the range of approximately 20 to 25°C.

All water-free reactions were performed under nitrogen atmosphere. Concentration or solvent removal under reduced pressure was performed using a rotary evaporator, unless stated otherwise.

Compounds were prepared while having their functional groups protected with protecting groups, as necessary, and the protecting groups were removed after the protected form of a target molecule was completed. Selection of the protecting group, and attachment and detachment manipulations were performed, for example, according to the methods described in "Greene and Wuts, Protective Group in Organic Synthesis /2nd edition, John Wiley & Sons, 1991".

High performance liquid chromatography employed one of the following two conditions.

### High performance liquid chromatography condition 1

Column: Combi ODS (ODS, 5 µm, 4.6 mm I.D. x 50 mm, Wako Pure Chemicals), COSMOSIL (ODS, 5 µm, 4.6 mm I.D. x 50 mm, Nakalai Tesque), or Inertsil C18 (ODS, 5 µm, 4.6 mm I.D. x 50 mm, GL Science)
Mobile phase: (Solvent A) water containing 0.01 % trifluoroacetic acid, and (Solvent B) acetonitrile containing 0.01% trifluoroacetic acid.
Elution method: stepwise gradient elution in Solvent B: 10% to 95% (3.5 minutes), 95% to 10% (1 minute), and 10% (0.5 minutes)
Flow rate: 4.0 mL/minute

### High performance liquid chromatography condition 2

Column: Combi ODS (ODS, 5 µm, 4.6 mm I.D. x 50 mm, Wako Pure Chemicals), COSMOSIL (ODS, 5 µm, 4.6 mm I.D. x 50 mm, Nakalai Tesque), or Inertsil C18 (ODS, 5 µm, 4.6 mm I.D. x 50 mm, GL Science)
Mobile phase: (Solvent A) water containing 0.01 % trifluoroacetic acid, and (Solvent B) acetonitrile containing 0.01 % trifluoroacetic acid.
Elution method: stepwise gradient elution in Solvent B: 30% to 35% (0.2 minutes), 35% to 98% (3.3 minute), 98% to 30% (1 minute), and 30% B (0.5 minutes)
Flow rate: 4.0 mL/minute

### [Example 1]

### (9S)-9-Ethyl-9-{[methyl-(2-methylamino-ethyl)-amino]-acetoxy}-1-pentyl-1H,12H-pyrano[3",4 ":6',7']indolizino[1',2':6,5]pyrido[4,3,2-de]quinazoline-10,13(9H,15H)-dione hydrochloride (compound 1A)

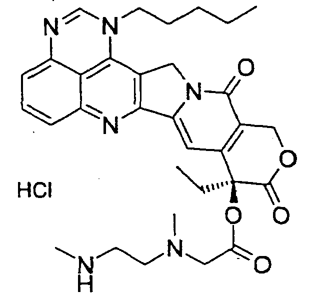

### Process 1-A

### {[2-(tert-Butoxycarbonyl-methyl-amino)-ethyl]-methyl-amino}-acetic acid benzyl ester

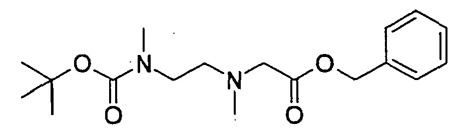

854 mg (4.54 mmol) of methyl-(2-methyl-amino-ethyl)-carbamic acid tert-butyl ester, which is a known substance (J. Med. Chem., 2000, 43, 3093), was dissolved in methylene chloride (50 mL), and then benzyl 2-bromoacetate (1.0 mL, 6.35 mmol) was added to this solution, and the mixture was stirred at room temperature for approximately 24 hours.

After completion of the reaction, the reaction solution was concentrated, and the residue obtained was purified by silica gel column chromatography (hexane:ethyl acetate = 5:1 to 3:1) to give 534.3 mg (35%) of {[2-(*tert*-butoxycarbonyl-methyl-amino)-ethyl]-methyl-amino}-acetic acid benzyl ester as a colorless viscous oil.
¹H-NMR (270MHz, CDCl₃) δ(ppm): 1.42 (9H, s), 2.40 (3H, s), 2.65 (2H, t, J = 7.1 Hz), 2.82 (3H, s), 3.20-3.38 (2H, m), 3.34 (2H, s), 5.13 (2H, s), 7.24-7.38 (5H, m)
ESI (LC-MS positive mode) m/z 337 (M+H).

### Process 1-B

### {[2-(tert-Butoxycarbonyl-methyl-amino)-ethyl]-methyl-amino}-acetic acid

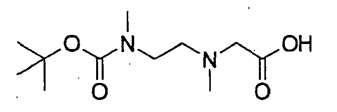

534.3 mg (1.59 mmol) of {[2-(*tert*-butoxycarbonyl-methyl-amino)-ethyl]-methyl-amino}-acetic acid benzyl ester prepared in Process 1-A was dissolved in methanol (20 mL), and then 51 mg of 5% palladium-carbon was added, and this was stirred for 1 hour under hydrogen atmosphere at room temperature. Insoluble substances were removed by filtration, and the filtrate was concentrated to give 391.1 mg (100%) of {[2-(*tert*-butoxycarbonyl-methyl-amino)-ethyl]-methyl-amino}-acetic acid as a colorless viscous oil.
¹H-NMR (270MHz, CDCl₃) δ(ppm): 1.43 (9H, s), 2.72 (3H, s), 2.87 (3H, s), 2.95-3.10 (2H, m), 3.45 (2H, s), 3.31-3.63 (2H, m)
ESI (LC-MS positive mode) m/z 247 (M+H)

### Process 1-C

### (9S)-9-({[2-(tert-Butoxycarbonyl-methyl-amino)-ethyl]-methyl-amino}-acetoxy)-9-ethyl-1-pent yl-1H,12H-pyrano[3",4":6',7']indolizino[1',2':6,5]pyrido[4,3,2-de]quinazoline-10,13 (9H,15H)-dione

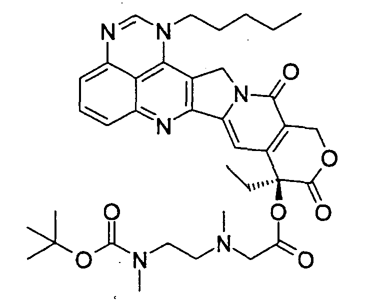

391 mg (1.59 mmol) of {[2-(*tert*-butoxycarbonyl-methyl-amino)-ethyl]-methyl-amino}-acetic acid prepared in Process 1-B, 279 mg (0.61 mmol) of (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione (prepared according to Example 2.15 of WO03/045952), 525 mg (2.74 mmol) of 1-ethyl-3-(3-dimethylaminopropyl)-carbodiimide hydrochloride, and 224 mg (1.83 mmol) of 4-dimethylaminopyridine were dissolved in methylene chloride (15 mL), and then stirred at room temperature for four hours.

The reaction solution was washed with 0.15 N aqueous hydrochloric acid, dried over anhydrous sodium sulfate, and then the solvent was distilled off under reduced pressure. By purifying the resulting residue using silica gel column chromatography (methylene chloride:methanol = 30:0 to 10:1), 136.4 mg (33%) of (9S)-9({[2-(*tert*-butoxycarbonyl-methyl-amino)-ethyl]-methyl-amino}-acetoxy)-9-ethyl-1-pentyl -1H;12H-pyrano[3",4":6',7']indolizino[1',2':6,5]pyrido[4,3,2-de]quinazoline-10,13(9H,15H)-di one was obtained as a yellow amorphous substance.
¹H-NMR (270MHz, CDCl₃) δ(ppm): 0.85-0.95 (6H, m), 1.27-1.50 (4H, m), 1.40 (9H, s), 1.70-1.84 (2H, m), 2.03-2.30 (2H, m), 2.39 (3H, s), 2.58-2.70 (2H, m), 2.80 (3H, s), 3.18-3.36 (2H, m), 3.48 (2H, s), 3.81 (2H, t, J = 7.3 Hz), 5.21 (2H, s), 5.37 (1H, d, J = 17.3 Hz), 5.64 (1H, d, J = 17.3 Hz), 7.07 (1H, s), 7.14 (1H, dd, J = 1.3, 7.3 Hz), 7.38 (1H, s), 7.53-7.70 (2H, m)
ESI (LC-MS positive mode) m/z 687 (M+H).

### Process 1-D

### (9S)-9-Ethyl-9- {[methyl-(2-methylamino-ethyl)-amino]-acetoxy}-1-pentyl-1H,12H-pyrano[3",4 ":6',7']indolizino[1',2':6,5]pyrido[4,3,2-de]quinazoline-10,13(9H,15H)-dione hydrochloride

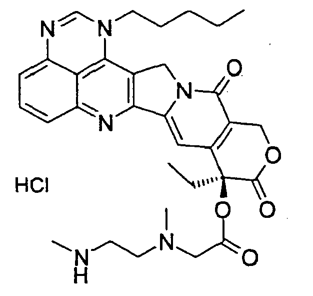

136.4 mg (0.20 mmol) of (9S)-9-({[2-(*tert*-butoxycarbonyl-methyl-amino)-ethyl]-methyl-amino}-acetoxy)-9-ethyl-1-penty 1-1H,12H-pyrano[3",4":6',7']indolizino[1',2':6,5]pyrido[4,3,2-de]quinazoline-10,13(9H,15H)-di one prepared in Process 1-C was dissolved in 1 N hydrochloric acid-acetic acid solution (3 mL), and then stirred at room temperature for 2.5 hours.

Ethyl acetate was added to the reaction solution, this was then stirred at room temperature for 30 minutes, and the resulting solid was collected by filtration, and 102.7 mg (74%) of (9S)-9-ethyl-9-{[methyl-(2-methylamino-ethyl)-amino]-acetoxy}-1-pentyl-1H,12H-pyrano[3",4":6',7']indolizino[1',2':6,5]pyrido[4,3,2-de]quinazoline-10,13(9H,15H)-dione hydrochloric acid (compound 1A) was obtained as a yellowish red solid.
¹H-NMR (270MHz, CD₃OD) δ(ppm): 0.99 (3H, t, J = 6.9 Hz), 1.09 (3H, t, J = 7.4 Hz), 1.40-1.62 (4H, m), 1.88-2.02 (2H, m), 2.15-2.31 (2H, m), 2.75 (3H, s), 2.96-3.06 (3H, m), 3.39-3.62 (4H, m), 4.25 (2H, t, J = 7.9 Hz), 4.44-4.60 (1H, m), 4.87-5.03 (1H, m), 5.53 (2H, s), 5.54 (1H, d, J = 17.2 Hz), 5.67 (1H, d, J = 17.2 Hz), 7.55 (1H, d, J = 7.3 Hz), 7.96-8.17 (3H, m), 8.33 (1H, s)
ESI (LC-MS positive mode) m/z 587 (M+H).

### [Example 2]

### (9S)-9-Ethyl-9-(glycyl-sarcosyloxy)-1-pentyl-1H,12H-pyrano[3",4":6',7']indolizino[1',2':6,5]pyrido[4,3,2-de]quinazoline-10,13 (9H,15H)-dione hydrochloride (compound 2A)

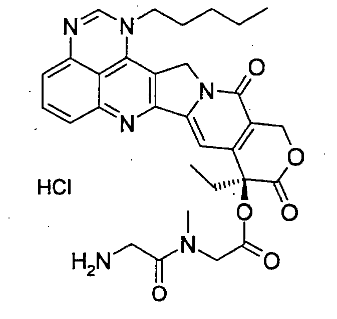

### Process 2-A

### (9S)-9-{[N-(tert-Butoxycarbonyl)-glycyl]-sarcosyloxy}-9-ethyl-1-pentyl-1H,12H-pyrano[3",4": 6',7']indolizino[1',2':6,5]pyrido[4,3,2-de]quinazoline-10,13(9H,15H)-dione

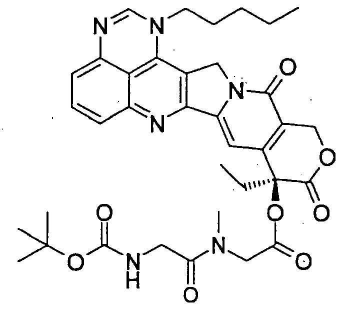

1.4 g (5.67 mmol) of [N-(*tert*-butoxycarbonyl)-glycyl]-sarcosine, which is a known substance (Helvetica Chimica Acta, 1991, 74, 197), 1.3 g (2.84 mmol) of (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione, 2.2 g (11.34 mmol) of 1-ethyl-3-(3-dimethylaminopropyl)-carbodiimide hydrochloride, and 1.4 g (11.34 mmol) of 4-dimethylaminopyridine were dissolved in methylene chloride (50 mL), and then stirred at room temperature for 1.5 hours.

The reaction solution was washed with 0.2 N aqueous hydrochloric acid and saturated aqueous sodium hydrogen carbonate solution, then dried over anhydrous sodium sulfate, and then the solvent was distilled off under reduced pressure. Purification of the resulting residue was performed using silica gel column chromatography (methylene chloride:methanol = 100:1 to 30:1), and as a result, 1.34 g (69%) of (9S)-9-{[N-(*tert*-butoxycarbonyl)-glycyl]-sarcosyloxy}-9-ethyl-1-pentyl-1H,12H-pyrano[3",4":6 ',7']indolizino[1',2':6,5]pyrido[4,3,2-de]quinazoline-10,13(9H,15H)-dione was obtained as a yellowish red amorphous substance.
¹H-NMR (270MHz, CDCl₃) δ(ppm):
Rotamer A* 0.86-1.04 (6H, m), 1.24-1.53 (4H, m), 1.38 (9H, s), 1.70-1.90 (2H, m), 2.03-2.36 (2H, m), 3.04 (3H, s), 3.83 (2H, t, J = 7.1 Hz), 3.96-4.05 (2H, m), 4.12 (1H, d, J = 17.7 Hz), 4.57 (1H, d, J = 17.7 Hz), 5.21 (2H, s), 5.38 (1H, d, J = 17.3 Hz), 5.46-5.56 (1H, m), 5.66 (1H, d, J = 17.3 Hz), 7.10-7.21 (2H, m), 7.40 (1H, s), 7.60-7.75 (2H, m);
Rotamer B* 0.86-1.04 (6H, m), 1.24-1.53 (4H, m), 1.38 (9H, s), 1.70-1.90 (2H, m), 2.03-2.36 (2H, m), 3.02 (3H, s), 3.83 (2H, t, J = 7.1 Hz), 3.90-3.96 (2H, m), 4.12 (1H, d, J = 17.7 Hz), 4.57 (1H, d, J = 17.7 Hz), 5.24 (2H, s), 5.38 (1H, d, J = 17.3 Hz), 5.46-5.56 (1H, m), 5.68 (1H, d, J = 17.3 Hz), 7.10-7.21 (2H, m), 7.40 (1H, s), 7.60-7.75 (2H, m).
*The ratio of the two rotamers, A and B, was approximately 5:1.
ESI (LC-MS positive mode) m/z 687 (M+H).

### Process 2-B

### (9S)-9-Ethyl-9-(glycyl-sarcosyloxy)-1-pentyl-1H,12H-pyrano[3",4":6',7']indolizino[1',2':6,5]py rido[4,3,2-de]quinazoline-10,13(9H,15H)-dione hydrochloride

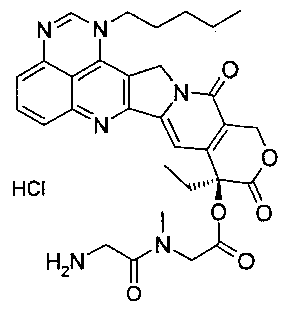

1.33 g (1.94 mmol) of (9S)-9- {[N-(*tert*-butoxycarbonyl)-glycyl]-sarcosyloxy}-9-ethyl-1-pentyl-1H, 12H-pyrano[3",4":6 ',7']indolizino[1',2':6,5]pyrido[4,3,2-de]quinazoline-10,13(9H,15H)-dione, which was prepared in Process 2-A, was dissolved in 1 N hydrochloric acid-acetic acid solution (15 mL), and then stirred at room temperature for 2 hours and 45 minutes.

Ethyl acetate was added to the reaction solution, which was then stirred at room temperature for 30 minutes, the resulting solid was collected by filtration, and 1.28 g (100%) of (9S)-9-ethyl-9-(glycyl-sarcosyloxy)-1-pentyl-1H,12H-pyrano[3",4":6',7']indolizino[1',2':6,5]py rido[4,3,2-de]quinazoline-10,13 (9H,15H)-dione hydrochloric acid (compound 2A) was obtained as a yellow solid.
¹H-NMR (270MHz, CD₃OD) δ(ppm):
Rotamer A* 0.99 (3H, t, J = 7.1 Hz), 1.03 (3H, t, J = 7.6 Hz), 1.34-1.58 (4H, m), 1.90-2.00 (2H, m), 2.14-2.30 (2H, m), 3.11 (3H, s), 3.99 (2H, s), 4.25 (2H, t, J = 7.3 Hz), 4.56 (1H, d, J = 17.9 Hz), 4.57 (1H, d, J = 17.9 Hz), 5.51 (1H, d, J = 17.4 Hz), 5.52 (2H, s), 5.61 (1H, d, J = 17.4 Hz), 7.52-7.57 (1H, m), 7.89 (1H, s), 7.80 (1H, d, J = 8.2 Hz), 8.10 (1H, t, J = 8.2 Hz), 8.38 (1H, s);
Rotamer B* 0.99 (3H, t, J = 7.1 Hz), 1.08 (3H, t, J = 7.3 Hz), 1.34-1.58 (4H, m), 1.90-2.00 (2H, m), 2.14-2.30 (2H, m), 3.08 (3H, s), 3.90 (1H, d, J = 16.5 Hz), 4.02 (1H, d, J = 16.5 Hz), 4.25 (2H, t, J = 7.3 Hz), 4.62 (1H, d, J = 18.8 Hz), 4.76 (1H, d, J = 18.8 Hz), 5.52 (2H, s), 5.53 (1H, d, J = 16.9 Hz), 5.65 (1H, d, J = 16.9 Hz), 7.52-7.57 (1H, m), 8.03 (1H, d, J = 8.2 Hz), 8.10 (1H, t, J = 8.2 Hz), 8.19 (1H, s), 8.34 (1H, s).
*The ratio of the two rotamers, A and B, was approximately 3:2.
ESI (LC-MS positive mode) m/z 587 (M+H).

### [Example 3]

### (9S)-9-{[(2-Amino-ethyl)-methyl-amino]-acetoxy}-9-ethyl-1-pentyl-1H,12H-pyrdno[3",4":6',7']indolizino[1',2':6,5]pyrido[4,3,2-de]quinazoline-10,13(9H,15H)-dione hydrochloride (compound 3A)

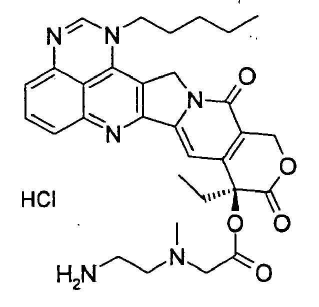

### Process 3-A

### {[2-(tert-Butoxycarbonylamino)-ethyl]-methyl-amino}-acetic acid benzyl ester

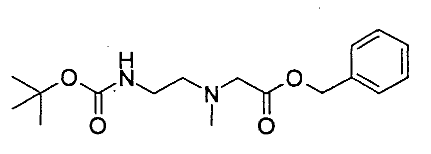

500 mg (1.42 mmol) of sarcosine benzyl ester *p*-toluenesulfonate, 478 mg (2.13 mmol) of 2-(*tert*-butoxycarbonylamino)-ethyl bromide, and 0.25 mL (2.13 mmol) of diisopropyl-ethylamine were dissolved in methylene chloride (10 mL), and then stirred at room temperature for approximately three days. After completion of the reaction, the reaction solution was concentrated and the residue obtained was purified by silica gel column chromatography (ethyl acetate) to yield 175 mg (38%) of {[2-(*tert*-butoxycarbonylamino)-ethyl]-methyl-amino}-acetic acid benzyl ester as a colorless viscous oil.
¹H-NMR (270MHz, CDCl₃) δ(ppm): 1.44 (9H, s), 2.37 (3H, s), 2.63 (2H, t, J = 6.0 Hz), 3.20 (2H, br.q), 3.33 (2H, s), 5.15 (1H, br.s), 5.16 (2H, s), 7.32-7.39 (5H, m)
ESI (LC-MS positive mode) m/z 323 (M+H).

### Process 3-B

### {[2-(tert-Butoxycarbonylamino)-ethyl]-methyl-amino}-acetic acid

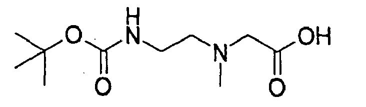

162 mg (0.5 mmol) of {[2-(*tert*-butoxycarbonylamino)-ethyl]-methyl-amino}-acetic acid benzyl ester prepared in Process 3-A was dissolved in methanol (5 mL), and then 5% palladium-carbon was added to it, and this was stirred under a hydrogen atmosphere at room temperature for one hour. After filtering off the insoluble material, the filtrate was concentrated to yield 116 mg (100%) of {[2-(*tert*-butoxycarbonylamino)-ethyl]-methyl-amino}-acetic acid as a colorless viscous oil.
¹H-NMR (270MHz, CD₃OD) δ(ppm): 1.45 (9H, s), 2.91(3H, s), 3.22 (2H, t, J = 6.3 Hz), 3.41 (2H, t, J = 6.3 Hz), 3.63 (2H, s)
ESI (LC-MS positive mode) m/z 233 (M+H).

### Process 3-C

### (9S)-9-({[2-(tert-Butoxycarbonylamino)-ethyl]-methyl-amino}-acetoxy)-9-ethyl-1-pentyl-1H,12 H-pyrano[3";4":6',7']indolizino[1',2':6,5]pyrido[4,3,2-de]quinazoline-10,13(9H,15H)-dione

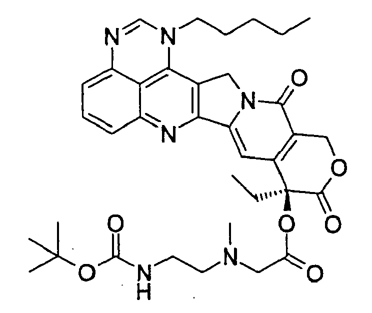

112 mg (0.48 mmol) of {[2-(*tert*-butoxycarbonylamino)-ethyl]-methyl-amino}-acetic acid prepared in Process 3-B, 157 mg (0.34 mmol) of (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione, 197 mg (1.03 mmol) of 1-ethyl-3-(3-dimethylaminopropyl)-carbodiimide hydrochloride, and 83 mg (0.68 mmol) of 4-dimethylaminopyridine were dissolved in methylene chloride (10 mL), and then stirred at room temperature for three hours.

The reaction solution was washed with 0.3 N aqueous hydrochloric acid solution, dried over anhydrous sodium sulfate, and then the solvent was distilled off under reduced pressure. By purifying the obtained residue by silica gel column chromatography (methylene chloride:methanol = 50:1), 61 mg (27%) of (9S)-9-({[2-(*tert*-butoxycarbonylamino)-ethyl]-methyl-amino}-acetoxy)-9-ethyl-1-pentyl-1H,12 H-pyrano[3",4":6',7']indolizino[1',2':6,5]pyrido[4,3,2-de]quinazoline-10,13(9H,15H)-dione was obtained as a yellow viscous oil.
¹H-NMR (270MHz, CDCl₃) δ(ppm): 0.88-1.00 (6H, m), 1.32-1.53 (4H,m), 1.42 (9H, s), 1.73-1.86 (2H, m), 2.08-2.36 (2H, m), 2.39 (3H, s), 2.63 (2H, t, J =5.9 Hz), 3.20 (2H,br.q), 3.46 (2H, s), 3.82 (2H, t, J = 7.1 Hz), 5.21 (1H, br.s), 5.22 (2H, s), 5.40 (1H, d, J = 17.2 Hz), 5.67 (1H, d, J = 17.2 Hz), 7.10 (1H, s), 7.14 (1H, br.d), 7.40 (1H, br.s), 7.58-7.70 (2H, m)
ESI (LC-MS positive mode) m/z 673 (M+H).

### Process 3-D

### (9S)-9-{[(2-Amino-ethyl)-methyl-amino]-acetoxy}-9-ethyl-1-pentyl-1H,12H-pyrano[3",4":6',7'] indolizino[1',2':6,5]pyrido[4,3,2-de]quinazoline-10,13(9H,15H)-dione hydrochloride

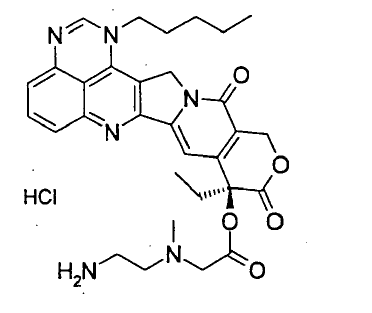

58 mg (0.086 mmol) of (9S)-9-({[2-(*tert*-butoxycarbonylamino)-ethyl]-methyl-amino}-acetoxy)-9-ethyl-1-pentyl-1H,12 H-pyrano[3",4":6',7']indolizino[1',2':6,5]pyrido[4,3,2-de]quinazoline-10,13(9H,15H)-dione prepared in Process 3-C was dissolved in 1N hydrochloric acid-acetic acid solution (2 mL), and then stirred at room temperature for four hours.

Ethyl acetate was added to the reaction solution, which was then stirred at room temperature for 30 minutes, and the resulting solid was collected by filtration, and thus, 54 mg (93%) of (9S)-9-{[(2-amino-ethyl)-methyl-amino]-acetoxy}-9-ethyl-1-pentyl1H,12H-pyrano[3",4":6',7']i ndolizino[1',2':6,5]pyrido[4,3,2-de]quinazoline-10,13(9H,15H)-dionehydrochloride (compound 3A) was obtained as a yellow solid.
¹H-NMR (270MHz, CD₃OD) δ(ppm): 0.99 (3H, t, J = 6.9 Hz), 1.09 (3H, t, J = 7.3 Hz), 1.39-1.62 (4H, m), 1.88-2.02 (2H, m), 2.16-2.30 (2H, m), 3.07 (3H, s), 3.37-3.61 (4H, m), 4.25 (2H, br.t), 4.62 (1H, d, J = 17.5 Hz), 5.05 (1H, d, J = 17.5 Hz), 5.52 (2H, s), 5.53 (1H, d, J = 17.5 Hz), 5.67 (1H, d, J = 17.5 Hz), 7.55 (1H, br.d), 7.98-8.16 (3H, m), 8.32 (1H, s)
ESI (LC-MS positive mode) m/z 573 (M+H).

### [Example4]

### (9S)-9-Ethyl-9-(sarcosyl-sarcosyloxy)-1-pentyl-1H,12H-pyrano[3",4":6',7']indolizino[1',2':6,5]pyrido[4,3,2-de]quinazoline-10,13(9H,5H)-dione hydrochloride (compound 4A)

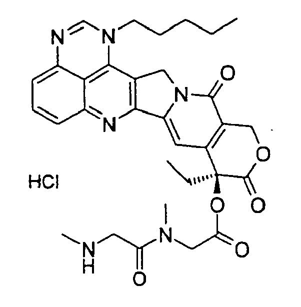

### Process 4-A

### (9S)-9-{[N-tert-Butoxycarbonyl)-sarcosyl]-sarcosyloxy}-9-ethyl-1-pentyl-1H,12H-pyrano[3",4":6',7']indolizino[1',2':6,5]pyrido[4,3,2-de]quinazoline-10,13(9H,15H)-dione

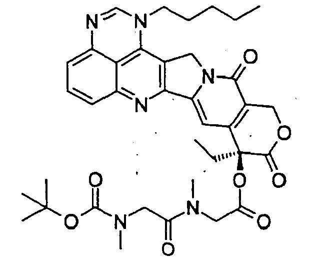

113 mg (0.44 mmol) of [N-(*tert*-butoxycarbonyl)-sarcosyl]-sarcosine, which is a known substance, 100 mg (0.22 mmol) of (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione, 125 mg (0.65 mmol) of 1-ethyl-3-(3-dimethylaminopropyl)-carbodiimide hydrochloride, and 53 mg (0.44 mmol) of 4-dimethylaminopyridine were dissolved in methylene chloride (6 mL), and then stirred at room temperature for 3.5 hours.

The reaction solution was washed with 0.25 N aqueous hydrochloric acid and aqueous sodium hydrogen carbonate solution, dried over anhydrous sodium sulfate, and then the solvent was distilled off under reduced pressure. The obtained residue was purified by silica gel column chromatography (methylene chloride:methanol = 50:1), 103 mg (68%) of (9S)-9-{[N-*tert*-butoxycarbonyl)-sarcosyl]-sarcosyloxy}-9-ethyl-1-pentyl-1H,12H-pyrano[3",4":6',7']indolizino[1',2':6,5]pyrido[4,3,2-de]quinazoline-10,13(9H,15H)-dione was obtained as a yellow amorphous substance.
¹H-NMR (400MHz, CDCl₃) δ(ppm):
0.88-1.02 (6H, m), 1.25-1.51(13H, m), 1.73-1.82 (2H, m), 2.11-2.32 (2H, m), 2.84 (3H, m), 3.02 (3H, m), 3.80 (2H, br.t), 3.92-4.29 (3H, m), 4.58-4.71 (1H, m), 5.12-5.70 (4H, m), 7.04-7.19 (2H, m), 7.39 (1H, m), 7.55-7.68 (2H, m)
ESI (LC-MS positive mode) m/z 701 (M+H).

### Process 4-B

### (9S)-9-Ethyl-9-(sarcosyl-sarcosyloxy)-1-pentyl-1H,12H-pyrano[3",4":6',7']indolizino[1',2':6,5] pyrido[4,3,2-de]quinazoline-10,13(9H,15H)-dione hydrochloride

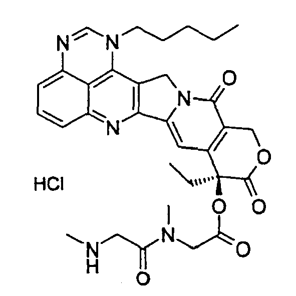

98 mg (0.14 mmol) of (9S)-9-{[N-*tert*-butoxycarbonyl)-sarcosyl]-sarcosyloxy}-9-ethyl-1-pentyl-1H,12H-pyrano[3",4":6',7']indolizino[1',2':6,5]pyrido[4,3,2-de]quinazoline-10,13(9H,15H)-dione prepared in Process 4-A was dissolved in 1 N hydrochloric acid-acetic acid solution (3 mL), and then stirred at room temperature for two hours.

Ethyl acetate was added to the reaction solution, which was then stirred at room temperature for 30 minutes, and the resulting solid was collected by filtration, and thus, 67 mg (71 %) of (9S)-9-ethyl-9-(sarcosyl-sarcosyloxy)-1-pentyl-1H,12H-pyrano[3",4":6',7']indolizino[1',2':6,5] pyrido[4,3,2-de]quinazoline-10,13(9H,15H)-dione hydrochloride (compound 4A) was obtained as a yellow solid.
¹H-NMR (270MHz, CD₃OD) δ(ppm):
0.96-1.11 (6H, m), 1.40-1.62 (4H, m), 1.87-2.01 (2H, m), 2.15-2.31 (2H, m), 2.71 (3H, s), 3.10 (3H, m), 4.05-4.15 (2H, m), 4.24 (2H, br.t), 4.55-4.70 (2H, m), 5.46-5.70 (4H, m), 7.52 (1H, m), 7.81-8.15 (3H, m), 8.34 (1H, m)
ESI (LC-MS positive mode) m/z 601 (M+H).

### [Example 5]

### (Aminoacetyl-methyl-amino-acetic acid (S)-4-ethyl-3,13-dioxo-3,4,12,13-tetrahydro-1H-2-oxa-6,12a-diaza-dibenzo[b,h]fluoren-4-yl ester hydrochloride (compound 5A)

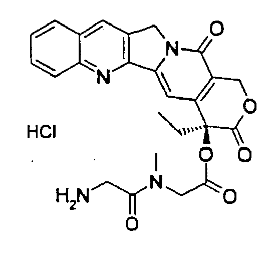

### Process 5-A

### (tert-Butoxycarbonylaminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester

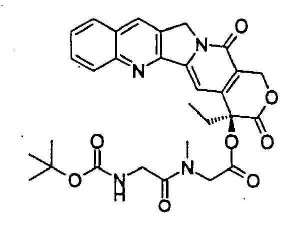

441 mg (1.93 mmol) of N-(*tert*-butoxycarbonyl)-glycyl]-sarcosine, 250 mg (0.77 mmol) of camptothecin, 343 mg (1.93 mmol) of 1-ethyl-3-(3-dimethylaminopropyl)-carbodiimide hydrochloride, and 87 mg (0.77 mmol) of 4-dimethylaminopyridine were dissolved in methylene chloride (130 mL), and then stirred at room temperature for 15 hours.

The reaction solution was washed with 0.3 N aqueous hydrochloric acid and sodium hydrogen carbonate, and dried over anhydrous sodium sulfate, and then the solvent was distilled off under reduced pressure. By purifying the obtained residue by silica gel column chromatography (methylene chloride:methanol = 30:1), 410 mg (99%) of (*tert*-butoxycarbonylaminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester was obtained as a yellow solid.
¹H-NMR (270MHz, CDCl₃) δ(ppm):
1.00 (3H, t, J = 7.6Hz), 1.35(9H, s), 2.08-2.34 (2H, m), 3.05 (3H, s), 3.90-4.24 (3H, m), 4.61 (1H, m), 5.28 (2H, br.s), 5.40 (1H, d, J = 17.2 Hz), 5.47 (1H, br.s), 5.69 (1H, d, J = 17.2 Hz), 7.30 (1H, s), 7.68 (1H, br.t), 7.84 (1H, br.t), 7.93 (1H, br.d), 8.29 (1H, br.d), 8.40 (1H, s)
FAB-MS (positive mode) m/z 577 (M+H).

### Process 5-B

### (Aminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester hydrochloric acid

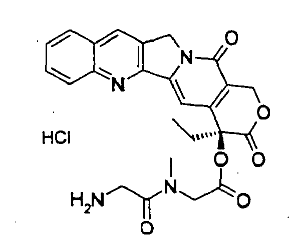

39 mg (0.068 mmol) of (*tert*-butoxycarbonylaminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester prepared in Process 5-A was dissolved in 1 N hydrochloric acid-ethyl acetate solution (2 mL), and then stirred at room temperature for four hours.

Ethyl acetate was added to the reaction solution, this was then stirred at room temperature for 30 minutes, and the resulting solid was collected by filtration to yield 28 mg (82%) of (aminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester hydrochloride (compound 5A) as a yellow solid.
¹H-NMR (270MHz, DMSO-d₆) δ(ppm):
0.92 (3H, t, J = 7.3Hz), 2.11-2.24 (2H, m), 2.92-2.98 (3H, m), 3.90 (2H, m), 4.40-4.78 (2H, m), 5.30 (2H, br.s), 5.40-5.60 (2H, m), 7.18-7.27 (1H, m), 7.75 (1H, br.t), 7.89 (1H, br.t), 8.12-8.28 (2H, m), 8.25(br.s), 8.72 (1H, s)
ESI (LC-MS positive mode) m/z 477 (M+H).

### [Example 6]

### (9S)-9-{2-[(R-2-A-mino-2-methoxycarbonyl)ethanesulfonyl]ethoxycarbonyloxy}-9-ethyl-1-penty 1-1H,12H-pyrano[3",4":6',7']indolizino[1',2':6,5]pyrido[4,3,2-de]quinazoline-10,13(9H,15H)-di one trifluoroacetic acid salt (compound 6A)

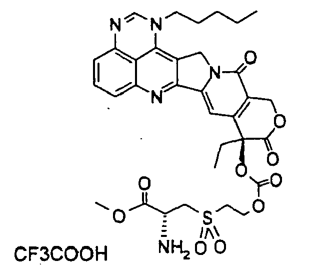

### Process 6-A

### (9S)-9-(2-Bromo-ethoxycarbonyloxy)-9-ethyl-1-pentyl-1H,12H-pyrano[3",4":6',7']indolizino[1' ,2' :6,5]pyrido[4,3;2-de]quinazoline-10,13(9H,15H)-dione

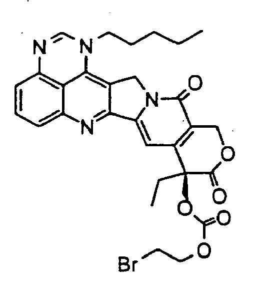

620 mg (1.35 mmol) of (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione, 0.29 mL (2.70 mmol) of 2-bromoethyl chloroformate, 0.47 mL (2.70 mmol) of diisopropyl-ethylamine, and 165 mg (1.35 mmol) of 4-dimethylaminopyridine were dissolved in methylene chloride (20 mL), and then stirred at room temperature for approximately 24 hours.

The reaction solution was washed with 0.3 N aqueous hydrochloric acid, dried over anhydrous sodium sulfate, and then the solvent was distilled off under reduced pressure. By purifying the obtained residue by silica gel column chromatography (ethyl acetate), 681 mg (83%) of (9S)-9-(2-bromo-ethoxycarbonyloxy)-9-ethyl-1-pentyl-1H,12H-pyrano[3",4":6',7']indolizino[1',2':6,5]pyrido[4,3,2-de]quinazoline-10,13(9H,15H)-dione was obtained as a yellow solid.
¹H-NMR (270MHz, CDCl₃) δ(ppm): 0.93 (3H, t, J = 7.3 Hz), 1.00 (3H, t, J = 7.6 Hz), 1.31-1.54 (4H, m), 1.72-1.86 (2H, m), 2.08-2.35 (2H, m), 3.50 (2H, t, J = 6.2Hz), 3.83 (2H, t, J = 7.3Hz), 4.41 (2H, t, J = 6.2Hz), 5.23 (2H, s), 5.37 (1H, d, J = 17.0 Hz), 5.68 (1H, d, J = 17.0 Hz), 7.16 (1H, br.d), 7.22 (1H, s), 7.40 (1H, s), 7.59-7.71 (2H, m)
ESI (LC-MS positive mode) m/z 609, 611 (M+H).

### Process 6-B

### (9S)-9-{2-[(R-2-tert-Butoxycarbonylamino-2-methoxycarbonyl)ethanesulfanyl]ethoxycarbonylo xy}-9-ethyl-1-pentyl-1H,12H-pyrano[3",4":6',7']indolizino[1',2':6,5]pyrido[4,3,2-de]quinazoline-10,13(9H,15H)-dione

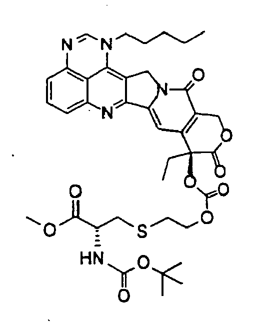

50 mg (0.082 mmol) of (9S)-9-(2-bromo-ethoxycarbonyloxy)-9-ethyl-1-pentyl-1H,12H-pyrano[3",4":6',7']indolizino[1',2':6,5]pyrido[4,3,2-de]quinazoline-10,13(9H,15H)-dione prepared in Process 6-A, 58 mg (0.25 mmol) of *tert*-butoxycarbonyl-cysteine methyl ester, and 34 mg (0.25 mmol) of potassium carbonate were stirred in acetonitrile (2mL), at room temperature for six hours.

Following the addition of methylene chloride to the reaction mixture, the solution was washed with 0.3 N aqueous hydrochloric acid, dried over anhydrous sodium sulfate, and then the solvent was distilled off under reduced pressure. By purifying the obtained residue by silica gel column chromatography (methylene chloride:methanol = 50:1), 51 mg (82%) of (9S)-9-{2-[(R-2-*tert*-butoxycarbonylamino-2-methoxycarbonyl)ethanesulfanyl]ethoxycarbonylo xy}-9-ethyl-1-pentyl-1H,12H-pyrano[3",4":6',7']indolizino[1',2':6,5]pyrido[4,3,2-de]quinazolin e-10,13(9H,15H)-dione was obtained as a yellow solid.
¹H-NMR (270MHz, CDCl₃) δ(ppm): 0.93 (3H, t, J = 7.0 Hz), 0.98 (3H, t, J = 7.6 Hz), 1.30-1.54 (4H, m), 1.41 (9H, s), 1.68-1.86 (2H, m), 2.05-2.34 (2H, m), 2.78 (2H, t, J = 6.8Hz), 2.89-3.05 (2H, m), 3.71 (3H, s), 3.82 (2H, t, J = 7.3Hz), 4.23 (2H, t, J = 6.8Hz), 4.46 (1H, m), 5.23 (2H, s), 5.37 (1H, d, J = 17.0 Hz), 5.42 (1H, m), 5.67 (1H, d, J = 17.0 Hz), 7.17 (1H, br.d), 7.22 (1H, s), 7.40 (1H, s), 7.60-7.70 (2H, m)
ESI (LC-MS positive mode) m/z 764 (M+H).

### Process 6-C

### (9S)-9-{2-[(R-2-tert-Butoxycarbonylamino-2-methoxycarbonyl)ethanesulfonyl]ethoxycarbonyloxy} -9-ethyl-1-pentyl-1H,12H-pyrano[3",4":6',7']indolizino[1',2'6,5]pyrido[4,3,2-de] quinazolin e-10,13(9H,15H)-dione

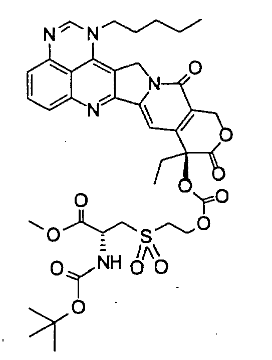

98 mg (0.13 mmol) of (9S)-9-{2-[(R-2-*tert*-butoxycarbonylamino-2-methoxycarbonyl)ethanesulfanyl]ethoxycarbonylo xy}-9-ethyl-1-pentyl-1H,12H-pyrano[3",4":6',7']indolizino[1',2':6,5]pyrido[4,3,2-de]quinazolin e-10,13(9H,15H)-dione prepared in Process 6-B, and 158 mg (0.26 mmol) of Oxone™ were stirred in methanol (5 mL) at room temperature for 2.5 hours.

Methylene chloride was added to the reaction mixture, the mixture was washed with water and dried over anhydrous sodium sulfate, and the solvent was distilled off under reduced pressure. By purifying the obtained residue by silica gel column chromatography (methylene chloride:methanol = 40:1), 93 mg (92%) of (9S)-9-{2-[(R-2-*tert*-butoxycarbonylamino-2-methoxycarbonyl)ethanesulfonyl]ethoxycarbonylo xy}-9-ethyl-1-pentyl-1H,12H-pyrano[3",4":6',7']indolizino[1',2':6,5]pyrido[4,3,2-de]quinazolin e-10,13(9H,15H)-dione was obtained as a yellow solid.
ESI (LC-MS positive mode) m/z 796 (M+H).

### Process 6-D

### (9S)-9-{2-[(R-2-Amino-2-methoxycarbonyl)ethanesulfonyl]ethoxycarbonyloxy}-9-ethyl-1-penty 1-1H,12H-pyrano[3",4":6',7']indolizino[1',2':6,5]pyrido[4,3,2-de]quinazoline-10,13(9H,15H)-di one trifluoroacetate

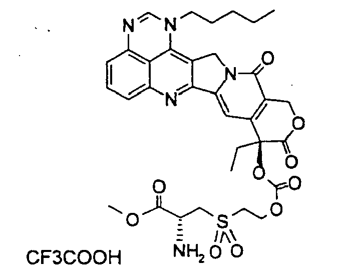

93 mg (0.12 mmol) of (9S)-9-{2-[(R-2-*tert*-butoxycarbonylamino-2-methoxycarbonyl)ethanesulfonyl]ethoxycarbonyloxy}-9-ethyl-1-pentyl-1H,12H-pyrano[3",4":6',7']indolizino[1',2':6,5]pyrido[4,3,2-de]quinazolin e-10,13(9H,15H)-dione prepared in Process 6-C, was dissolved in trifluoroacetic acid (3 mL), and then stirred at room temperature for one hour.

Diethyl ether was added to the reaction solution, and stirred at room temperature for 10 minutes. The resulting solid was collected by filtration to yield 88 mg (94%) of (9S)-9-{2-[(R-2-amino-2-methoxycarbonyl)ethanesulfonyl]ethoxycarbonyloxy}-9-ethyl-1-pentyl -1H,12H-pyrano[3",4":6',7']indolizino[1',2':6,5]pyrido[4,3,2-de]quinazoline-10,13(9H,15H)-di one trifluoroacetate (compound 6A) as a yellow solid.
¹H-NMR (270MHz, CD₃OD) δ(ppm): 0.95 (3H, t, J = 7.3 Hz), 1.03 (3H, t, J = 7.3 Hz), 1.37-1.56 (4H, m), 1.76-1.92 (2H, m), 2.10-2.28 (2H, m), 3.69-4.05 (6H, m), 3.90 (3H, s), 4.48-4.82 (3H, m), 5.35 (2H, s), 5.48 (1H, d, J = 16.8 Hz), 5.64 (1H, d, J = 16.8 Hz), 7.13 (1H, br.d), 7.28 (1H, s), 7.56 (1H, d, J = 8.3 Hz), 7.71 (1H, m),7.80 (1H, s)
ESI (LC-MS positive mode) m/z 696 (M+H).

### [Example 7]

### (9S)-9-{2-[(R-2-Amino-2-ethoxycarbonyl)ethanesulfonyl]ethoxycarbonyloxy}-9-ethyl-1-pentyl-1H,12H-pyrano[3",4":6',7']indolizino[1',2':6,5]pyrido[4,3,2-de]quinazoline-10,13(9H,15H)-dio ne hydrochloride (compound 7A)

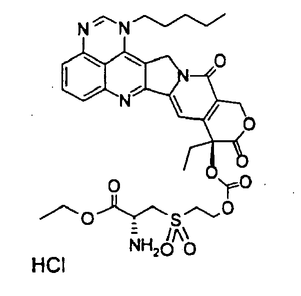

### Process 7-A

### (9S)-9-{2-[(R-2-tert-Butoxycarbonylamino-2-ethoxycarbonyl)ethanesulfanyl]ethoxycarbonyloxy }-9-ethyl-1-pentyl-1H,12H-pyrano[3",4":6',7']indolizino[1',2':6,5]pyrido[4,3,2-de]quinazoline-10,13(9H,15H)-dione

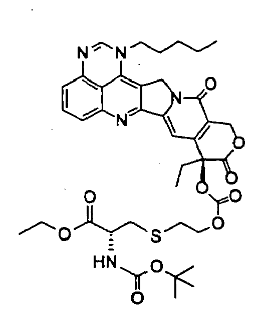

270 mg (0.44 mmol) of (9S)-9-(2-bromo-ethoxycarbonyloxy)-9-ethyl-1-pentyl-1H,12H-pyrano[3",4":6',7']indolizino[1',2':6,5]pyrido[4,3,2-de]quinazoline-10,13(9H,15H)-dione, 333 mg (1.33 mmol) of *tert*-butoxycarbonyl-cysteine ethyl ester, and 184 mg (1.33 mmol) of potassium carbonate were stirred in acetonitrile (10 mL) at room temperature for 20 hours.

Following the addition of methylene chloride to the reaction mixture, the mixture was washed with 0.3 N aqueous hydrochloric acid, dried over anhydrous sodium sulfate, and the solvent was distilled off under reduced pressure. By purifying the obtained residue by silica gel column chromatography (methylene chloride:methanol = 50:1), 159 mg (46%) of (9S)-9-{2-[(R-2-*tert*-butoxycarbonylamino-2-ethoxycarbonyl)ethanesulfanyl]ethoxycarbonyloxy }-9-ethyl-1-pentyl-1H,12H-pyrano[3",4":6',7']indolizino[1',2':6,5]pyrido[4,3,2-de]quinazoline-10,13(9H,15H)-dione was obtained as a yellow oil.
¹H-NMR (270MHz, CDCl₃) δ(ppm): 0.93 (3H, t, J = 7.3 Hz), 0.97 (3H, t, J = 7.3 Hz), 1.25 (3H, t, J = 7.3 Hz), 1.32-1.53 (4H, m), 1.41 (9H, s), 1.73-1.85 (2H, m), 2.05-2.35 (2H, m), 2.79 (2H, t, J = 6.9Hz), 2.88-3.05 (2H, m), 3.83 (2H, t, J = 7.3Hz), 4.09-4.28 (4H, m), 4.43 (1H, m), 5.23 (2H, s), 5.37 (1H, d, J = 17.2 Hz), 5.43 (1H, m), 5.67 (1H, d, J = 17.2 Hz), 7.17 (1H, br.d), 7.21 (1H, s), 7.40 (1H, s), 7.60-7.70 (2H, m)
ESI (LC-MS positive mode) m/z 778 (M+H).

### Process 7-B

### (9S)-9-{2-[(R-2-tert-Butoxycarbonylamino-2-ethoxycarbonyl)ethanesulfonyl]ethoxycarbonylox y}-9-ethyl-1-pentyl-1H,12H-pyrano[3",4":6',7']indolizino[1',2':6,5]pyrido[4,3,2-de]quinazoline -10,13(9H,15H)-dione

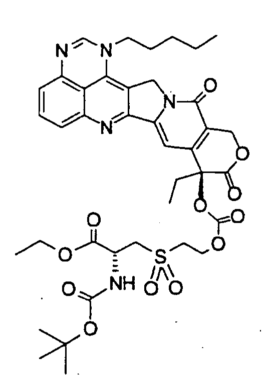

159 mg (0.20 mmol) of (9S)-9-{2-[(R-2-*tert*-butoxycarbonylamino-2-ethoxycarbonyl)ethanesulfanyl]ethoxycarbonyloxy }-9-ethyl-1-pentyl-1H,12H-pyrano[3",4":6',7']indolizino[1',2':6,5]pyrido[4,3,2-de]quinazoline-10,13(9H,15H)-dione prepared in Process 7-A, and 252 mg (0.41 mmol) of Oxone™ were stirred in methanol (8 mL) at room temperature for two hours.

Following the addition of methylene chloride to the reaction mixture, the mixture was washed with water and dried over anhydrous sodium sulfate, and the solvent was distilled off under reduced pressure. By purifying the obtained residue by silica gel column chromatography (methylene chloride:methanol = 40:1), 141 mg (88%) of (9S)-9-{2-[(R-2-*tert*-butoxycarbonylamino-2-ethoxycarbonyl)ethanesulfonyl]ethoxycarbonyloxy }-9-ethyl-1-pentyl-1H,12H-pyrano[3",4":6',7']indolizino[1',2':6,5]pyrido[4,3,2-de]quinazoline-10,13(9H,15H)-dione was obtained as a yellow solid.
¹H-NMR (270MHz, CDCl₃) δ(ppm): 0.93 (3H, t, J = 6.9 Hz), 0.99 (3H, t, J = 7.6 Hz), 1.29 (3H, t, J = 7.3 Hz), 1.32-1.55 (4H, m), 1.45 (9H, s), 1.72-1.87 (2H, m), 2.07-2.32 (2H, m), 3.25-3.52 (2H, m), 3.73 (2H, m), 3.84 (2H, t, J = 7.3Hz), 4.23 (2H, q, J = 7.3Hz), 4.55 (2H, br.t), 4.71 (1H, m), 5.25 (2H, s), 5.37 (1H, d, J = 17.2 Hz), 5.69 (1H, d, J = 17.2 Hz), 5.76 (1H, d, J =7.9Hz), 7.18 (1H, br.d), 7.19 (1H, s), 7.40 (1H, s), 7.61-7.72 (2H, m)
ESI (LC-MS positive mode) m/z 810 (M+H).

### Process 7-C

### (9S)-9-{2-[R-2-Amino-2-ethoxycarbonyl)ethanesulfonyl]ethoxycarbonyloxy}-9-ethyl-1-pentyl-1H,12H-pyrano[3",4":6',7']indolizino[1',2':6,5]pyrido[4,3,2-de]quinazoline-10,13(9H,15H)-dion e hydrochloride

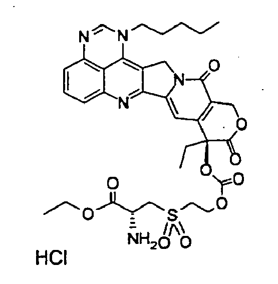

140 mg (0.17 mmol) of (9S)-9-{2-[(R-2-*tert*-butoxycarbonylamino-2-ethoxycarbonyl)ethanesulfonyl]ethoxycarbonyloxy }-9-ethyl-1-pentyl-1H,12H-pyrano[3",4":6',7']indolizino[1',2':6,5]pyrido[4,3,2-de]quinazoline-10,13(9H,15H)-dione prepared in Process 7-B, was dissolved in 1N hydrochloric acid-acetic acid solution (5 mL), and then stirred at room temperature for one hour.

Ethyl acetate was added to the reaction solution, and stirred at room temperature for 30 minutes. The resulting solid was collected by filtration to yield 127 mg (94%) of (9S)-9-{2-[R-2-amino-2-ethoxycarbonyl)ethanesulfonyl]ethoxycarbonyloxy}-9-ethyl-1-pentyl-1 H,12H-pyrano[3",4":6',7']indolizino[1',2':6,5]pyrido[4,3,2-de]quinazoline-10,13(9H,15H)-dion e hydrochloride (compound 7A) as a yellow solid.
¹H-NMR (400MHz, CD₃OD) δ(ppm): 0.99 (3H, t, J = 7.6 Hz), 1.04 (3H, t, J = 7.2 Hz), 1.36 (3H, t, J = 7.2 Hz), 1.40-1.60 (4H, m), 1.92-2.00 (2H, m), 2.10-2.25 (2H, m), 3.70-3.86 (3H, m), 4.10 (1H, dd, J =15.2,4.0Hz), 4.25 (2H, t, J = 8.0Hz), 4.36 (2H, q, J = 7.6Hz), 4.60 (2H, t, J = 5.2Hz), 4.77 (1H, m), 5.52 (1H, d, J = 16.8 Hz), 5.54 (2H, s), 5.65 (1H, d, J = 16.8 Hz), 7.54 (1H, d, J = 7.6 Hz), 7.83 (1H, s), 7.91 (1H, d, J = 8.0 Hz), 8.09 (1H, br.t), 8.38 (1H, s)
ESI (LC-MS positive mode) m/z 710 (M+H).

### [Example 8]

### (9S)-9-[2-(2-Aminoethanesulfonyl)ethoxycarbonyloxy]-9-ethyl-1-pentyl-1H,12H-pyrano[3",4": 6',7']indolizino[1',2':6,5]pyrido[4,3,2-de]quinazoline-10,13(9H,15H)-dione hydrochloride (compound 8A)

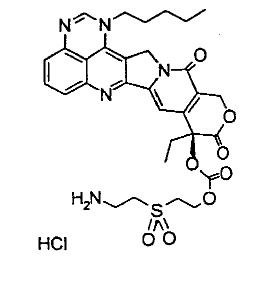

### Process 8-A

### (9S)-9-[2-(2-tert-Butoxycarbonylaminoethanesulfonyl)ethoxycarbonyloxy]-9-ethyl-1-pentyl-1H, 12H-pyrano[3",4":6',7']indolizino[1',2':6,5]pyrido[4,3,2-de]quinazoline-10,13(9H,15H)-dione

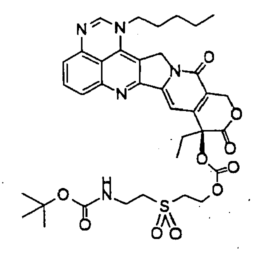

825 mg (1.8 mmol) of (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione, 543 mg (2.7 mmol) of *p*-nitrophenyl chloroformate, 0.47 mL (2.7 mmol) of diisopropyl-ethylamine, and 220 mg (1.8 mmol) of 4-dimethylaminopyridine were dissolved in methylene chloride (16 mL) on ice, and then stirred at room temperature for three hours. Next, 1.6 g (6.3 mmol) of 2-(2-*tert*-butoxycarbonylaminoethanesulfonyl)ethanol (Tetrahedron, 55 (1999), 6623-6634) was added to this solution, and this was stirred at room temperature for approximately 24 hours.

The reaction solution was washed with 0.3 N aqueous hydrochloric acid, dried over anhydrous sodium sulfate, and then the solvent was distilled off under reduced pressure. By purifying the obtained residue by silica gel column chromatography (methylene chloride methanol = 30:1), 550 mg (42%) of (9S)-9-[2-(2-*tert*-butoxycarbonylaminoethanesulfonyl)ethoxycarbonyloxy]-9-ethyl-1-pentyl-1H, 12H-pyrano[3",4":6',7']indolizino[1',2':6,5]pyrido[4,3,2-de]quinazoline-10,13(9H,15H)-dione was obtained as a yellow solid.
¹H-NMR (270MHz, CDCl₃) δ(ppm): 0.94 (3H, t, J = 7.3 Hz), 1.00 (3H, t, J = 7.3 Hz),. 1.33-1.55 (4H, m), 1.43 (9H, s), 1.73-1.86 (2H, m), 2.07-2.31 (2H, m), 3.20-3.49 (4H, m), 3.70 (2H, m), 3.84 (2H, t, J = 7.3Hz), 4.57 (2H, br.t), 5.25 (2H, s), 5.37 (1H, d, J = 17.2 Hz), 5.45 (1H, m), 5.69 (1H, d, J = 17.2 Hz), 7.15-7.20 (2H, m), 7.41 (1H, s), 7.61-7.73 (2H, m)
ESI (LC-MS positive mode) m/z 738 (M+H).

### Process 8-B

### (9S)-9-[2-(2-Aminoethanesulfonyl)ethoxycarbonyloxy]-9-ethyl-1-pentyl-1H,12H-pyrano[3",4": 6',7']indolizino[1',2':6,5]pyrido[4,3,2-de]quinazoline-10,13(9H,15H)-dione hydrochloride

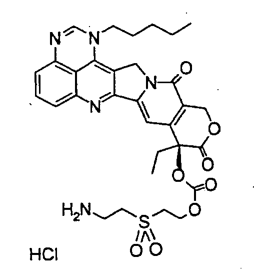

530 mg (0.72 mmol) of (9S)-9-[2-(2-*tert*-butoxycarbonylaminoethanesulfonyl)ethoxycarbonyloxy]-9-ethyl-1-pentyl-1H, 12H-pyrano[3",4":6',7']indolizino[1',2':6,5]pyrido[4;3,2-de]quinazoline-10,13(9H,15H)-dione prepared in Process 8-A was dissolved in 1 N hydrochloric acid-acetic acid solution (10 mL), and then stirred at room temperature for one hour.

Ethyl acetate was added to the reaction solution, which was then stirred at room temperature for 30 minutes, and the resulting solid was collected by filtration to yield 480 mg (94%) of (9S)-9[2-(2-aminoethanesulfonyl)ethoxycarbonyloxy]-9-ethyl-1-pentyl-1H,12H-pyrano[3",4":6' , 7']indolizino[1',2':6,5]pyrido[4,3,2-de]quinazoline-10,13(9H,15H)-dione hydrochloride (compound 8A) as a yellow solid.
¹H-NMR (270MHz, CD₃OD) δ(ppm): 0.94-1.05 (6H, m), 1.40-161 (4H, m), 1.90-2.04 (2H, m), 2.07-2.28 (2H, m), 3.48-3.81 (6H, m), 4.25 (2H, br.t), 4.55 (2H, m), 5.51 (1H, d, J = 17.2 Hz), 5.53 (2H, s), 5.65 (1H, d, J = 17.2 Hz), 7.52 (1H, d, J = 7.6 Hz), 7.90 (1H, s), 7.93 (1H, d, J = 8.6 Hz), 8.08 (1H, br.t), 8.35 (1H, s)
ESI (LC-MS positive mode) m/z 638 (M+H).

### [Test Example 1]

Parenteral administration of the water-soluble camptothecin prodrugs represented by the above mentioned formula (2) into mouse models of human pancreatic cancer exhibited strong cancer growth inhibition activity. Thus, these prodrug compounds are useful as antitumor agents. An example of the antitumor activity is shown below.

### Determination of antitumor effects

Antitumor effects were determined for representative examples of the compound groups of the present invention. Antitumor effects were determined using tumor-bearing mice generated by subcutaneous transplantation of human pancreatic cancer cell line AsPC-1 obtained from American Type Culture Collection (Virginia, USA) into the right ventral neck of BALB/c nude mice purchased from Japan Charles River.

After a one-week quarantine period, the purchased nude mice were subjected to subcutaneous transplantation of 4.8 x 10⁶ AsPC-1 cells to their right ventral neck. Mice with a tumor size reaching 200 mm³ or so were subjected to the experiment.

The compounds were dissolved in 1 mM citric acid/physiological saline (pH3.1-3.2), and were administered intravenously. The administration was carried out once a week for three weeks, for a total of three administrations. The antitumor effect was determined as tumor growth inhibition (TGI), using the following equation.

Tumor growth inhibition (TGI) (%) = {(1- amount of tumor growth in the group treated with the drug/amount of tumor growth in the control group) x 100} The results are shown in Table 3.

**Table 3**

| Compound | Dose (mg/kg/inj.) | TGI (%) on Day 21 |
|---|---|---|
| Compound 2A | 60 (47 in terms of free base) | 73 |

### [Test Example 2]

Parenteral administration of the water-soluble camptothecin prodrugs represented by the above-mentioned formula (2) into mouse models of human pancreatic cancer showed strong activity of cancer growth inhibition. Thus, these prodrug compounds are useful as antitumor agents. An example of the antitumor activity is shown below.

### Determination of antitumor effects

Antitumor effects were determined for representative examples of the compound groups of the present invention. Antitumor effects were determined using tumor-bearing mice generated by subcutaneous transplantation of human pancreatic cancer cell line Capan-1 obtained from American Type Culture Collection (Virginia, USA) into the right ventral neck of BALB/c nude mice purchased from Japan Charles River.

After a 19-day quarantine period, the purchased nude mice were subjected to subcutaneous transplantation of 10 x 10⁶ Capan-1 cells to their right ventral neck. Mice with a tumor size reaching 260 mm³ or so were subjected to the experiment.

The compounds were dissolved in 1 mM citric acid/physiological saline (pH3.1-3.2), and were administered intravenously. The administration was carried out once a week for three weeks, for a total of three administrations. The antitumor effect was determined as tumor growth inhibition (TGI), using the following equation.

Tumor growth inhibition (TGI) (%) = {(1- amount of tumor growth in the group treated with the drug/amount of tumor growth in the control group) x 100} The results are shown in Table 4.

**Table 4**

| Compound | Dose (mg/kg/inj.) | TGI (%) on Day 21 |
|---|---|---|
| Compound 2A | 66 (47 in terms of free base) | 122 |

### [Test Example 3]

Parenteral administration of the water-soluble camptothecin prodrugs represented by the above-mentioned formula (2) into mouse models of human pancreatic cancer showed strong cancer growth inhibition activity. Thus, these prodrug compounds are useful as antitumor agents. An example of the antitumor activity is shown below.

### Determination of antitumor effects

Antitumor effects were determined for representative examples of the compound groups of the present invention. Antitumor effects were determined using tumor-bearing mice generated by subcutaneous transplantation of human pancreatic cancer cell line BxPC-3 obtained from American Type Culture Collection (Virginia, USA) into the right ventral neck of BALB/c nude mice purchased from Japan Charles River.

After a seven-day quarantine period, the purchased nude mice were subjected to subcutaneous transplantation of 9 x 10⁶ BxPC-3 cells to their right ventral neck. Mice with a tumor size reaching 400 mm³ or so were subjected to the experiment.

The compounds were dissolved in 1 mM citric acid/physiological saline (pH3.1-3.2), and were administered intravenously. The administration was carried out once a week for three weeks, for a total of three administrations. The antitumor effect was determined as tumor growth inhibition (TGI), using the following equation.

Tumor growth inhibition (TGI) (%) = {(1- amount of tumor growth in the group treated with the drug/amount of tumor growth in the control group) x 100} The results are shown in Table 5.

**Table 5**

| Compound | Dose (mg/kg/inj.) | TGI (%) on Day 14 | TGI (%) on Day 21 |
|---|---|---|---|
| Compound 2A | 66 (47 in terms of free base) | 55 | 47 |

### [Test Example 4]

Parenteral administration of the water-soluble camptothecin prodrugs represented by the above-mentioned formula (2) into mouse models of human ovarian cancer showed strong cancer growth inhibition activity. Thus, these prodrug compounds are useful as antitumor agents. An example of the antitumor activity is shown below.

### Determination of antitumor effects

Antitumor effects were determined for representative examples of the compound groups of the present invention. Antitumor effects were determined using tumor-bearing mice generated by the subcutaneous transplantation of human ovarian cancer cell line PA-1 obtained from American Type Culture Collection (Virginia, USA) into the right ventral neck of BALB/c nude mice purchased from Japan Charles River.

After a one-week quarantine period, the purchased nude mice were subjected to subcutaneous transplantation of 9.8 x 10⁶ PA-1 cells to their right ventral neck. Mice with a tumor size reaching 200 mm³ or so were subjected to the experiment.

The compounds were dissolved in physiological saline, and were administered intravenously. The administration was carried out once a week for three weeks, for a total of three administrations. The antitumor effect was determined as tumor growth inhibition (TGI), using the following equation.

Tumor growth inhibition (TGI) (%) = {(1- amount of tumor growth in the group treated with the drug/amount of tumor growth in the control group) x 100} The results are shown in Table 6.

**Table 6**

| Compound | Dose (mg/kg/inj.) | TGI (%) on Day 21 |
|---|---|---|
| Compound 2A | 40 (32 in terms of free base) | 84 |

### [Test Example 5]

Parenteral administration of the water-soluble camptothecin prodrugs represented by the above-mentioned formula (2) into mouse models of human ovarian cancer showed strong cancer growth inhibition activity. Thus, these prodrug compounds are useful as antitumor agents. An example of the antitumor activity is shown below.

### Determination of antitumor effects

Antitumor effects were determined for representative examples of the compound groups of the present invention. Antitumor effects were determined using tumor-bearing mice generated by subcutaneous transplantation of the human ovarian cancer cell line SK-OV-3 obtained from American Type Culture Collection (Virginia, USA) into the right ventral neck of BALB/c nude mice purchased from Japan Charles River.

After a six-day quarantine period, the purchased nude mice were subjected to subcutaneous transplantation of a 2-3 mm square piece of the SK-OV-3 tumor to their right ventral neck. Mice with a tumor size reaching 210 mm³ or so were subjected to the experiment.

The compounds were dissolved in physiological saline, and were administered intravenously. The administration was carried out once a week for three weeks, for a total of three administrations. The antitumor effect was determined as tumor growth inhibition (TGI), using the following equation.

Tumor growth inhibition (TGI) (%) = {(1- amount of tumor growth in the group treated with the drug/amount of tumor growth in the control group) x 100} The results are shown in Table 7.

**Table 7**

| Compound | Dose (mg/kg/inj.) | TGI (%) on Day 21 |
|---|---|---|
| Compound 2A | 60 (47 in terms of free base) | 52 |

### [Test Example 6]

Parenteral administration of the water-soluble camptothecin prodrugs represented by the above-mentioned formula (2) into mouse models of human liver cancer showed strong cancer growth inhibition activity. Thus, these prodrug compounds are useful as antitumor agents. An example of the antitumor activity is shown below.

### Determination of antitumor effects

Antitumor effects were determined for representative examples of the compound groups of the present invention. Antitumor effects were determined using tumor-bearing mice generated by subcutaneous transplantation of human liver cancer cell line PLC/PRF/5 obtained from Human Science into the right ventral neck of BALB/c nude mice purchased from Japan Charles River.

After a one-week quarantine period, the purchased nude mice were subjected to subcutaneous transplantation of 20 x 10⁶ PLC/PRF/5 cells to their right ventral neck. Mice with a tumor size reaching 200 mm³ or so were subjected to the experiment.

The compounds were dissolved in physiological saline, and were administered intravenously. The administration was carried out once a week for three weeks, for a total of three administrations. The antitumor effect was determined as tumor growth inhibition (TGI), using the following equation.

Tumor growth inhibition (TGI) (%) = {(1- amount of tumor growth in the group treated with the drug/amount of tumor growth in the control group) x 100} The results are shown in Table 8.

**Table 8**

| Compound | Dose (mg/kg/inj.) | TGI (%) on Day 21 |
|---|---|---|
| Compound 2A | 60 (47 in terms of free base) | 84 |

Although compound 2A is a hydrochloride salt, by changing the reaction batch, reaction scale, reaction solvent, reaction time, reaction temperature, types and equivalents of reagents, methods of treating the reaction solution, normality (N) of the aqueous hydrochloric acid solution used when washing the reaction solution in step 2-A, and/or normality of hydrochloric acid used in step 2-B, there may be difference in the amount (mole ratio with respect to the free base) of hydrochloric acid added as salt to the free camptothecin derivatives (free base). Therefore, the dose of compound 2A used in Test Examples 1 to 6 was indicated both in terms of the dose based on the actual measurement of the weight and the dose based on the weight obtained by converting the actually measured weight to the free base weight.

### Industrial Applicability

Since water-soluble prodrugs used in the present invention, pharmaceutically acceptable salts thereof, or hydrates or solvates thereof have ester chains of particular structures, they show excellent water-solubility, because of their rapid chemical conversion to the active form, the interspecies or individual differences are small. Water-soluble prodrugs used in the present invention, pharmaceutically acceptable salts thereof, or hydrates or solvates thereof are extremely useful for application to water-insoluble pharmaceutical agents such as camptothecin, which comprise an alcoholic hydroxyl group and are effective as preventive or therapeutic agents for cell proliferative disorders such as pancreatic cancer, ovarian cancer, and liver cancer.

## Claims

1. A preventive or therapeutic agent for pancreatic cancer, ovarian cancer, or liver cancer comprising a water-soluble prodrug represented by formula (1), or a pharmaceutically acceptable salt thereof, or a hydrate or solvate of the prodrug or pharmaceutically acceptable salt, 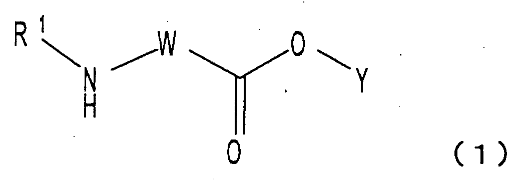 (wherein,
R¹ represents a hydrogen atom, or a C1-C6 alkyl group;
W represents a divalent group comprising a tertiary amino group or a divalent group comprising a sulfonyl group; and
Y represents a residue of a compound represented by Y-OH comprising an alcoholic hydroxyl group, wherein said Y-OH is a camptothecin, a taxane, or an anticancer nucleotide).

2. The preventive or therapeutic agent for pancreatic cancer, ovarian cancer, or liver cancer of claim 1, wherein the water-soluble prodrug is represented by formula (2): 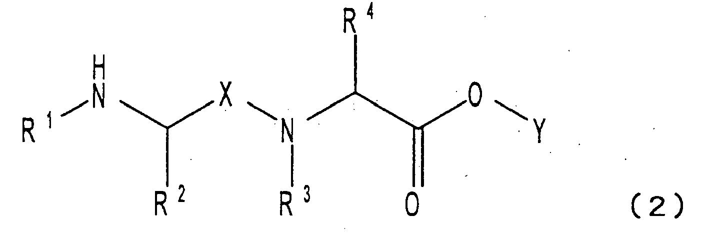 (wherein,
R¹ and Y are defined as in formula (1);
X represents a C=O or a C1-C3 alkylene group;
R² and R⁴ each independently represents a hydrogen atom, a C1-C6 alkyl group, or an amino acid side chain; andR³ represents a C1-C6 alkyl group).

3. The preventive or therapeutic agent for pancreatic cancer, ovarian cancer, or liver cancer of claim 2, wherein R¹ is a hydrogen atom, a methyl group, or an ethyl group.

4. The preventive or therapeutic agent for pancreatic cancer, ovarian cancer, or liver cancer of claim 2 or 3, wherein R² is a hydrogen atom or a methyl group.

5. The preventive or therapeutic agent for pancreatic cancer, ovarian cancer, or liver cancer of any one of claims 2 to 4, wherein R³ is a C1-C3 alkyl group.

6. The preventive or therapeutic agent for pancreatic cancer, ovarian cancer, or liver cancer of any one of claims 2 to 5, wherein R⁴ is a hydrogen atom or a methyl group.

7. The preventive or therapeutic agent for pancreatic cancer, ovarian cancer, or liver cancer of claim 1, wherein the water-soluble prodrug is represented by formula (3): 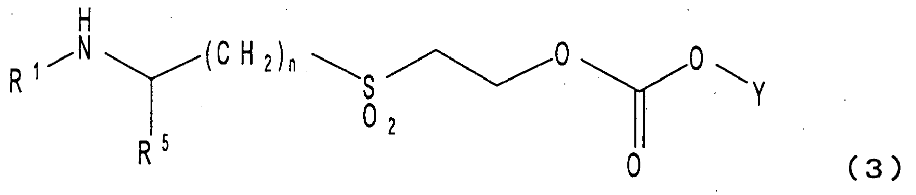 [wherein,
R¹ and Y are defined as in formula (1);
n represents an integer from 1 to 6; and
R⁵ represents a hydrogen atom or -COOR⁶ (wherein R⁶ represents a hydrogen atom, or a C1-C6 alkyl group)].

8. The preventive or therapeutic agent for pancreatic cancer, ovarian cancer, or liver cancer of claim 7, wherein R¹ is a hydrogen atom, a methyl group, or an ethyl group.

9. The preventive or therapeutic agent for pancreatic cancer, ovarian cancer, or liver cancer of claim 7 or 8, wherein n is 1, and R⁵ is a hydrogen atom or -COOR⁶ (wherein R⁶ represents a hydrogen atom or a C1-C6 alkyl group).

10. The preventive or therapeutic agent for pancreatic cancer, ovarian cancer, or liver cancer of claim 7 or 8, wherein n is an integer from 2 to 6, and R⁵ is a hydrogen atom.

11. The preventive or therapeutic agent for pancreatic cancer, ovarian cancer, or liver cancer of claims 1 to 10, wherein the hydroxyl group (-OH) of Y-OH is a secondary or tertiary alcoholic hydroxyl group.

12. The preventive or therapeutic agent for pancreatic cancer, ovarian cancer, or liver cancer of any one of claims 1 to 11, wherein Y-OH is an insoluble compound.

13. The preventive or therapeutic agent for pancreatic cancer, ovarian cancer, or liver cancer of any one of claims 1 to 12, wherein Y is a group represented by formula (4): 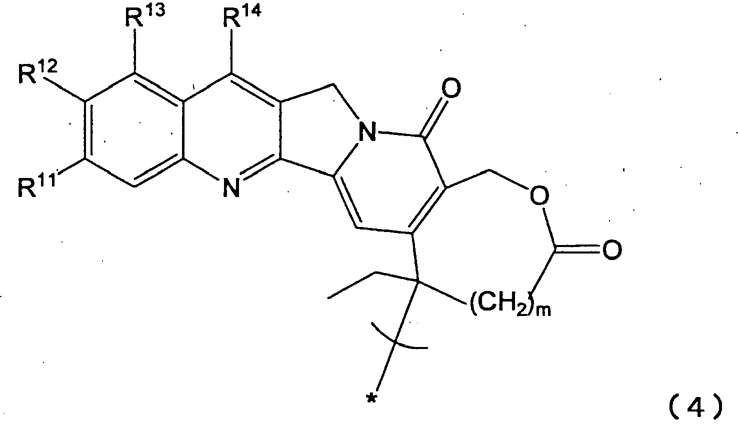 [wherein,
* indicates a linkage site;
m is either 0 or 1;
R¹¹ represents a hydrogen atom, a halogen atom, or a C1-C6 alkyl group;
R¹² represents a hydrogen atom, a halogen atom, a C1-C6 alkyl group, or a hydroxyl group;
R¹³ represents a hydrogen atom, an amino group, a nitro group, or a (dimethylamino)methyl group;
R¹⁴ represents a hydrogen atom, a C1-C6 alkyl group, a (4-methylpiperazinyl)methyl group, or a (*tert*-butoxyimino)methyl group;
R¹³ and R¹⁴, and R¹¹ and R¹², may each be linked to each other to form a 5- or 6-membered ring, wherein the 5- or 6-membered ring may comprise one to two heteroatoms, and one to three substituents selected from Group A described below, wherein the substituents of Group A may further comprise one to three substituents selected from Group B described below:
Group A: a C1-C10 alkyl group, an amino group, a mono-C1-C8 alkylamino group, a di-C1-C8 alkylamino group, a C1-C8 alkoxy group, a C1-C8 alkylthio group, and a group represented by X= (wherein X represents an oxygen atom or a sulfur atom);
Group B: a C1-C6 alkoxy group, a hydroxy group, a halogen atom, an amino group, a mono-C1-C6 alkylamino group, a di-C1-C6 alkylamino group, a C3-C7 cycloalkyl group, a heterocycle, and an aryl ring (the aryl ring may comprise one to three substituents selected from the group consisting of a hydroxy group, a C1-C6 alkoxy group, a halogen atom, an amino group, a mono-C1-C6 alkylamino group, and a
di-C1-C6 alkylamino group)].

14. The preventive or therapeutic agent for pancreatic cancer, ovarian cancer, or liver cancer of claim 13, wherein Y is a group represented by formula (5): 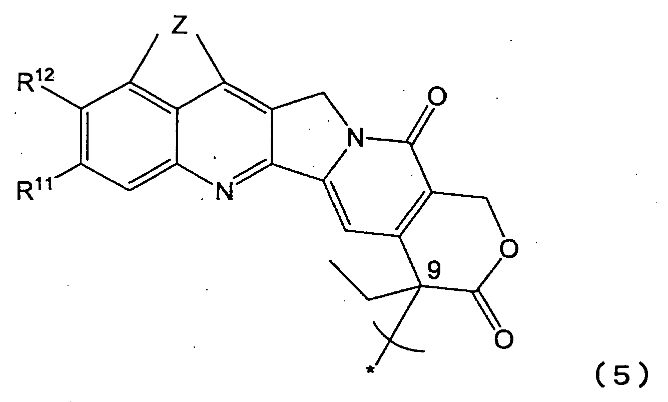 {wherein,
* indicates a linkage site;
R¹¹ and R¹² are each defined as in claim 13; and
Z represents -NH-C(=X)-N(R²¹)- or -N=C(R²²)-N(R²¹)-
[wherein R²¹ represents a hydrogen atom or a C1-C10 alkyl group that may comprise one to three substituents selected from Group B described below:
Group B: a C1-C6 alkoxy group, a hydroxy group, a halogen atom, an amino group, a mono-C1-C6 alkylamino group, a di-C1-C6 alkylamino group, a C3-C7 cycloalkyl group, a heterocycle, and an aryl ring (the aryl ring may comprise one to three substituents selected from the group consisting of a hydroxy group, a C1-C6 alkoxy group, a halogen atom, an amino group, a mono-C1-C6 alkylamino group, and a di-C1-C6 alkylamino group);
R²² represents a hydrogen atom, an amino group, or a C1-C6 alkyl group that may comprise one to three substituents selected from Group C described below, a C1-C6 alkoxy group that may comprise one to three substituents selected from Group C described below, a C1-C6 alkylthio group that may comprise one to three substituents selected from Group C described below, a mono-C1-C6 alkylamino group that may comprise one to three substituents selected from Group C described below, or a di-C1-C6 alkylamino group that may comprise one to three substituents selected from Group C described below:
Group C: a C1-C6 alkoxy group, a hydroxy group, a halogen atom, an amino group, a mono-C1-C6 alkylamino group, a di-C1-C6 alkylamino group, a C3-C7 cycloalkyl group, a heterocycle, and an aryl ring (the aryl ring may comprise one to three substituents selected from the group consisting of a hydroxy group, a C1-C6 alkoxy group, an amino group, a halogen atom, a mono-C1-C6 alkylamino group, and a di-C1-C6 alkylamino group); and
X represents an oxygen atom or a sulfur atom]}.

15. The preventive or therapeutic agent for pancreatic cancer, ovarian cancer, or liver cancer of claim 14, wherein Y is a group represented by formula (6): 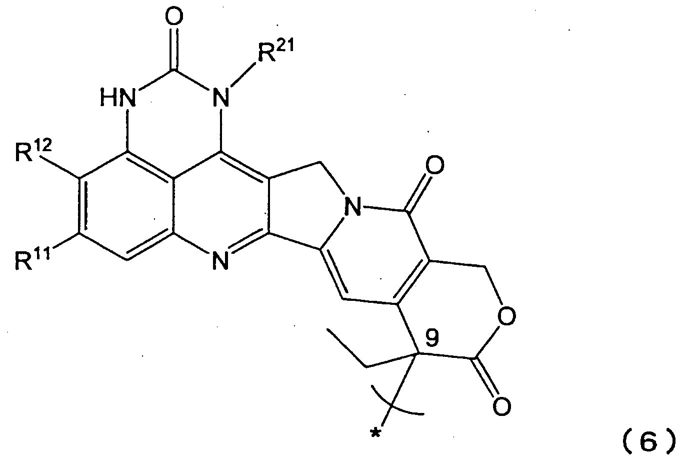 [wherein * indicates a linkage site; and
R¹¹, R¹², and R²¹ are each defined as in claim 14].

16. The preventive or therapeutic agent for pancreatic cancer, ovarian cancer, or liver cancer of claim 15, wherein R¹¹ and R¹² are hydrogen atoms; and
R²¹ is a hydrogen atom, or a C1-C8 alkyl group that may comprise a substituent selected from Group D described below:
Group D: a C1-C3 alkoxy group, a hydroxy group, a halogen atom, an amino group, a mono-C1-C3 alkylamino group, a di-C1-C3 alkylamino group, a C3-C7 cycloalkyl group, a heterocycle, and an aryl ring (the aryl ring may comprise one to three substituents selected from the group consisting of a hydroxy group, a C1-C3 alkoxy group, and a halogen atom).

17. The preventive or therapeutic agent for pancreatic cancer, ovarian cancer, or liver cancer of claim 15 or 16, wherein Y is a residue of a compound (Y-OH) comprising at least one alcoholic hydroxyl group, wherein the compound is selected from the group consisting of:
a) (9S)-1-butyl-9-ethyl-9-hydroxy-1H,12H-pyrano[3",4":6',7']indolizino[1',2':6,5]pyrido[4,3,2-de]quinazoline-2,10,13(3H,9H,15H)-trione;
b) (9S)-9-ethyl-9-hydroxy-1-[2-(4-morpholino)ethyl]-1H,12H-pyrano[3",4":6',7']indolizino[1',2':6,5]pyrido[4,3,2-de]quinazoline-2,10,13(3H,9H,15H)-trione;
c) (9S)-1-[3-(dimethylamino)propyl]-9-ethyl-9-hydroxy-1H,12H-pyrano[3",4":6',7']indolizino[1',2':6,5]pyrido[4,3,2-de]quinazoline-2,10,13(3H,9H,15H)-trione;
d) (9S)-9-ethyl-9-hydroxy-1-phenethyl-1H,12H-pyrano[3",4":6',7']indolizino[1',2':6,5]pyrido[4,3,2-de]quinazoline-2,10,13(3H,9H,15H)-trione;
e) (9S)-9-ethyl-9-hydroxy-1-[2-(pyridin-2-yl)ethyl]-1H,12H-pyrano[3",4":6',7']indolizino[1',2':6,5]pyrido[4,3,2-de]quinazoline-2,10,13(3H,9H,15H)-trione;
f) (9S)-9-ethyl-1-heptyl-9-hydroxy-1H,12H-pyrano[3",4":6',7']indolizino[1',2':6,5]pyrido[4,3,2-de]quinazoline-2,10,13(3H,9H,15H)-trione;
g) (9S)-9-ethyl-9-hydroxy-1-propyl-1H,12H-pyrano[3",4":6',7']indolizino[1',2':6,5]pyrido[4,3,2-de]quinazoline-2,10,13(3H,9H,15H)-trione;
h) (9S)-9-ethyl-9-hydroxy-1-[2-(pyridin-3-yl)ethyl]-1H,12H-pyrano[3",4":6',7']indolizino[1',2':6,5]pyrido[4,3,2-de]quinazoline-2,10,13(3H,9H,15H)-trione;
i) (9S)-9-ethyl-9-hydroxy-1-(3-phenylpropyl)-1H,12H-pyrano[3",4":6',7']indolizino[1',2':6,5]pyrido[4,3,2-de]quinazoline-2,10,13(3H,9H,15H)-trione;
j) (9S)-9-ethyl-9-hydroxy-1-(2-methylpropyl)-1H,12H-pyrano[3",4":6',7']indolizino[1',2':6,5]pyrido[4,3,2-de]quinazoline-2,10,13(3H,9H,15H)-trione;
k) (9S)-9-ethyl-1-hexyl-9-hydroxy-1H,12H-pyrano[3",4":6',7']indolizino[1',2':6,5]pyrido[4,3,2-de]quinazoline-2,10,13(3H,9H,15H)-trione;
l) (9S)-9-ethyl-9-hydroxy-1-pentyl-1H,12H-pyrano[3",4":6',7']indolizino[1',2':6,5]pyrido[4,3,2-de]quinazoline-2,10,13(3H,9H,15H)-trione;
m) (9S)-9-ethyl-9-hydroxy-1-[2-(4-methoxyphenyl)ethyl]-1H,12H-pyrano[3",4":6',7']indolizino[1',2' :6,5]pyrido[4,3,2-de]quinazoline-2,10,13(3H,9H,15H)-trione;
n) (9S)-1-benzyl-9-ethyl-9-hydroxy-1H,12H-pyrano[3",4":6',7']indolizino[1',2':6,5]pyrido[4,3,2-de]quinazoline-2,10,13(3H,9H,15H)-trione;
o) (9S)-9-ethyl-9-hydroxy-1-(3-methylbutyl)-1H,12H-pyrano[3",4":6',7']indolizino[1',2':6,5]pyrido [4,3,2-de]quinazoline-2,10,13(3H,9H,15H)-trione;
P) (9S)-1,9-diethyl-9-hydroxy-1H,12H-pyrano[3",4"6',7']indolizino[1',2':6,5]pyrido[4,3,2-de]quinazoline-2,10,13(3H,9H,15H)-trione;
q) (9S)-1-[2-(4-chlorophenyl)ethyl]-9-ethyl-9-hydroxy-1H,12H-pyrano[3",4":6',7']indolizino[1',2':6,5]pyrido[4,3,2-de]quinazoline-2,10,13(3H,9H,15H)-trione;
r) (9S)-9-ethyl-1-[2-(4-fluorophenyl)ethyl]-9-hydroxy-1H,12H-pyrano[3",4":6',7']indolizino[1',2':6,5]pyrido[4,3,2-de]quinazoline-2,10,13(3H,9H,15H)-trione;
s) (9S)-9-ethyl-9-hydroxy-1-(1-methylethyl)-1H,12H-pyrano[3",4":6',7']indolizino[1',2':6,5]pyrido[4,3,2-de]quinazoline-2,10,13(3H,9H,15H)-trione; and
t) (9S)-1-(3,3-dimethylbutyl)-9-ethyl-9-hydroxy-1H,12H-pyrano[3",4":6',7']indolizino[1',2':6,5]pyrido[4,3,2-de]quinazoline-2,10,13(3H,9H,15H)-trione.

18. The preventive or therapeutic agent for pancreatic cancer, ovarian cancer, or liver cancer of claim 14, wherein Y is a group represented by formula (7): 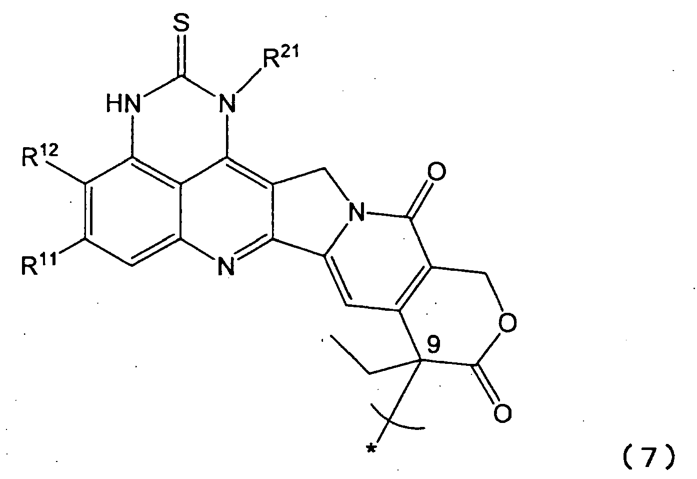 (wherein,
* indicates a linkage site; and
R¹¹, R¹², and R²¹ are each defined as in claim 14).

19. The preventive or therapeutic agent for pancreatic cancer, ovarian cancer, or liver cancer of claim 18, wherein R¹¹ and R¹² are hydrogen atoms; and
R²¹ is a hydrogen atom, or a C1-C8 alkyl group that may comprise a substituent selected from Group D described below:
Group D: a C1-C3 alkoxy group, a hydroxy group, a halogen atom, an amino group, a mono-C1-C3 alkylamino group, a di-C1-C3 alkylamino group, a C3-C7 cycloalkyl group, a heterocycle, and an aryl ring (the aryl ring may comprise one to three substituents selected from the group consisting of a hydroxy group, a C 1-C3 alkoxy group, and a halogen atom).

20. The preventive or therapeutic agent for pancreatic cancer, ovarian cancer, or liver cancer of claim 18 or 19, wherein Y is a residue of a compound (Y-OH) comprising at least one alcoholic hydroxyl group, wherein the compound is selected from the group consisting of:
a) (9S)-9-ethyl-9-hydroxy-1-(3-methylbutyl)-1H,12H-pyrano[3'',4'':6',7']indolizino[1',2':6,5]pyri do[4,3,2-de]quinazoline-2(3H)-thione-10,13(9H,15H)-dione;
b) (9S)-9-ethyl-9-hydroxy-1-phenethyl-1H,12H-pyrano[3",4":6',7']indolizino[1',2':6,5]pyrido[4,3,2-de]quinazoline-2(3H)-thione-10,13(9H,15H)-dione; and
c) (9S)-9-ethyl-9-hydroxy-1-pentyl-1H,12H-pyrano[3",4":6',7']indolizino[1',2':6,5]pyrido[4,3,2-d e]quinazoline-2(3H)-thione-10,13(9H,15H)-dione.

21. The preventive or therapeutic agent for pancreatic cancer, ovarian cancer, or liver cancer of claim 14, wherein Y is a group represented by formula (8): 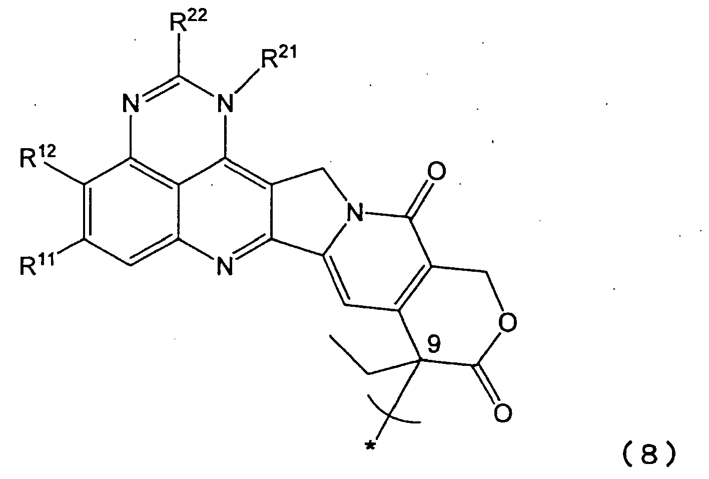 (wherein,
* indicates a linkage site; and
R¹¹, R¹², R²¹, and R²² are each defined as in claim 14).

22. The preventive or therapeutic agent for pancreatic cancer, ovarian cancer, or liver cancer of claim 21, wherein R¹¹ is a hydrogen atom;
R¹² is a hydrogen atom or a C1-C3 alkyl group;
R²¹ is a hydrogen atom, or a C1-C8 alkyl group that may comprise one to three substituents selected from Group D described below; and
R²² is a hydrogen atom, an amino group, or a C1-C6 alkyl group that may comprise one to three substituents selected from Group D described below, a C1-C6 alkoxy group that may comprise one to three substituents selected from Group D described below, a C1-C6 alkylthio group that may comprise one to three substituents selected from Group D described below, a mono-C1-C6 alkylamino group that may comprise one to three substituents selected from Group D described below, or a di-C1-C6 alkyl amino group that may comprise one to three substituents selected from Group D described below:
Group D: a C1-C3 alkoxy group, a hydroxy group, a halogen atom, an amino group, a mono-C1-C3 alkylamino group, a di-C1-C3 alkylamino group, a C3-C7 cycloalkyl group, a heterocycle, and an aryl ring (the aryl ring may comprise one to three substituents selected from the group consisting of a hydroxy group, a C1-C3 alkoxy group, and a halogen atom).

23. The preventive or therapeutic agent for pancreatic cancer, ovarian cancer, or liver cancer of claim 21 or 22, wherein Y is a residue of a compound (Y-OH) comprising at least one alcoholic hydroxyl group, wherein the compound is selected from the group consisting of:
a) (9S)-1-butyl-9-ethyl-9-hydroxy-1H,12H-pyrano[3",4":6',7']indolizino[1',2':6,5]pyrido[4,3,2-de]quinazoline-10,13(9H,15H)-dione;
b) (9S)-9-ethyl-9-hydroxy-1-[2-(4-morpholino)ethyl]-1H,12H-pyrano[3",4":6',7']indolizino[1',2': 6,5]pyrido[4,3,2-de]quinazoline-10,13(9H,15H)-dione;
c) (9S)-9-ethyl-9-hydroxy-1-propyl-1H,12H-pyrano[3",4":6',7']indolizino[1',2':6,5]pyrido[4,3,2-de]quinazoline-10,13(9H,15H)-dione;
d) (9S)-1-benzyl-9-ethyl-9-hydroxy-1H,12H-pyrano[3",4":6',7']indolizino[1',2':6,5]pyrido[4,3,2-de]quinazoline-10,13(9H,15H)-dione;
e) (9S)-9-ethyl-9-hydroxy-1-phenethyl-1H,12H-pyrano[3",4":6',7']indolizino[1',2':6,5]pyrido[4,3,2-de]quinazoline-10,13(9H,15H)-dione;
f) (9S)-2,9-diethyl-9-hydroxy-1-phenethyl-1H,12H-pyrano[3",4":6',7']indolizino[1',2':6,5]pyrido [4,3,2-de]quinazoline-10,13(9H,15H)-dione;
g) (9S)-9-ethyl-9-hydroxy-1-(3-phenylpropyl)-1H,12H-pyrano[3",4":6',7']indolizino[1',2':6,5]pyr ido[4,3,2-de]quinazoline-10,13(9H,15H)-dione;
h) (9S)-9-ethyl-9-hydroxy-1-(3-methylbutyl)-1H,12H-pyrano[3",4":6',7']indolizino[1',2':6,5]pyri do[4,3,2-de]quinazoline-10,13(9H,15H)-dione;
i) (9S)-2,9-diethyl-9-hydroxy-1-(3-methylbutyl)-1H,12H-pyrano[3",4":6',7']indolizino[1',2':6,5] pyrido[4,3,2-de]quinazoline-10,13(9H,15H)-dione;
j) (9S)-2,9-diethyl-9-hydroxy-1-(2-methylpropyl)-1H,12H-pyrano[3",4":6',7']indolizino[1',2':6,5 ]pyrido[4,3,2-de]quinazoline-10,13(9H,15H)-dione;
k) (9S)-9-ethyl-1-heptyl-9-hydroxy-1H,12H-pyrano[3",4":6',7']indolizino[1'2':6,5]pyrido[4,3,2-d e]quinazoline-10,13(9H,15H)-dione;
l) (9S)-9-ethyl-9-hydroxy-1-methyl-1H,12H-pyrano[3",4":6',7']indolizino[1',2':6,5]pyrido[4,3,2-de]quinazoline-10,13(9H,15H)-dione;
m) (9S)-9-ethyl-9-hydroxy-1-(2-methylpropyl)-1H,12H-pyrano[3",4":6',7']indolizino[1',2':6,5]pyr ido[4,3,2-de]quinazoline-10,13(9H,15H)-dione;
n) (9S)-9-ethyl-1-hexyl-9-hydroxy-1H,12H-pyrano[3",4":6',7']indolizino[1',2':6,5]pyrido[4,3,2-d e]quinazoline-10,13(9H,15H)-dione;
o) (9S)-9-ethyl-9-hydroxy-1-pentyl-1H,12H-pyrano[3",4":6',7']indolizino[1',2':6,5]pyrido[4,3,2-d e]quinazoline-10,13(9H,15H)-dione;
P) (9S)-1,9-diethyl-9-hydroxy-1H,12H-pyrano[3",4":6',7']indolizino[1',2':6,5]pyrido[4,3,2-de]qui nazoline-10,13(9H,15H)-dione;
q) (9S)-9-ethyl-9-hydroxy-1-[2-(4-methoxyphenyl)ethyl]-1H,12H-pyrano[3",4":6',7']indolizino[1',2':6,5]pyrido[4,3,2-de]quinazoline-10,13(9H,15H)-dione;
r) (9S)-1-[2-(4-chlorophenyl)ethyl]-9-ethyl-9-hydroxy-1H,12H-pyrano[3",4"6',7']indolizino[1',2 ':6,5]pyrido[4,3,2-de]quinazoline-10,13(9H,15H)-dione;
s) (9S)-9-ethyl-1-[2-(4-fluorophenyl)ethyl]-9-hydroxy-1H,12H-pyrano[3",4":6',7']indolizino[1',2':6,5]pyrido[4,3,2-de]quinazoline-10,13(9H,15H)-dione;
t) (9S)-9-ethyl-1-[2-(4-fluorophenyl)ethyl]-9-hydroxy-2-methyl-1H,12H-pyrano[3",4":6',7']indoli zino[1',2':6,5]pyrido[4,3,2-de]quinazoline-10,13(9H,15H)-dione;
u) (9S)-9-ethyl-9-hydroxy-1-(1-methylethyl)-1H,12H-pyrano[3",4":6',7']indolizino[1',2':6,5]pyri do[4,3,2-de]quinazoline-10,13(9H, 15H)-dione;
v) (9S)-1-(3,3-dimethylbutyl)-9-ethyl-9-hydroxy-1H,12H-pyrano[3",4":6',7']indolizino[1',2':6,5] pyrido [4,3,2-de]quinazoline-10,13(9H,15H)-dione;
w) (9S)-9-ethyl-9-hydroxy-2-methoxy-1-(3-methylbutyl)-1H,12H-pyrano[3",4":6',7']indolizino[1',2':6,5]pyrido[4,3,2-de]quinazoline-10,13(9H,15H)-dione;
x) (9S)-2,9-diethyl-9-hydroxy-1-(3-methylbutyl)-1H,12H-pyrano[3",4":6',7']indolizino[1',2':6,5] pyrido[4,3,2-de]quinazoline-10,13(9H,15H)-dione;
y) (9RS)-9-ethyl-9-hydroxy-4-methyl-1-pentyl-1H,12H-pyrano[3",4":6',7']indolizino[1',2':6,5]pyrido [4,3,2-de]quinazoline-10,13(9H,15H)-dione;
z) (9S)-9-ethyl-9-hydroxy-1-(2-hydroxyethyl)-1H,12H-pyrano[3",4":6',7']indolizino[1',2':6,5]pyrido[4,3,2-de]quinazoline-10,13(9H,15H)-dione;
aa) (9S)-9-ethyl-9-hydroxy-1-(2-hydroxyethyl)-2-methyl-1H,12H-pyrano[3",4":6',7']indolizino[1',2':6,5]pyrido[4,3,2-de]quinazoline-10,13(9H,15H)-dione;
bb) (9S)-9-ethyl-9-hydroxy-2-methyl-1-pentyl-1H,12H-pyrano[3",4":6',7']indolizino[1',2':6,5]pyrido[4,3,2-de]quinazoline-10,13(9H,15H)-dione;
cc) (9S)-2,9-diethyl-9-hydroxy-1-pentyl-1H,12H-pyrano[3",4":6',7']indolizino[1',2':6,5]pyrido[4,3,2-de]quinazoline-10,13(9H,15H)-dione;
dd) (9S)-9-ethyl-9-hydroxy-1-pentyl-2-propyl-1H,12H-pyrano[3",4":6',7]indolizino[1',2':6,5]pyrido[4,3,2-de]quinazoline-10,13(9H,15H)-dione;
ee) (9S)-9-ethyl-9-hydroxy-2-hydroxymethyl-1-pentyl-1H,12H-pyrano[3",4":6',7']indolizino[1',2':6,5]pyrido[4,3,2-de]quinazoline-10,13(9H,15H)-dione;
ff) (9S)-9-ethyl-9-hydroxy-2-hydroxymethyl-1-(2-methylpropyl)-1H,12H-pyrano[3",4":6',7']indolizino[1',2':6,5]pyrido[4,3,2-de]quinazoline-10,13(9H,15H)-dione;
gg) (9S)-9-ethyl-9-hydroxy-2-hydroxymethyl-1-(3-methylbutyl)-1H,12H-pyrano[3",4":6',7']indolizino[1',2':6,5]pyrido[4,3,2-de]quinazoline-10,13(9H,15H)-dione;
hh) (9S)-2-chloromethyl-9-ethyl-9-hydroxy-1-(3-methylbutyl)-1H,12H-pyrano[3",4":6',7']indolizino[1',2':6,5]pyrido[4,3,2-de]quinazoline-10,13(9H,15H)-dione;
ii) (9S)-2-aminomethyl-9-ethyl-9-hydroxy-1-pentyl-1H,12H-pyrano[3",4":6',7']indolizino[1',2':6, 5]pyrido[4,3,2-de]quinazoline-10,13(9H,15H)-dione;
jj) (9S)-9-ethyl-9-hydroxy-1-pentyl-2-trifluoromethyl-1H,12H-pyrano[3",4":6',7']indolizino[1',2': 6,5]pyrido[4,3,2-de]quinazoline-10,13 (9H,15H)-dione;
kk) (9S)-9-ethyl-9-hydroxy-1-(3-methylbutyl)-2-methylthio-1H,12H-pyrano[3",4":6',7']indolizino[1',2':6,5]pyrido[4,3,2-de]quinazoline-10,13(9H,15H)-dione;
ll) (9S)-9-ethyl-2-etliylthio-9-hydroxy-1-(3-methylbutyl)-1H,12H-pyrano[3",4":6',7']indolizino[1',2':6,5]pyrido[4,3,2-de]quinazoline-10,13(9H,15H)-dione;
mm) (9S)-2-(dimethylamino)-9-ethyl-9-hydroxy-1-(2-methylpropyl)-1H,12H-pyrano[3",4":6',7']ind olizino[1',2':6,5]pyrido[4,3,2-de]quinazoline-10,13(9H,15H)-dione; and
nn) (9S)-2-(butylamino)-9-ethyl-9-hydroxy-1-(3-methylbutyl)-1H,12H-pyrano[3",4":6',7']indolizin o[1',2':6,5]pyrido[4,3,2-de]quinazoline-10,13(9H,15H)-dione.

24. The preventive or therapeutic agent for pancreatic cancer, ovarian cancer, or liver cancer of claim 1, wherein the water-soluble prodrug represented by formula (1) is at least one prodrug selected from the group consisting of:
(a) (9S)-9-ethyl-9-{[methyl-(2-methylamino-ethyl)-amino]-acetoxy}-1-pentyl-1H,12H-pyrano[3",4' ':6',7']indolizino[1',2':6,5]pyrido[4,3,2-de]quinazoline-10,13(9H,15H)-dione;
(b) (9S)-9-ethyl-9-(glycyl-sarcosyloxy)-1-pentyl-1H,12H-pyrano[3",4":6',7']indolizino[1',2':6,5]p yrido[4,3,2-de]quinazoline-10,13(9H,15H)-dione;
(c) (9S)-9-{[(2-amino-ethyl)-methyl-amino]-acetoxy}-9-ethyl-1-pentyl-1H,12H-pyrano[3",4":6',7']indolizino[1',2':6,5]pyrido[4,3,2-de]quinazoline-10,13(9H,15H)-dione;
(d) (9S)-9-ethyl-9-(sarcosyl-sarcosyloxy)-1-pentyl-1H,12H-pyrano[3",4":6',7']indolizino[1',2':6,5] pyrido[4,3,2-de]quinazoline-10,13(9H,15H)-dione;
(e) (9S)-9-[2-(2-aminoethanesulfonyl)ethoxycarbonyloxy]-9-ethyl-1-pentyl-1H,12H-pyrano[3'',4": 6',7']indolizino[1',2':6,5]pyrido[4,3,2-de]quinazoline-10,13(9H,15H)-dione;
(f) (aminoacetyl-methyl-amino)-acetic acid (S)-4-ethyl-3,13-dioxo-3,4,12,13-tetrahydro-1H-2-oxa-6,12a-diaza-dibenzo[b,h]fluoren-4-yl ester;
(g) (9S)-9-{2-[(R-2-amino-2-methoxycarbonyl)ethanesulfonyl]ethoxycarbonyloxy}-9-ethyl-1-pentyl -1H,12H-pyrano[3",4":6',7']indolizino[1',2':6,5]pyrido[4,3,2-de]quinazoline-10,13(9H,15H)-di one;
(h) (9S)-9-{2-[(R-2-amino-2-ethoxycarbonyl)ethanesulfonyl]ethoxycarbonyloxy}-9-ethyl-1-pentyl-1H,12H-pyrano[3",4":6',7']indolizino[1',2':6,5]pyrido[4,3,2-de]quinazoline-10,13(9H,15H)-di one;
(i) (9S)-9-ethyl-9-(*N*-methylalanyl-*N*-methylalanyloxy)-1-pentyl-1H,12H-pyrano[3",4":6',7']indoli zino[1',2':6,5]pyrido[4,3,2-de]quinazoline-10,13(9H,15H)-dione; and
(j) (9S)-9-ethyl-9-(sarcosyl-*N*-methylalanyloxy)-1-pentyl-1H,12H-pyrano[3",4":6',7']indolizino[1',2':6,5]pyrido[4,3,2-de]quinazoline-10,13(9H,15H)-dione.

25. Use of a compound represented by formula (9) in the production of a preventive or therapeutic agent for pancreatic cancer, ovarian cancer, or liver cancer, comprising a water-soluble prodrug represented by formula (2), a pharmaceutically acceptable salt thereof, or a hydrate or solvate of the prodrug or pharmaceutically acceptable salt: 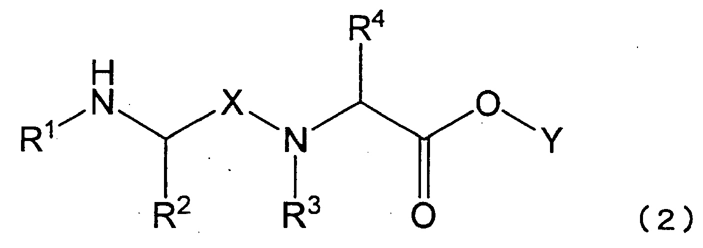 [wherein,
X represents C=O or a C1-C3 alkylene group;
Y represents a residue of a compound represented by Y-OH which comprises an alcoholic hydroxyl group, wherein said Y-OH is a camptothecin, a taxane, or an anticancer nucleotide;
R¹ represents a hydrogen atom or a C1-6 alkyl group;
R² and R⁴ each independently represents a hydrogen atom, a C1-6 alkyl group, or an amino acid side chain; and
R³ represents a C1-C6 alkyl group, 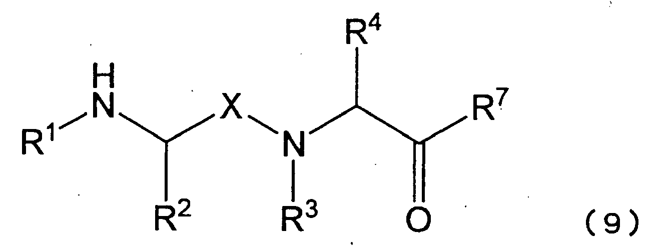 [wherein,
X, R¹, R², R³, and R⁴ are defined as in the above-mentioned formula (2);
the nitrogen atom where R¹ binds to may be protected with a protecting group, and
R⁷ represents a halogen atom or a group represented by OR⁸ (wherein R⁸ represents a hydrogen atom or a C1-C6 alkyl group)].

26. A method for preventing or treating pancreatic cancer, ovarian cancer or liver cancer, which comprises the step of administering an effective dose of a water-soluble prodrug represented by formula (1), or a pharmaceutically acceptable salt, or a hydrate or solvate of the prodrug or pharmaceutically acceptable salt to a patient in need of a prevention or treatment of pancreatic cancer, ovarian cancer, or liver cancer: 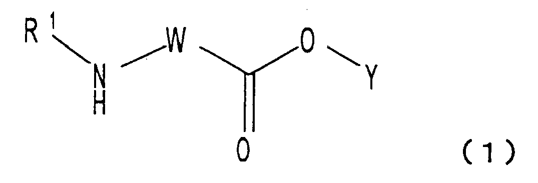 (wherein,
R¹ represents a hydrogen atom or a C1-C6 alkyl group;
W represents a divalent group comprising a tertiary amino group or a divalent group comprising a sulfonyl group;
Y represents a residue of a compound represented by Y-OH comprising an alcoholic hydroxyl group, wherein said Y-OH is a camptothecin, a taxane, or an anticancer nucleotide).
